# EUROPEAN PATENT APPLICATION

(11) **EP 0 947 515 A1**
(43) Date of publication of application: **06.10.1999**
(21) Application number: 97912545.7
(22) Date of filing: 25.11.1997
(51) Int. Cl.: C07D 471/04, A61K 31/435

(54) **NOVEL NAPHTHYRIDINE DERIVATIVES**

(30) Priority: 26.11.1996 JP 33152396
(71) Applicant: SUMITOMO PHARMACEUTICALS COMPANY, LIMITED, Osaka-shi, Osaka-fu 541 (JP)
(72) Inventor: MURAOKA, Masami, Toyonaka-shi, Osaka 560 (JP); IORIYA, Katsuhisa, Osaka-shi, Osaka 532 (JP); OHASHI, Naohito, Takatsuki-shi, Osaka 569-11 (JP); YAGI, Hideki, Nishinomiya-shi, Hyogo 662 (JP)
(74) Representative: VOSSIUS & PARTNER
(86) International application number: JP9704276
(87) International publication number: WO9823615

(57) **Abstract**

Naphthyridine derivative of the formula: 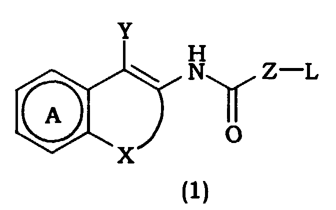 wherein Ring A is substituted or unsubstituted pyridine ring, X is ―N(R²)―CO― (R² is H, alkyl, substituted alkyl, etc.), Z is a direct bond, ―NH―, C₁₋₂ alkylene, or ―CH=CH―, Y is aromatic group or substituted aromatic group, L is alkyl, substituted alkyl, aromatic group or substituted aromatic group, or an acid addition salt thereof, these compounds exhibiting acyl-CoA: cholesterol acyl transferase inhibitory activity, and being useful as an agent for prophylaxis or treatment of hyperlipidemia, atherosclerosis, and related diseases thereof.

## Description

### TECHNICAL FIELD

The present invention relates to a naphthyridine derivative or an acid addition salt thereof, which shows acyl-CoA: cholesterol acyl transferase (ACAT) inhibitory activity, and is useful as an agent for treatment of hyperlipidemia and atherosclerosis, and a use thereof.

### PRIOR ART

Cerebral vessel disorders such as stroke, or myocardial infarction, which rank in high in causes of death in developed countries, break out with being accompanied by atherosclerosis as basal disease. From the results of epidemiology research, it is pointed out that hypercholesterolemia is one of risk factors for atherosclerosis, and there are mainly used anti-hyperlipidemic agents, which can reduce cholesterol level in blood, in the prophylaxis or treatment thereof. However, there is no sufficiently effective agent in terms of the effects thereof. Recently, it is observed that cells derived from macrophage accumulate cholesterol ester droplet within the cells and become foam cells in atherosclerotic lesions, and it is clarified that these foam cells deeply participate in the developments of atherosclerotic lesions (Atheriosclerosis, 10, 164-177, 1990). In addition, it is reported that ACAT activity is increased and cholesterol esters are accumulated in the vascular wall of atherosclerotic lesions (Biochem. Biophys. Acta, 617, 458-471, 1980). Therefore, an inhibitor of ACAT, which catalyses cholesterol esterification, is expected to suppress the formation or the development of atherosclerotic lesions as a result of the inhibition of foam cell formation and of cholesterol accumulation in vascular wall.

On the other hand, cholesterol in food is absorbed in the free form at intestinal epidermal cells, and then released in the form of chylomicron esterified by ACAT into the blood. Therefore, an inhibitor of ACAT is expected to reduce the cholesterol level in the blood caused by the inhibition of absorption of cholesterol in food at the intestine and reabsorption of cholesterol released into the intestine (J. Lipid. Research, 34, 279-294, 1993).

Japanese Patent Publication (Kokai) Nos. 3-181465, 3-223254 and Japanese Patent Publication (Kohyo) No. 6-501025 disclose some kinds of quinoline derivatives having ACAT inhibitory activity, and Japanese Patent Publication (Kokai) No. 5-32666 discloses some kinds of thienopyridine derivatives having an ACAT inhibitory activity, but these compounds are different in structure from the present compounds.

### DISCLOSURE OF INVENTION

An object of the present invention is to provide a naphthyridine derivative having an ACAT inhibitory activity and being useful as an agent for treatment of hyperlipidemia and atherosclerosis.

The present inventors have intensively studied in order to obtain a compound having a potent ACAT inhibitory activity, and have found that a compound of the following formula (1) and an acid addition salt thereof show such an activity, and then have accomplished the present invention. The compounds of the present invention have a different structure from the above-mentioned known compounds, and they are novel compounds.

A naphthyridine derivative of the formula: 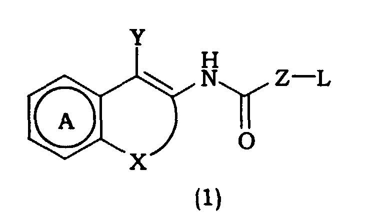 wherein Ring A is a substituted or unsubstituted pyridine ring,
X is a group of the formula: 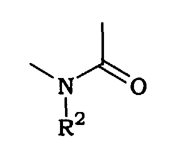 wherein R² is a hydrogen atom, an alkyl group, a substituted alkyl group, an alkenyl group, a substituted alkenyl group, an alkynyl group, a substituted alkynyl group, a cycloalkyl group, or a substituted cycloalkyl group, or a group of the formula: 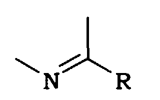 wherein R is a hydrogen atom or a group: ―OR¹ (R¹ is an alkyl group, a substituted alkyl group, an alkenyl group, a substituted alkenyl group, an alkynyl group, or a substituted alkynyl group),
Z is a direct bond, ―NH―, an alkylene group having 1 to 2 carbon atoms, or ―CH=CH―,
Y is an aromatic group or a substituted aromatic group,
L is an alkyl group, a substituted alkyl group, an alkenyl group, a substituted alkenyl group, a cycloalkyl group, a substituted cycloalkyl group, an aromatic group, or a substituted aromatic group, or an acid addition salt thereof.

Each group in the present invention is explained below.

Ring A is a substituted or unsubstituted pyridine ring, and the nitrogen atom thereof may be at any position except for the fused positions of the fused ring (that is, the nitrogen atom cannot be a bridgehead atom of the fused ring), and the preferable Ring A is the groups of the following formulae (a), (b) and (c).

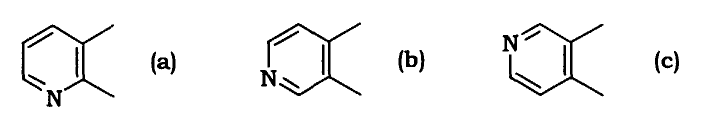

Besides, the substituent of the pyridine ring may be, for example, a lower alkyl group, a halogen atom, a cyano group, a trifluoromethyl group, a nitro group, an amino group, a mono-lower alkylamino group, a di-lower alkylamino group, a hydroxy group, a lower alkoxy group, a lower alkylthio group, a lower alkylsulfinyl group, a lower alkylsulfonyl group, etc.

The term "lower" in the present invention means that alkyl moiety described with "lower" is a lower alkyl group, and the lower alkyl group includes an alkyl group having 1 to 6 carbon atoms such as methyl, ethyl, propyl, 2-propyl, butyl, pentyl, hexyl, etc. The halogen atom is fluorine atom, chlorine atom, bromine atom, and iodine atom. The substituted pyridine ring has one or more substituents which are the same or different.

The alkyl group or the alkyl moiety of the substituted alkyl group for R¹, R² and Y includes, for example, a straight chain or branched chain alkyl group having 1 to 15 carbon atoms, such as methyl, ethyl, propyl, 2-propyl, butyl, 2-butyl, 2-methylpropyl, 1,1-dimethylethyl, pentyl, 3-pentyl, 3-methylbutyl, hexyl, 3-hexyl, 4-methyl-pentyl, 4-heptyl, octyl, 4-octyl, decyl, etc.

The alkenyl group or the alkenyl moiety of the substituted alkenyl group for R¹ and R² includes, for example, a straight chain or branched chain alkenyl group having 2 to 15 carbon atoms, such as vinyl, allyl, 2-propenyl, 2-methyl-2-propenyl, 2-butenyl, 3-butenyl, 3-methyl-2-butenyl, 4-pentenyl, 3-hexenyl, 3-ethyl-2-pentenyl, 4-ethyl-3-hexenyl, etc.

The alkynyl group or the alkynyl moiety of the substituted alkynyl group for R¹ and R² includes, for example, a straight chain or branched chain alkynyl group having 3 to 15 carbon atoms, such as 2-propynyl, 3-butynyl, 4-pentynyl, 3-hexynyl, 5-methyl-2-hexynyl, 6-methyl-4-heptynyl, etc.

The alkyl group or the alkyl moiety of the substituted alkyl group for L includes, for example, a straight chain or branched chain alkyl group having 1 to 20 carbon atoms, such as methyl, ethyl, propyl, 2-propyl, butyl, 2-butyl, 2-methylpropyl, 1,1-dimethylethyl, pentyl, 3-pentyl, hexyl, heptyl, octyl, undecyl, dodecyl, hexadecyl, 2,2-dimethyldodecyl, 2-tetradecyl, n-octadecyl, etc.

The alkenyl group or the alkenyl moiety of the substituted alkenyl group for L includes, for example, a straight chain or branched chain alkenyl group having 3 to 20 carbon atoms and having 1 to 2 double bonds, such as 2-propenyl, 2-butenyl, 3-methyl-2-butenyl, 3-pentenyl, 2-octenyl, 5-nonenyl, 4-undecenyl, 5-heptadecenyl, 3-octa-decenyl, 9-octadecenyl, 2,2-dimethyl-9-octadecenyl, 9,12-octadecadienyl, etc.

The cycloalkyl group or the cycloalkyl moiety of the substituted cycloalkyl group includes, for example, a cycloalkyl group having 3 to 7 carbon atoms, such as cyclopropyl, cyclobutyl, cyclopentyl, cyclohexyl, cycloheptyl, etc.

The aromatic group includes, for example, an aryl group and a heteroaryl group.

The aryl group includes, for example, an aryl group having carbon atoms of not more than 10, such as phenyl group, naphthyl group, etc.

The heteroaryl group or the heteroaryl moiety of the heteroarylmethyl group includes, for example, a 5- to 6-membered heteromonocyclic group having 1 to 2 nitrogen atoms, a 5- to 6-membered heteromonocyclic group having 1 to 2 nitrogen atoms and one oxygen atom or one sulfur atom, a 5-membered heteromonocyclic group having one oxygen atom or one sulfur atom, a heterobicyclic group formed by fusing a 6-membered ring and a 5- or 6-membered ring and having 1 to 4 nitrogen atoms, such as 2-pyridyl, 3-pyridyl, 4-pyridyl, 2-thienyl, 3-thienyl, 3-oxadiazolyl, 1-imidazolyl, 2-imidazolyl, 2-thiazolyl, 3-isothiazolyl, 2-oxazolyl, 3-isoxazolyl, 2-furyl, 3-furyl, 3-pyrrolyl, 2-quinolyl, 8-quinolyl, 2-quinazolinyl, 8-purinyl, etc.

The substituted aromatic group has one or more substituents which are the same or different, and the substituents are, for example, a halogen atom, a cyano group, a trifluoromethyl group, a nitro group, a hydroxy group, a methylenedioxy group, a lower alkyl group, a lower alkoxy group, a benzyloxy group, a lower alkanoyloxy group, an amino group, a mono-lower alkylamino group, a di-lower alkylamino group, a carbamoyl group, a lower alkylaminocarbonyl group, a di-lower alkylaminocarbonyl group, a carboxyl group, a lower alkoxycarbonyl group, a lower alkylthio group, a lower alkylsulfinyl group, a lower alkylsulfonyl group, a lower alkanoylamino group, a lower alkylsulfonamido group, or a group of the formula: ―M¹―E―Q (M¹ is a direct bond, an oxygen atom, a sulfur atom, or a group of the formula: ―NR³― (R³ is a hydrogen atom or a lower alkyl group), E is a divalent hydrocarbon group having 1 to 15 carbon atoms and optionally containing an unsaturated bond, or a phenylene group, Q is a hydrogen atom, a hydroxy group, a carboxyl group, a lower alkoxycarbonyl group, a benzyloxycarbonyl group, a halogen atom, a cyano group, a benzyloxy group, a lower alkoxy group, a lower alkanoyloxy group, a lower alkylthio group, a lower alkylsulfinyl group, a lower alkylsulfonyl group, a benzenesulfonyloxy group being optionally substituted by an alkyl group (e.g., p-toluenesulfonyloxy group), a lower alkanoylamino group, a lower alkoxycarbonylamino group, a lower alkylsulfonamido group, a phthalimido group, a cycloalkyl group, an aryl group, a substituted aryl group, a heteroaryl group, a substituted heteroaryl group, a group of the formula: ―NR⁴R⁵ (R⁴ and R⁵ are independently a hydrogen atom, a lower alkyl group, a di-lower alkylamino-substituted lower alkyl group, a lower alkoxy-substituted lower alkyl group, a cycloalkyl group, a lower alkoxycarbonyl group, a heteroarylmethyl group, or an aralkyl group, or R⁴ and R⁵ may combine each other, and with the adjacent nitrogen atom to which they bond, form a saturated cyclic amino group having 4 to 8 carbon atoms as ones forming the said ring, and optionally having one ―NR⁸― (R⁸ is a hydrogen atom, a lower alkyl group, a phenyl group, a lower alkoxycarbonyl group, or a benzyl group) or one oxygen atom in the cycle thereof), or a group of the formula: ―C(=O)NR⁴R⁵ (R⁴ and R⁵ are the same as defined above)), or an acid addition salt thereof.

The divalent hydrocarbon group having 1 to 15 carbon atoms and optionally having an unsaturated bond includes, for example, an alkylene chain having 1 to 6 carbon atoms, preferably having 1 to 4 carbon atoms, such as methylene, ethylene, trimethylene, tetramethylene, pentamethylene, hexamethylene, etc., an alkenylene chain such as propenylene, butenylene, etc., an alkynylene chain such as ethynylene, propynylene, butynylene, or an alkynylene chain such as an alkynylene of the following formula: 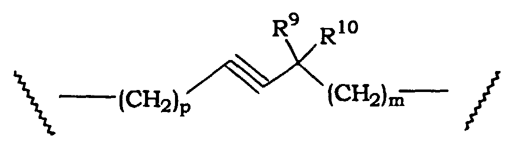 (R⁹ and R¹⁰ are independently a hydrogen atom, a methyl group, an ethyl group or a propyl group, or R⁹ and R¹⁰ may combine each other to form a cycloalkane ring having 3 to 7 carbon atoms, m is an integer of 0 to 6, preferably 0 or 1, and p is an integer of 0 to 6, preferably 0 or 1), specifically the groups of the following formulae:

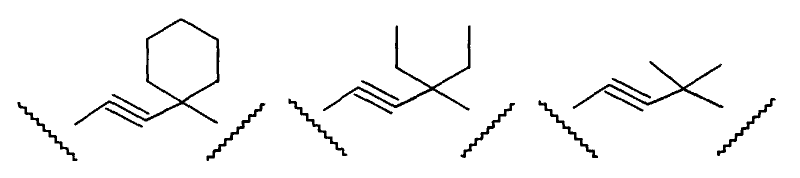

The substituted aryl group for Q has one or more substituents which are the same or different, and the substituents are, for example, a halogen atom, a cyano group, a trifluoromethyl group, a nitro group, a hydroxy group, a methylenedioxy group, a lower alkyl group, a lower alkoxy group, a benzyloxy group, a lower alkanoyloxy group, an amino group, a mono-lower alkylamino group, a di-lower alkylamino group, a carbamoyl group, a lower alkylaminocarbonyl group, a di-lower alkylaminocarbonyl group, a carboxyl group, a lower alkoxycarbonyl group, a lower alkylthio group, a lower alkylsulfinyl group, a lower alkylsulfonyl group, a lower alkanoylamino group, or a lower alkylsulfonamido group.

The heteroaryl group for Q includes, for example, a 5- to 6-membered cyclic group having 1 to 3 nitrogen atoms, a 5-membered cyclic group having one oxygen atom or one sulfur atom, or a bicyclic group formed by fusing a 6-membered ring and a 5- or 6-membered ring, and having 1 to 4 nitrogen atoms, such as 2-pyridyl, 3-pyridyl, 4-pyridyl, 1-pyrrolyl, 1-imidazolyl, 1,2,4-triazol-1-yl, 2-thienyl, 3-thienyl, 2-furyl, 3-furyl, 2-quinolyl, etc. The substituted heteroaryl group for Q has one or more substituents which are the same or different, and the substituents are, for example, a lower alkyl group, a lower alkoxy group, a halogen atom, etc.

The cyclic amino group formed by NR⁴R⁵ includes, for example, a group represented by the formula: 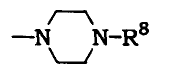 (wherein R⁸ is the same as defined above) such as 4-lower alkyl- 1-piperazinyl, 4-phenyl-1-piperazinyl, 4-benzyl-1-piperazinyl, etc., or a monocyclic group such as 1-pyrrolidinyl, 1-piperidinyl, 1-homopiperidinyl, 4-morpholinyl, etc., or a bicyclic group such as 3-azabicyclo[3.2.2]nonane, etc.

The substituted alkyl group, the substituted cycloalkyl group, the substituted alkenyl group, and the substituted alkynyl group have one or more substituents which are the same or different, and the substituents are, for example, a halogen atom, a cyano group, a phenoxy group, a benzyloxy group, a trifluoromethyl group, a hydroxy group, a lower alkoxy group, a lower alkanoyloxy group, an amino group, a mono-lower alkylamino group, a di-lower alkylamino group, a carbamoyl group, a lower alkylaminocarbonyl group, a di-lower alkylaminocarbonyl group, a lower alkoxycarbonylamino group, a carboxyl group, a lower alkoxycarbonyl group, a lower alkylthio group, a lower alkylsulfinyl group, a lower alkylsulfonyl group, a lower alkanoylamino group, a lower alkylsulfonamide group, a tri-lower alkylsilyl group, a phthalimide group, a heteroaryl group, a saturated heterocyclic group, or a group of the formula: ―M²―E―Q (M² is an oxygen atom, a sulfur atom, or a group of the formula: ―NR³ (R³ is the same as defined above), E and Q are the same as defined above). The heteroaryl group is the same heteroaryl groups for the above-mentioned Q. The saturated heterocyclic group includes, for example, a 5- to 8-membered cyclic group having one nitrogen atom, a 6- to 8-membered cyclic group having two nitrogen atoms, and a 6- to 8-membered cyclic group having one nitrogen atom and one oxygen atom, such as 1-piperidinyl, 1-pyrrolidinyl, etc.

The substituted alkyl group includes an alkyl group having 1 to 6 carbon atoms which is substituted by a cycloalkyl group or a substituted cycloalkyl group, or an aralkyl group or a substituted aralkyl group.

The aralkyl group or the substituted aralkyl group includes an alkyl group having 1 to 6 carbon atoms which is substituted by the above-mentioned aryl group or substituted aryl group, for example, benzyl, 1-phenylethyl, 2-phenylethyl, 2-naphthylmethyl, etc.

The preferable groups for Y are, for example, a phenyl group or pyridyl group which may optionally be substituted. The substituted phenyl group and the substituted pyridyl group have one or more substituents which are the same or different, and the preferable substituents are, for example, a halogen atom such as fluorine atom, chlorine atom, etc., a cyano group, a trifluoromethyl group, a nitro group, a hydroxy group, methylenedioxy group, a lower alkyl group, a lower alkoxy group, a lower alkanoyloxy group, an amino group, a mono-lower alkylamino group, a di-lower alkylamino group, a carbamoyl group, a lower alkylaminocarbonyl group, a di-lower alkylaminocarbonyl group, a carboxyl group, a lower alkoxycarbonyl group, a lower alkylthio group, a lower alkysulfinyl group, a lower alkylsulfonyl group, a lower alkanoylamino group, a lower alkylsulfonamido group, or a group of the formula: ―M¹―E―Q (M', E and Q are the same as defined above). The preferable groups for E are, for example, a straight alkylene, alkenylene or alkynylene chain having 1 to 6 carbon atoms, and the more preferable ones are a straight alkylene or alkynylene chain having 1 to 3 carbon atoms. The preferable groups for Q are, for example, a hydroxy group, a halogen atom, a cyano group, a lower alkoxy group, a lower alkanoyloxy group, a lower alkylthio group, a lower alkylsulfinyl group, a lower alkylsulfonyl group, a lower alkanoylamino group, a lower alkylsulfonamido group, a heteroaryl group, or a group of the formula: ―NR⁴R⁵ (R⁴ and R⁵ are the same as defined above), and the more preferable one are a substituted or unsubstituted heteroaryl group (e.g., 2-pyridyl, 3-pyridyl, 2-methyl-3-pyridyl, 4-pyridyl, 1-imidazolyl, 1,2,4-triazol-1-yl, etc.), or a group of the formula: ―NR⁴R⁵ (R⁴ and R⁵ are the same as defined above). The preferable group of the formula: ―NR⁴R⁵ (R⁴ and R⁵ are the same as defined above) includes, for example, dimethylamino, diethylamino, diisopropylamino, pyrrolidinyl, piperidinyl, morpholinyl, 4-methyl-piperazinyl, etc.

The preferable groups for L are a phenyl or heteroaryl group which may optionally be substituted, and the more preferable groups for L are a phenyl or pyridyl group which is substituted by 1 to 3 groups selected from a halogen atom such as a fluorine atom, chlorine atom, etc., a lower alkyl group, a lower alkoxy group and a lower alkylthio group, for example, 2,6-diisopropylphenyl, 2,4,6-trimethylphenyl, 2,4,6-trimethoxyphenyl, 2,4-difluorophenyl, 2,4,6-trifluorophenyl, 2,4-bis(methylthio)pyridin-3-yl, 2,4-bis(methylthio)-6-methylpyridin-3-yl, etc.

The preferable groups for X are groups of the following formulae:

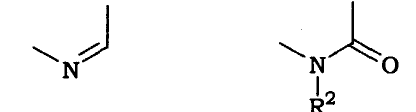

The preferable groups for R² are, for example, a hydrogen atom, an alkyl group, a substituted alkyl group, an alkenyl group, or a substituted alkenyl group. The substituted alkyl group and the substituted alkenyl group have one or more substituents which are the same or different, and the preferable substituents are, for example, a halogen atom such as fluorine atom, chlorine atom, etc., a cyano group, a benzyloxy group, a hydroxy group, a lower alkoxy group, a lower alkanoyloxy group, a carbamoyl group, a lower alkylaminocarbonyl group, a di-lower alkylaminocarbonyl group, a carboxyl group, a lower alkoxycarbonyl group, a lower alkylthio group, a lower alkylsulfinyl group, a lower alkylsulfonyl group, an aryl group, a lower alkanoylamino group, a lower alkylsulfonamido group, a phthalimido group, a heteroaryl group, a saturated heterocyclic group, etc. The more preferable substituents are, for example, a fluorine atom, a chlorine atom, a cyano group, a hydroxy group, a carbamoyl group, a 2-pyridyl group, a 3-pyridyl group, a 4-pyridyl group, etc.

The acid for forming an acid addition salt includes, for example, inorganic acids such as hydrochloric acid, hydrobromic acid, hydroiodic acid, sulfuric acid, nithric acid, etc., or organic acids such as acetic acid, oxalic acid, citric acid, malic acid, tartaric acid, fumaric acid, maleic acid, methanesulfonic acid, etc.

The compounds of the present invention may have a stereoisomer due to an asymmetric carbon atom thereof. In such cases, the present compounds also include each isomer or a mixture thereof.

The present compounds and an acid addition salt thereof may be in the form of an anhydrous product thereof, or in the form of a solvate thereof such as hydrate.

The compounds of the above-mentioned formula (1) or an acid addition salt thereof can be administered either parenterally or orally when used as the above-mentioned drug. The present compounds can be formulated into liquid preparations such as solutions, emulsions, suspensions, etc., and can be administered in the form of an injection, and if necessary, buffering agents, solubilizers and isotonic agents may be added thereto. The present compounds can also be administered rectally in the form of a suppository. The present compounds can also be administered orally in the form of a conventional administration form such as tablets, capsules, syrups, and suspension. These pharmaceutical preparations can be formulated by mixing an active ingredient with conventional carriers or diluents, binding agents or stabilizers by a conventional manner.

The dosage, the frequency of administration of the present compounds may vary according to the conditions, ages, weights of the patients and the administration form, etc., but the present compounds can be administered in a dosage of 1 to 500 mg, preferably 1 to 100 mg per day in adult, once a day, or divided into 2 - 4 dosage units.

The naphthyridine derivative which is an active ingredient of the present invention may be prepared by the following processes.

The compound (1) of the present invention wherein Z is ―NH― may be prepared by the following processes. 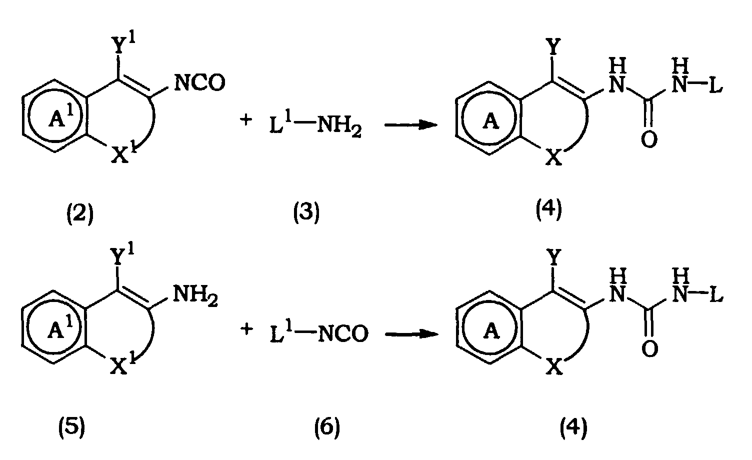 wherein Ring A, X, Y and B are the same as defined above, Ring A¹ is the same groups for Ring A but when these groups contain a reactive group as a substituent such as an amino group, an alkylamino group, a hydroxy group, etc. then these reactive groups should be protected, X¹ is the same groups for X, but when these groups contain a reactive group as a substituent such as an amino group, an alkylamino group, a hydroxy group, a carboxyl group, etc., then these reactive groups should be protected, Y¹ is the same groups for Y, but when these groups contain a reactive group as a substituent such as an amino group, an alkylamino group, a hydroxy group, a carboxyl group, etc., then these reactive groups should be protected, and L¹ is the same groups for L but when these groups contain a reactive group as a substituent such as an amino group, an alkylamino group, a hydroxy group, a carboxyl group, etc., then these reactive groups should be protected.

The isocyanate derivative (2) and the amine derivative (3) or an acid addition salt thereof are usually reacted in a solvent at a temperature of from 0°C to a boiling point of the solvent, preferably at a temperature of from room temperature to 120°C, and if necessary, the protecting groups of the product are removed to give the urea derivative (4). The solvent may be any solvent which does not disturb the reaction, but preferably ethers (e.g., ethyl ether, isopropyl ether, tetrahydrofuran, dioxane, etc), aromatic hydrocarbons (e.g., benzene, toluene, etc.), esters (e.g., methyl acetate, ethyl acetate, etc.), N,N-dimethylformamide, dimethylsulfoxide, and the like.

When the amine derivative (3) is used in the form of an acid addition salt thereof, the reaction may smoothly proceed by converting the compound (3) into a free form, if necessary. In this case, an agent for converting the compound (2) into a free form is preferably a tertiary amine such as triethylamine, etc., or an aromatic amine such as pyridine, etc. On the other hand, the amine derivative (5) or an acid addition salt thereof and the isocyanate derivative (6) are reacted to give the urea derivative (4), as well.

The protecting groups for amino group, alkylamino group, hydroxy group, carboxyl group, etc., may be conventional protecting groups which are used in the field of the organic chemistry, for example, the protecting group for hydroxy group may be benzyl group, acetyl group, etc., and the protecting group for amino group may be benzyl group, etc., and these protecting groups may be introduced and removed by a conventional method, such as by a method disclosed in PROTECTIVE GROUPS IN ORGANIC SYNTHESIS, 2nd ed., John Wiley & Sons, Inc.; New York.

The compound of the formula (7) among the urea derivatives (4), may be derived into other urea derivative of the formula (9) by reacting with an alkylating agent of the formula (8), and if necessary, followed by removing the protecting groups of the product. 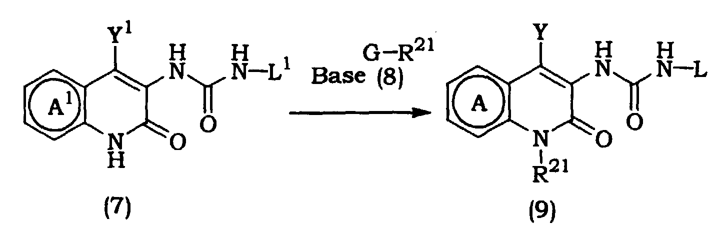 wherein Ring A, Y, L, Ring A¹, Y¹, and L¹ are the same as defined above, R²¹ is the same groups for R² but when these groups contain a reactive group as a substituent such as an amino group, an alkylamino group, a hydroxy group, a carboxyl group, etc., then these reactive groups should be protected, and G is a leaving group.

The alkylation reaction is carried out in a solvent at a temperature of from 0°C to 100°C, preferably at a temperature of from room temperature to 70°C in the presence of a base. The solvent includes, for example, ethers (e.g., tetrahydrofuran, dioxane), ketones (e.g., acetone, 2-butanone), aromatic hydrocarbons (e.g., benzene, toluene), dimethylformamide, etc. The base includes, for example, sodium hydride, potassium carbonate, sodium carbonate, triethylamine, etc. The leaving group represented by G is usually a halogen atom such as chlorine atom, bromine atom, iodine atom, etc., or an aromatic sulfonyloxy group such as p-toluenesulfonyloxy group.

The compound (1) wherein Z is a direct bond, an alkylene group having 1 to 2 carbon atoms, or ―CH=CH― can be prepared by the following process. 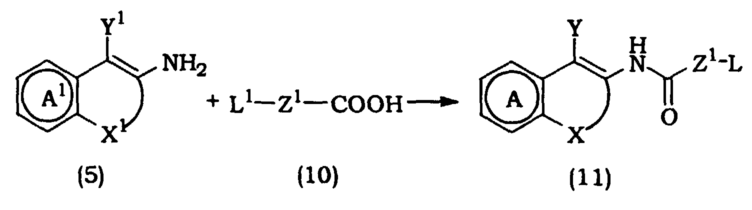 wherein Ring A, Y, L, X, Ring A¹, Y¹, L¹ and X¹ are the same as defined above, Z¹ is a direct bond, an alkylene group having 1 to 2 carbon atoms, or ―CH=CH―.

The amine derivative of the formula (5) or an acid addition salt thereof is condensed with the carboxylic acid derivative of the formula (10) in a solvent at a temperature of from 0°C to 100°C, preferably at a temperature of from 0°C to 60°C with using a condensation agent, and the protecting groups of the product are removed, if necessary, to give the amide derivative of the formula (11). The condensation agent includes, for example, dicyclohexylcarbodiimide (DCC), diethyl cyanophosphate (DEPC), 1-ethyl-3-(3'-dimethylaminopropyl)carbodiimide hydrochloride (WSC), etc. The reaction may preferably be carried out by addition of a base in an amount of 1 to 5 mole equivalents, preferably in an amount of 1 to 3 mole equivalents, to the amount of the amine derivative (5) or acid addition salts thereof. The base is preferably tertiary amines such as triethylamine, etc., or aromatic amines such as pyridine, etc. The solvent may be, for example, ethers (e.g., tetrahydrofuran, dioxane, etc.), aromatic hydrocarbons (e.g., benzene, toluene, etc.), esters (e.g., ethyl acetate, etc.), N,N-dimethylformamide, dimethylsulfoxide, etc.

The carboxylic acid derivative (10) may be converted into a reactive derivative thereof, and then reacted with the amine derivative (5) in a solvent at a temperature of from -10°C to 120°C, preferably at a temperature of from 0°C to 60°C to give the amide derivative (11). The reactive derivative of the compound (10) includes, for example, an acid chloride, an acid bromide, an acid anhydride, or a mixed acid anhydride with methyl carbonate, ethyl carbonate. The reaction may preferably be carried out by addition of a base in an amount of 1 to 5 mole equivalents, preferably in an amount of 1 to 3 mole equivalents. The base includes, for example, tertiary amines such as triethylamine, etc., aromatic amines such as pyridine, alkali metal carbonates such as sodium carbonate, potassium carbonate, alkali metal hydrogen carbonates such as sodium hydrogen carbonate, etc. The solvent may be, for example, halogenated solvents (e.g., chloroform, dichloromethane, etc.), ethers (e.g., tetrahydrofuran, dimethoxyethane, etc.), aromatic hydrocarbons (e.g., benzene, toluene, etc.), esters (e.g., ethyl acetate, etc.), pyridine or N,N-dimethylformamide.

The amide derivative (11) wherein Z¹ is ―CH₂CH₂― can also be prepared by reduction of the amide derivative (10) wherein Z¹ is ―CH=CH―. The reduction is carried out in a solvent by using a reducing agent (e.g., lithium aluminum hydride, sodium borohydride, lithium borohydride, etc.) in an amount of 0.5 to 5 mole equivalents, preferably in an amount of 0.5 to 2 mole equivalents, at a temperature of from -5°C to 120°C, preferably at a temperature of from 0°C to 80°C. The solvent includes, for example, alcohols (e.g., methanol, ethanol, etc.) and ethers (e.g., ethyl ether, tetrahydrofuran, dioxane, etc.). The reduction is also carried out by catalytic reduction. For example, the reduction is carried out by using as a catalyst palladium carbon, platinum oxide, Raney-nickel, etc. in a solvent under atmospheric pressure to a pressure of 5 atms of hydrogen gas, at a temperature of from 0°C to 100°C, preferably at a temperature of from room temperature to 60°C. The solvent may be alcohols (e.g., methanol, ethanol, etc.), formic acid, acetic acid, etc.

The substituents of Ring A, Y, X or L in the urea derivative (4) and the amide derivative (11) thus obtained can be converted into others, if necessary. For example, a lower alkylthio group can be converted into a lower alkylsulfonyl group by oxidization. A nitro group is converted into an amino group by reduction. An amino group can be alkylated to a mono- or di-alkylamino group, or an amino group can also be acylated. A 3-chloropropoxy group is converted into a 3-(1-imidazolyl)propoxy group. A halogen atom such as bromine atom or iodine atom can be converted into a 1-propargyl group having a hydroxy group, an amino group, etc. at 3-position thereof, by using a palladium catalyst. Moreover, such propargyl group can be converted into a propyl group by a hydrogenation reaction. Such conversion reactions can be carried out by using a well-known technique which is usually applied in the organic chemistry field. As one of the conversion reactions of the substituents, the alkylation reaction can be carried out as follows. 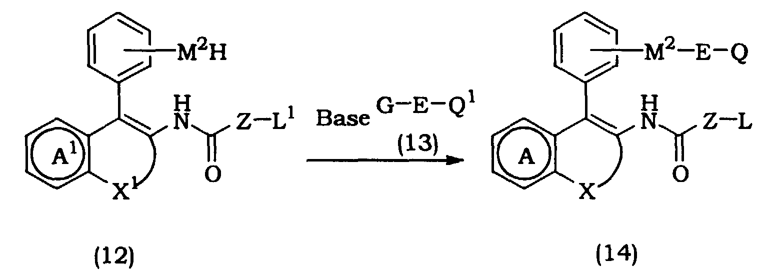 wherein Ring A, L, X, Z, E, Q, G, M², Ring A¹, L¹, and X¹ are the same as defined above, Q¹ is the same groups for Q but when these groups contain a reactive group as a substituent such as an amino group, an alkylamino group, a hydroxy group, a carboxyl group, etc., then these reactive groups should be protected.

The compound (12) is reacted with the alkylating agent (13) in a solvent, and if necessary, the protecting groups of the product are removed, to give the compound (14). The reaction is usually carried out at a temperature of from 0°C to 100°C, preferably at a temperature of from room temperature to 70°C, in the presence of a base. The solvent may be, for example, ethers (e.g., tetrahydrofuran, dioxane, etc.), aromatic hydrocarbons (e.g., benzene, toluene, etc.), ketones (e.g., acetone, 2-butanone, etc.), dimethylformamide, etc. The base may be, for example, sodium hydride, potassium carbonate, sodium carbonate, triethylamine, etc. When potassium carbonate or sodium carbonate is used, the efficiency of the reaction is optionally increased by addition of sodium iodide or potassium iodide. The leaving group represented by G is usually halogen atoms such as chlorine atom, bromine atom, iodine atom, etc., or an aromatic sulfonyloxy group such as p-toluenesulfonyloxy group, etc.

The starting compound (2) or (5) for preparing the compound (1) of the present invention or an acid addition salt thereof can be prepared by the following process or by a modified process thereof. 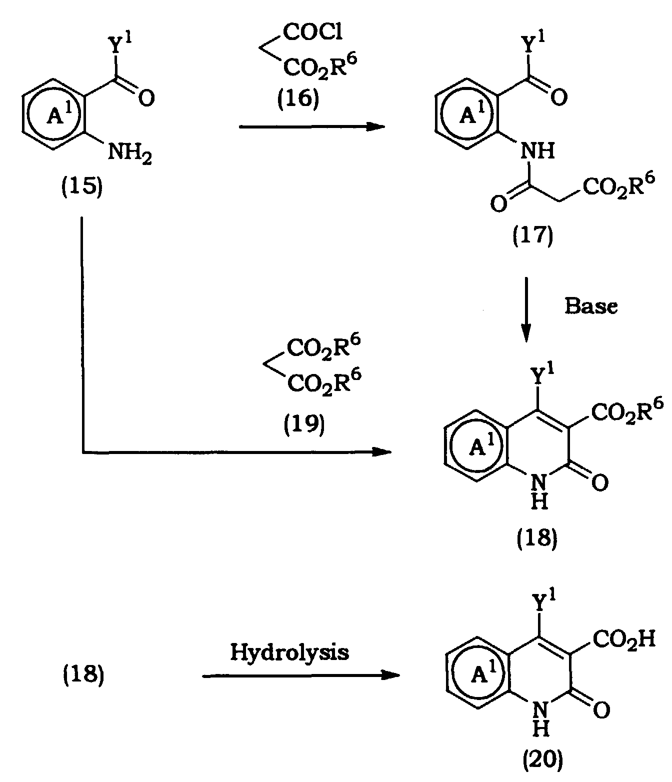 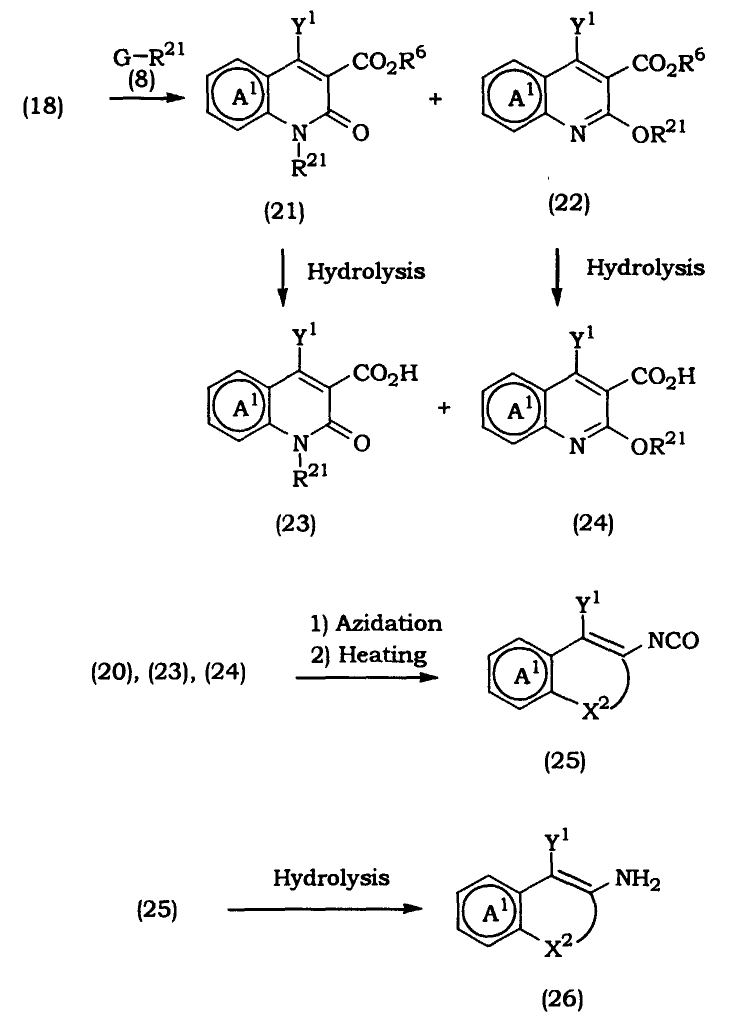 wherein Ring A¹, Y¹, R²¹ and G are the same as defined above, R⁶ is a lower alkyl group, and X² is ―NH―CO―, ―NR²¹―CO― or ―N=C(OR²¹)―.

The starting compound (15) is prepared by the method disclosed in the literature, for example, J. Heterocyclic Chem., 26, 105-112, 1989, or a modified method thereof. The lower alkyl group represented by R⁶ is preferably one having 1 to 4 carbon atoms, such as methyl, ethyl, propyl, isopropyl, butyl, etc.

The aminoketone derivative (15) is reacted with the acid chloride (16) in the presence of a base in a solvent at a temperature of from -20°C to 150°C, preferably at a temperature of from 0°C to 120°C, to give the amide derivative (17). The solvent may be, for example, ethers (e.g., ethyl ether, tetrahydrofuran, dioxane, etc.), aromatic hydrocarbons (e.g., benzene, toluene, etc.), halogenated solvents (e.g., dichloromethane, chloroform, etc.), pyridine, dimethylformamide, etc. The base includes, for example, triethylamine, pyridine, potassium carbonate, sodium carbonate, sodium hydrogen carbonate, etc. The amide derivative (17) thus obtained is subjected to cyclization reaction in a solvent such as benzene, toluene, tetrahydrofuran, dimethoxyethane, etc., at a temperature of from 0°C to 200°C, preferably at a temperature of from room temperature to 170°C, in the presence of a base in an amount of 0.1 to 3 mole equivalents, preferably in an amount of 0.1 to 2 mole equivalents to give the compound (18). The base includes, for example, potassium t-butoxide, sodium methoxide, sodium ethoxide, piperidine, triethylamine, 1,5-diazabicyclo[4.3.0]non-5-ene (DBN), 1,8-diazabicyclo[5.4.0]-7-undecene (DBU), and 1,4-diazabicyclo[2.2.2]octane (DABCO). The compound (18) can also be prepared by heating the compound (15) with the malonic acid diester derivative (19) in the presence of an amine (e.g., piperidine, pyrrolidine, triethylamine, pyridine, DBN, DBU, DABCO, etc.), or potassium fluoride, tetrabutylammonium fluoride, at a temperature of from 60°C to 200°C, without a solvent.

On the other hand, the compound (18) is reacted with the alkylating agent (8) in the presence of a base at a temperature of from 0°C to 150°C, preferably at a temperature of from room temperature to 100°C, in a solvent, to give the N-alkyl compound (21) and/or the O-alkyl compound (22). The solvent includes, for example, alcohols (e.g., methanol, ethanol, etc.), ethers (e.g., tetrahydrofuran, dioxane, etc.), ketones (e.g., acetone, 2-butanone, etc.), and dimethylformamide. The base includes, for example, sodium hydroxide, potassium hydroxide, sodium methoxide, sodium ethoxide, potassium t-butoxide, sodium hydride, potassium carbonate, sodium carbonate, triethylamine, etc. The leaving group represented by G is usually a halogen atom such as chlorine atom, bromine atom, iodine atom, etc., or an aromatic sulfonyloxy group such as p-toluenesulfonyloxy group. In this reaction, there is obtained a mixture of the compound (21) and the compound (22), but both compounds can be separated by recrystallization or chromatography. On the other hand, the compound (21) can be preferentially obtained by selecting the kinds of the compound (18), the kinds of the solvents, the kinds of the base, or reaction temperature.

The hydrolysis of the compound (18), the compound (21) and the compound (22) is carried out by a conventional method, for example, in a solvent such as methanol, ethanol, isopropanol, tetrahydrofuran, dioxane, dimethoxyethane, etc., at a temperature of from 0°C to 150°C, preferably at a temperature of from 0°C to 100°C, by using a hydroxide of an alkali metal or an alkaline earth metal such as sodium hydroxide, potassium hydroxide, barium hydroxide, etc. The carboxylic acid derivative of the formulae (20), (23) or (24) can be converted into the isocyanate derivative (25) by a conventional method, and if necessary, the compound (25) is further converted into the amine derivative (26). For example, the carboxylic acid derivative of the formulae (20), (23) or (24) is converted into an acid azide compound by using an azidation agent (e.g., diphenylphosphoryl azide (DPPA), etc.) in an amount of 1 to 3 mole equivalents, in the presence of a base (e.g., triethylamine, N-methylmorpholine, etc.), at a temperature of from 0°C to 150°C, preferably at a temperature of from room temperature to 120°C in a solvent such as aromatic hydrocarbons (e.g., benzene, toluene), N,N-dimethylformamide, etc., and the acid azide compound thus obtained is heated at a temperature of from 20°C to 200°C, preferably at a temperature of from 30°C to 150°C without isolating from the reaction mixture, to give the compound (25). Moreover, the compound (25) is hydrolyzed in the same manner as in the hydrolysis of the compound (18), (21) or (22), to give the compound (26).

Some of the compounds (5) can be prepared by the following process, or by a modified process thereof. 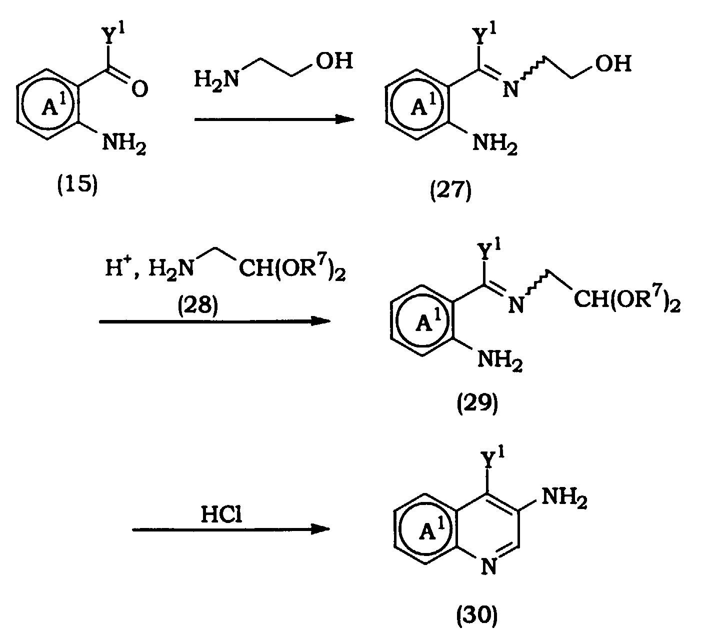 wherein Ring A¹ and Y¹ are the same as defined above, and R⁷ is an alkyl group.

The alkyl group for R⁷ is preferably one having 1 to 4 carbon atoms such as methyl, ethyl, propyl, isopropyl, butyl, etc.

The aminoketone derivative (15) is treated in the same manner as disclosed in Yakugaku Zasshi, vol. 93, p. 1263 (1973), or a modified method thereof, to give the aminonaphthyridine derivative (30).

The present compounds obtained by the present process, and the intermediates therefor may be purified by a conventional method, for example, column chromatography, recrystallization, etc. The solvent for recrystallization may be, for example, alcohols (e.g., methanol, ethanol, 2-propanol, etc.), ethers (e.g., diethyl ether, etc.), esters (e.g., ethyl acetate, etc.), aromatic solvents (e.g., toluene, etc.), ketones (e.g., acetone, etc.), hydrocarbons (e.g., hexane, etc.), or a mixture of these solvents, which is selected according to the kinds of the compound to be recrystallized.

The representatives of the present compound obtained by the above process are as follows:
N-[1-methyl-4-{3-(4-pyridylmethoxy)phenyl}-1,2-dihydro-2-oxo-1,8-naphthyridin-3-yl]-N'-(2,6-diisopropylphenyl)urea
N-[1-benzyl-4-{3-(4-pyridylmethoxy)phenyl}-1,2-dihydro-2-oxo-1,8-naphthyridin-3-yl]-N'-(2,6-diisopropylphenyl)urea
N-[1-cyclopropylmethyl-4-{3-(2-diethylaminoethoxy)phenyl}-1,2-dihydro-2-oxo-1,8-naphthyridin-3-yl]-N'-(2,6-diisopropylphenyl)urea
N-[1-cyclopropylmethyl-4-{3-(4-pyridylmethoxy)phenyl}-1,2-dihydro-2-oxo-1,8-naphthyridin-3-yl]-N'-(2,6-diisopropylphenyl)urea
N-[ 1-cyclopentylmethyl-4-{3-(4-pyridylmethoxy)phenyl}-1,2-dihydro-2-oxo-1,8-naphthyridin-3-yl]-N'-(2,6-diisopropylphenyl)urea
N-[1-cyclohexylmethyl-4-{3-(4-pyridylmethoxy)phenyl}-1,2-dihydro-2-oxo-1,8-naphthyridin-3-yl]-N'-(2,6-diisopropylphenyl)urea
N-[1-ethyl-4-{3-(4-pyridylmethoxy)phenyl}-1,2-dihydro-2-oxo-1,8-naphthyridin-3-yl]-N'-(2,6-diisopropylphenyl)urea
N-[1-(2-phenylethyl)-4-{3-(4-pyridylmethoxy)phenyl}-1,2-dihydro-2-oxo- 1,8-naphthyridin-3-yl]-N'-(2,6-diisopropylphenyl)urea
N-[1-propyl-4-{3-(4-pyridylmethoxy)phenyl}-1,2-dihydro-2-oxo-1,8-naphthyridin-3-yl]-N'-(2,6-diisopropylphenyl)urea
N-[1-(2-propenyl)-4-{3-(4-pyridylmethoxy)phenyl}-1,2-dihydro-2-oxo- 1,8-naphthyridin-3-yl]-N'-(2,6-diisopropylphenyl)urea
N-[1-(2-methyl-2-propenyl)-4-{3-(4-pyridylmethoxy)phenyl}-1,2-dihydro-2-oxo-1,8-naphthyridin-3-yl]-N'-(2,6-diisopropylphenyl)urea
N-[1-isopropyl-4-{3-(4-pridylmethoxy)phenyl}-1,2-dihydro-2-oxo-1,8-naphthyridin-3-yl]-N'-(2,6-diisopropylphenyl)urea
N-[1-butyl-4-[3-{2-(2-pyridyl)ethoxy}phenyl]-1,2-dihydro-2-oxo-1,8-naphthyridin-3-yl]-N'-(2,6-diisopropylphenyl)urea
N-[1-butyl-4-[3-{2-(3-pyridyl)ethoxy}phenyl]-1,2-dihydro-2-oxo-1,8-naphthyridin-3-yl]-N'-(2,6-diisopropylphenyl)urea
N-[1-butyl-4-[3-{2-(4-pyridyl)ethoxy}phenyl]-1,2-dihydro-2-oxo-1,8-naphthyridin-3-yl]-N'-(2,6-diisopropylphenyl)urea
N-[1-butyl-4-[3-{3-(2-pyridyl)propoxy)phenyl]-1,2-dihydro-2-oxo-1,8-naphthyridin-3-yl]-N'-(2,6-diisopropylphenyl)urea
N-(1-butyl-4-[3-{3-(3-pyridyl)propoxy}phenyl]-1,2-dihydro-2-oxo-1,8-naphthyridin-3-yl]-N'-(2,6-diisopropylphenyl)urea
N-[1-butyl-4-[3-(3-(4-pyridyl)propoxy)phenyl]- 1,2-dihydro-2-oxo-1,8-naphthyridin-3-yl]-N'-(2,6-diisopropylphenyl)urea
N-[1-butyl-4-{3-(3-diethylamino- 1-propynyl)phenyl}-1,2-dihydro-2-oxo-1,8-naphthyridin-3-yl]-N'-(2,6-diisopropylphenyl)urea
N-[1-butyl-4-(3-(3-hydroxy-1-propyhyl)phenyl}-1,2-dihydro-2-oxo-1,8-naphthyridin-3-yl]-N'-(2,6-diisopropylphenyl)urea
N-[1-butyl-4-{3-(3-hydroxypropyl)phenyl}-1,2-dihydro-2-oxo-1,8-naphthyridin-3-yl]-N'-(2,6-diisopropylphenyl)urea
N-[1-isobutyl-4-{3-(4-pyridylmethoxy)phenyl}-1,2-dihydro-2-oxo-1,8-naphthyridin-3-yl]-N'-(2,6-diisopropylphenyl)urea
N-[1-(3-butenyl) 4-{3-(4-pyridylmethoxy)phenyl}-1,2-dihydro-2-oxo-1,8-naphthyridin-3-yl]-N'-(2,6-diisopropylphenyl)urea
N-[1-(3-methyl-2-butenyl)-4-{3(4-pyridylmethoxy)phenyl}-1,2-dihydro-2-oxo-1,8-naphthyridin-3-yl]-N'-(2,6-diisopropylphenyl)urea
N-[1-pentyl-4-{3-(4-pyridylmethoxy)phenyl}-1,2-dihydro-2-oxo-1,8-naphthyridin-3-yl]-N'-(2,6-diisopropylphenyl)urea
N-[1-(4-pentenyl)-4-{3-(4-pyridylmethoxy)phenyl}-1,2-dihydro-2-oxo-1,8-naphthyridin-3-yl]-N'-(2,6-diisopropylphenyl)urea
N-[1-(4-pentynyl)-4-{3-(4-pyridylmethoxy)phenyl}-1,2-dihydro-2-oxo-1,8-naphthyridin-3-yl]-N'-(2,6-diisopropylphenyl)urea
N-[1-(4-methylpentyl)-4-{3-(4-pyridylmethoxy)phenyl}-1,2-dihydro-2-oxo-1,8-naphthyridin-3-yl]-N'-(2,6-diisopropylphenyl)urea
N-[1-hexyl-4-{3-(4-pyridylmethoxy)phenyl}-1,2-dihydro-2-oxo-1,8-naphthyridin-3-yl]-N'-(2,6-diisopropylphenyl)urea
N-[ 1-octyl-4-{3-(4-pyridylmethoxy)phenyl}- 1,2-dihydro-2-oxo-1,8-naphthyridin-3-yl]-N'-(2,6-diisopropylphenyl)urea
N-[1-decyl-4-{3-(4-pyridylmethoxy)phenyl}-1,2-dihydro-2-oxo-1,8-naphthyridin-3-yl]-N'-(2,6-diisopropylphenyl)urea
N-[1-methyl-4-[3-{3-(4-phenyl-1-piperazinyl)propoxy}phenyl]-1,2-dihydro-2-oxo-1,8-naphthyridin-3-yl]-N'-butylurea
N-[1-benzyl-4-{3-(3-pyridylmethoxy)phenyl}-1,2-dihydro-2-oxo-1,8-naphthyridin-3-yl]-N'-(2,6-diisopropylphenyl)urea
N-[1-benzyl-4-[3-(2-(2-pyridyl)ethoxy)phenyl]-1,2-dihydro-2-oxo-1,8-naphthyridin-3-yl]-N'-(2,6-diisopropylphenyl)urea
N-[1-benzyl-4-[3-{2-(3-pyridyl)ethoxy}phenyl]-1,2-dihydro-2-oxo-1,8-naphthyridin-3-yl]-N'-(2,6-diisopropylphenyl)urea
N-[1-benzyl-4-[3-{2-(4-pyridyl)ethoxy}phenyl]-1,2-dihydro-2-oxo-1,8-naphthyridin-3-yl]-N'-(2,6-diisopropylphenyl)urea
N-[1-benzyl-4-[3-{2-(1,2,4-triazol-1-yl)ethoxy}phenyl]-1,2-dihydro-2-oxo-1,8-naphthyridin-3-yl]-N'-(2,6-diisopropylphenyl)urea
N-[1-benzyl-4-[3-{3-(4-pyridyl)propoxy}phenyl]-1,2-dihydro-2-oxo-1,8-naphthyridin-3-yl]-N'-(2,6-diisopropylphenyl)urea
N-[1-benzyl-4-[3-{3-(1,2,4-triazol-1-yl)propoxy}phenyl]-1,2-dihydro-2-oxo-1,8-naphthyridin-3-yl]-N'-(2,6-diisopropylphenyl)urea
N-[1-cyclohexylmethyl-4-{3-(2-diethylaminoethoxy)phenyl]-1,2-dihydro-2-oxo-1,8-naphthyridin-3-yl]-N'-(2,6-diisopropylphenyl)urea
N-[1-cyclohexylmethyl-4-{3-(3-pyridylmethoxy)phenyl}-1,2-dihydro-2-oxo-1,8-naphthyridin-3-yl]-N'-(2,6-diisopropylphenyl)urea
N-[1-cyclohexylmethyl-4-[3-{3-(1,2,4-triazol-1-yl)propoxy)-phenyl]-1,2-dihydro-2-oxo-1,8-naphthyridin-3-yl]-N'-(2,6-diisopropylphenyl)urea
N-[1-(2-phenylethyl)-4-{3-(2-pyridylmethoxy)phenyl}-1,2-dihydro-2-oxo-1,8-naphthyridin-3-yl]-N'-(2,6-diisopropylphenyl)urea
N-[1-(2-phenylethyl)-4-{3-(3-pyridylmethoxy)phenyl}-1,2-dihydro-2-oxo-1,8-naphthyridin-3-yl]-N'-(2,6-diisopropylphenyl)urea
N-[1-(2-phenylethyl)-4-[3-{2-(3-pyridyl)ethoxy}phenyl}-1,2-dihydro-2-oxo-1,8-naphthyridin-3-yl]-N'-(2,6-diisopropylphenyl)urea
N-[1-(2-phenylethyl)-4-{3-(2-diethylaminoethoxy)phenyl}-1,2-dihydro-2-oxo-1,8-naphthyridin-3-yl]-N'-(2,6-diisopropylphenyl)urea
N-[1-(2-phenylethyl)-4-{3-(3-diethylamino-1-propynyl)phenyl}-1,2-dihydro-2-oxo-1,8-naphthyridin-3-yl]-N'-(2,6-diisopropylphenyl)urea
N-[1-(2-phenylethyl)-4-{3-(3-diethylaminopropyl)phenyl}-1,2-dihydro-2-oxo-1,8-naphthyridin-3-yl]-N'-(2,6-diisopropylphenyl)urea
N-[1-(2-phenylethyl)-4-{3-(3-hydroxy-1-propynyl)phenyl}-1,2-dihydro-2-oxo-1,8-naphthyridin-3-yl]-N'-(2,6-diisopropylphenyl)urea
N-[1-(2-phenylethyl)-4-{3-(3-hydroxypropyl)phenyl}-1,2-dihydro-2-oxo-1,8-naphthyridin-3-yl]-N'-(2,6-diisopropylphenyl)urea
N-[1-(2-phenylethyl)-4-{3-(2-diethylaminoethylthio)phenyl}-1,2-dihydro-2-oxo-1,8-naphthyridin-3-yl]-N'-(2,6-diisopropylphenyl)urea
N-[1-propyl-4-{3-(2-diethylaminoethoxy)phenyl}-1,2-dihydro-2-oxo-1,8-naphthyridin-3-yl]-N'-(2,6-diisopropylphenyl)urea
N-[1-propyl-4-{3-(3-pyridylmethoxy)phenyl}-1,2-dihydro-2-oxo-1,8-naphthyridin-3-yl]-N'-(2,6-diisopropylphenyl)urea
N-[1-propyl-4-[3-{3-(1,2,4-triazol-1-yl)propoxy}phenyl]-1,2-dihydro-2-oxo-1,8-naphthyridin-3-yl]-N'-(2,6-diisopropylphenyl)urea
N-[1-(2-methyl-2-propenyl)-4-{3-(2-diethylaminoethoxy)phenyl}-1,2-dihydro-2-oxo-1,8-naphthyridin-3-yl]-N'-(2,6-diisopropylphenyl)urea
N-[1-(2-methyl-2-propenyl)-4-{3-(3-pyridylmethoxy)phenyl}-1,2-dihydro-2-oxo-1,8-naphthyridin-3-yl]-N'-(2,6-diisopropylphenyl)urea
N-[1-(2-methyl-2-propenyl)-4-[3-{3-(1,2,4-triazol-1-yl)-propoxy}phenyl]-1,2-dihydro-2-oxo-1,8-naphthyridin-3-yl]-N'-(2,6-diisopropylphenyl)urea
N-[1-isobutyl-4-{3-(2-diethylaminoethoxy)phenyl}-1,2-dihydro-2-oxo-1,8-naphthyridin-3-yl]-N'-(2,6-diisopropylphenyl)urea
N-[1-isobutyl-4-{3-(3-pyridylmethoxy)phenyl}-1,2-dihydro-2-oxo-1,8-naphthyridin-3-yl]-N'-(2,6-diisopropylphenyl)urea
N-[1-isobutyl-4-[3-{3-(1,2,4-triazol-1-yl)propoxy}phenyl]-1,2-dihydro-2-oxo-1,8-naphthyridin-3-yl]-N'-(2,6-diisopropylphenyl)urea
N-[1-(3-phenylpropyl)-4-{3-(3-pyridylmethoxy)phenyl}-1,2-dihydro-2-oxo-1,8-naphthyridin-3-yl]-N'-(2,6-diisopropylphenyl)urea
N-[1-(3-phenylpropyl)-4-{3-(2-piperidinoethoxy)phenyl}-1,2-dihydro-2-oxo-1,8-naphthyridin-3-yl]-N'-(2,6-diisopropylphenyl)urea
N-[1-(3-phenylpropyl)-4-{3-(2-morpholinoethoxy)phenyl}-1,2-dihydro-2-oxo-1,8-naphthyridin-3-yl]-N'-(2,6-diisopropylphenyl)urea
N-[1-(3-phenylpropyl)-4-[3-{3-(1,2,4-triazol-1-yl)propoxy}-phenyl]-1,2-dihydro-2-oxo-1,8-naphthyridin-3-yl]-N'-(2,6-diisopropylphenyl)urea
N-[1-(3-phenylpropyl)-4-{3-(3-diethylamino-1-propynyl)phenyl}-1,2-dihydro-2-oxo-1,8-naphthyridin-3-yl]-N'-(2,6-diisopropylphenyl)urea
N-[1-(3-phenylpropyl)-4-{3-(3-diethylaminopropyl)phenyl}-1,2-dihydro-2-oxo-1,8-naphthyridin-3-yl]-N'-(2,6-diisopropylphenyl)urea
N-[1-(3-phenylpropyl)-4-{3-(3-hydroxy- 1-propynyl)phenyl}-1,2-dihydro-2-oxo-1,8-naphthyridin-3-yl]-N'-(2,6-diisopropylphenyl)urea
N-[1-(3-phenylpropyl)-4-{3-(3-hydroxypropyl)phenyl}-1,2-dihydro-2-oxo-1,8-naphthyridin-3-yl]-N'-(2,6-diisopropylphenyl)urea
N-[1-(3-phenylpropyl)-4-{3-(2-diethylaminoethylthio)phenyl}-1,2-dihydro-2-oxo-1,8-naphthyridin-3-yl]-N'-(2,6-diisopropylphenyl)urea
N-[1-(3-acetylaminopropyl)-4-{3-(3-pyridylmethoxy)phenyl}-1,2-dihydro-2-oxo-1,8-naphthyridin-3-yl]-N'-(2,6-diisopropylphenyl)urea
N-[1-(3-acetylaminopropyl)-4-{3-(2-diethylaminoethoxy)phenyl}-1,2-dihydro-2-oxo-1,8-naphthyridin-3-yl]-N'-(2,6-diisopropylphenyl)urea
N-[1-(3-acetylaminopropyl)-4-{3-(2-piperidinoethoxy)phenyl}-1,2-dihydro-2-oxo-1,8-naphthyridin-3-yl]-N'-(2,6-diisopropylphenyl)urea
N-[1-(3-acetylaminopropyl)-4-(3-(3-diethylamino-1-propynyl)-phenyl}-1,2-dihydro-2-oxo-1,8-naphthyridin-3-yl]-N'-(2,6-diisopropylphenyl)urea
N-[1-(3-acetylaminopropyl)-4-{3-(3-diethylaminopropyl)phenyl}-1,2-dihydro-2-oxo-1,8-naphthyridin-3-yl]-N'-(2,6-diisopropylphenyl)urea
N-[1-(3-acetylaminopropyl)-4-{3-(3-hydroxy-1-propynyl)phenyl}-1,2-dihydro-2-oxo-1,8-naphthyridin-3-yl]-N'-(2,6-diisopropylphenyl)urea
N-[1-(3-acetylaminopropyl)-4-{3-(3-hydroxypropyl)phenyl}-1,2-dihydro-2-oxo-1,8-naphthyridin-3-yl]-N'-(2,6-diisopropylphenyl)urea
N-[1-(3-hydroxypropyl)-4-{3-(2-pyridylmethoxy)phenyl}-1,2-dihydro-2-oxo-1,8-naphthyridin-3-yl]-N'-(2,6-diisopropylphenyl)urea
N-[1-(3-hydroxypropyl)-4-{3-(3-pyridylmethoxy)phenyl}-1,2-dihydro-2-oxo-1,8-naphthyridin-3-yl]-N'-(2,6-diisopropylphenyl)urea
N-[1-(3-hydroxypropyl)-4-{3-(2-diethylaminoethoxy)phenyl}-1,2-dihydro-2-oxo-1,8-naphthyridin-3-yl]-N'-(2,6-diisopropylphenyl)urea
N-[1-(3-hydroxypropyl)-4-{3-(3-diethylaminopropoxy)phenyl}-1,2-dihydro-2-oxo-1,8-naphthyridin-3-yl]-N'-(2,6-diisopropylphenyl)urea
N-[1-(3-hydroxypropyl)-4-{3-(3-piperidinopropoxy)phenyl}-1,2-dihydro-2-oxo-1,8-naphthyridin-3-yl]-N'-(2,6-diisopropylphenyl)urea
N-[1-(3-hydroxypropyl)-4-[3-{3-(1,2,4-triazol-1-yl)propoxy}-phenyl]-1,2-dihydro-2-oxo-1,8-naphthyridin-3-yl]-N'-(2,6-diisopropylphenyl)urea
N-[1-(3-hydroxypropyl)-4-(3-(3-diethylamino-1-propynyl)-phenyl}-1,2-dihydro-2-oxo-1,8-naphthyridin-3-yl]-N'-(2,6-diisopropylphenyl)urea
N-[1-(3-hydroxypropyl)-4-{3-(3-diethylaminopropyl)phenyl]-}-1,2-dihydro-2-oxo-1,8-naphthyridin-3-yl]-N'-(2,6-diisopropylphenyl)urea
N-[1-(3-hydroxypropyl)-4-{3-(3-hydroxy-1-propynyl)phenyl}-1,2-dihydro-2-oxo-1,8-naphthyridin-3-yl]-N'-(2,6-diisopropylphenyl)urea
N-[1-(3-hydroxypropyl)-4-{3-(3-hydroxypropyl)phenyl}-1,2-dihydro-2-oxo-1,8-naphthyridin-3-yl]-N'-(2,6-diisopropylphenyl)urea
N-[1-(3-butenyl)-4-{3-(2-diethylaminoethoxy)phenyl}-1,2-dihydro-2-oxo-1,8-naphthyridin-3-yl]-N'-(2,6-diisopropylphenyl)urea
N-[1-(3-butenyl)-4-{3-(3-pyridylmethoxy)phenyl}-1,2-dihydro-2-oxo-1,8-naphthyridin-3-yl)-N'-(2,6-diisopropylphenyl)urea
N-[1-(3-butenyl)-4-[3-{3-(1,2,4-triazol-1-yl)propoxy}phenyl]-1,2-dihydro-2-oxo-1,8-naphthyridin-3-yl]-N'-(2,6-diisopropylphenyl)urea
N-[1-(3-methyl-2-butenyl)-4-{3-(2-diethylaminoethoxy)phenyl}-1,2-dihydro-2-oxo-1,8-naphthyridin-3-yl]-N'-(2,6-diisopropylphenyl)urea
N-[1-(3-methyl-2-butenyl)-4-{3-(3-pyridylmethoxy)phenyl}-1,2-dihydro-2-oxo-1,8-naphthyridin-3-yl]-N'-(2,6-diisopropylphenyl)urea
N-[1-(3-methyl-2-butenyl)-4-[3-{3-(1,2,4-triazol-1-yl)propoxy}-phenyl]-1,2-dihydro-2-oxo-1,8-naphthyridin-3-yl]-N'-(2,6-diisopropylphenyl)urea
N-[1-(3-methylbutyl)-4-{3-(2-pyridylmethoxy)phenyl}-1,2-dihydro-2-oxo-1,8-naphthyridin-3-yl]-N'-(2,6-diisopropylphenyl)urea
N-[1-(3-methylbutyl)-4-{3-(3-pyridylmethoxy)phenyl}-1,2-dihydro-2-oxo-1,8-naphthyridin-3-yl]-N'-(2,6-diisopropylphenyl)urea
N-[1-(3-methylbutyl)-4-{3-(2-diethylaminoethoxy)phenyl}-1,2-dihydro-2-oxo-1,8-naphthyridin-3-yl]-N'-(2,6-diisopropylphenyl)urea
N-[1-(3-methylbutyl)-4-{3-(2-piperidinoethoxy)phenyl}-1,2-dihydro-2-oxo-1,8-naphthyridin-3-yl]-N'-(2,6-diisopropylphenyl)urea
N-[1-(3-methylbutyl)-4-{3-(2-morpholinoethoxy)phenyl}-1,2-dihydro-2-oxo-1,8-naphthyridin-3-yl]-N'-(2,6-diisopropylphenyl)urea
N-[1-(3-methylbutyl)-4-{3-(3-diethylaminopropoxy)phenyl}-1,2-dihydro-2-oxo-1,8-naphthyridin-3-yl]-N'-(2,6-diisopropylphenyl)urea
N-[1-(3-methylbutyl)-4-{3-(3-piperidinopropoxy)phenyl}-1,2-dihydro-2-oxo-1,8-naphthyridin-3-yl]-N'-(2,6-diisopropylphenyl)urea
N-[1-(3-methylbutyl)-4-[3-{3-(1,2,4-triazol-1-yl)propoxy}phenyl]-1,2-dihydro-2-oxo-1,8-naphthyridin-3-yl]-N'-(2,6-diisopropylphenyl)urea
N-[1-(3-methylbutyl)-4-{3-(3-diethylamino-1-propynyl)phenyl}-1,2-dihydro-2-oxo-1,8-naphthyridin-3-yl]-N'-(2,6-diisopropylphenyl)urea
N-[1-(3-methylbutyl)-4-{3-(3-diethylaminopropyl)phenyl}-1,2-dihydro-2-oxo-1,8-naphthyridin-3-yl]-N'-(2,6-diisopropylphenyl)urea
N-[1-(3-methylbutyl)-4-{3-(3-hydroxy-1-propynyl)phenyl}-1,2-dihydro-2-oxo-1,8-naphthyridin-3-yl]-N'-(2,6-diisopropylphenyl)urea
N-[1-(3-methylbutyl)-4-{3-(3-hydroxypropyl)phenyl}-1,2-dihydro-2-oxo-1,8-naphthyridin-3-yl]-N'-(2,6-diisopropylphenyl)urea
N-[1-(3-methylbutyl)-4-{3-(2-diethylaminoethylthio)phenyl}-1,2-dihydro-2-oxo-1,8-naphthyridin-3-yl]-N'-(2,6-diisopropylphenyl)urea
N-[1-(4-pentenyl)-4-{3-(2-pyridylmethoxy)phenyl}-1,2-dihydro-2-oxo-1,8-naphthyridin-3-yl]-N'-(2,6-diisopropylphenyl)urea
N-[1-(4-pentenyl)-4-{3-(3-pyridylmethoxy)phenyl}-1,2-dihydro-2-oxo-1,8-naphthyridin-3-yl]-N'-(2,6-diisopropylphenyl)urea
N-[1-(4-pentenyl)-4-{3-(2-diethylaminoethoxy)phenyl}-1,2-dihydro-2-oxo-1,8-naphthyridin-3-yl]-N'-(2,6-diisopropylphenyl)urea
N-[1-(4-pentenyl)-4-{3-(2-piperidinoethoxy)phenyl}-1,2-dihydro-2-oxo-1,8-naphthyridin-3-yl]-N'-(2,6-diisopropylphenyl)urea
N-[1-(4-pentenyl)-4-{3-(2-(1-pyrrolidinyl)ethoxy)phenyl}-1,2-dihydro-2-oxo-1,8-naphthyridin-3-yl]-N'-(2,6-diisopropylphenyl)urea
N-[1-(4-pentenyl)-4-{3-(2-morpholinoethoxy)phenyl}-1,2-dihydro-2-oxo-1,8-naphthyridin-3-yl]-N'-(2,6-diisopropylphenyl)urea
N-[1-(4-pentenyl)-4-{3-(3-dimethylaminopropoxy)phenyl}-1,2-dihydro-2-oxo-1,8-naphthyridin-3-yl]-N'-(2,6-diisopropylphenyl)urea
N-[1-(4-pentenyl)-4-{3-(3-diethylaminopropoxy)phenyl}-1,2-dihydro-2-oxo-1,8-naphthyridin-3-yl]-N'-(2,6-diisopropylphenyl)urea
N-[1-(4-pentenyl)-4-{3-(3-piperidinopropoxy)phenyl}-1,2-dihydro-2-oxo-1,8-naphthyridin-3-yl]-N'-(2,6-diisopropylphenyl)urea
N-[1-(4-pentenyl)-4-[3-{3-(1,2,4-triazol-1-yl)propoxy}phenyl]-1,2-dihydro-2-oxo-1,8-naphthyridin-3-yl]-N'-(2,6-diisopropylphenyl)urea
N-[1-(4-pentenyl)-4-{3-(3-diethylamino-1-propynyl)phenyl}-1,2-dihydro-2-oxo-1,8-naphthyridin-3-yl]-N'-(2,6-diisopropylphenyl)urea
N-[1-(4-pentenyl)-4-{3-(3-diethylaminopropyl)phenyl}-1,2-dihydro-2-oxo-1,8-naphthyridin-3-yl]-N'-(2,6-diisopropylphenyl)urea
N-[1-(4-pentenyl)-4-{3-(3-hydroxy-1-propynyl)phenyl}-1,2-dihydro-2-oxo-1,8-naphthyridin-3-yl]-N'-(2,6-diisopropylphenyl)urea
N-[1-(4-pentenyl)-4-{3-(3-hydroxypropyl)phenyl}-1,2-dihydro-2-oxo-1,8-naphthyridin-3-yl]-N'-(2,6-diisopropylphenyl)urea
N-[1-(4-pentenyl)-4-{3-(2-diethylaminoethylthio)phenyl}-1,2-dihydro-2-oxo-1,8-naphthyridin-3-yl]-N'-(2,6-diisopropylphenyl)urea
N-[1-(4-pentynyl)-4-{3-(2-diethylaminoethoxy)phenyl}-1,2-dihydro-2-oxo-1,8-naphthyridin-3-yl]-N'-(2,6-diisopropylphenyl)urea
N-[1-(4-pentynyl)-4-{3-(3-pyridylmethoxy)phenyl}-1,2-dihydro-2-oxo-1,8-naphthyridin-3-yl]-N'-(2,6-diisopropylphenyl)urea
N-[1-(4-pentynyl)-4-[3-{3-(1,2,4-triazol-1-yl)propoxy}phenyl]-1,2-dihydro-2-oxo-1,8-naphthyridin-3-yl]-N'-(2,6-diisopropylphenyl)urea
N-[1-(4-methylpentyl)-4-{3-(2-diethylaminoethoxy)phenyl}-1,2-dihydro-2-oxo-1,8-naphthyridin-3-yl]-N'-(2,6-diisopropylphenyl)urea
N-[1-(4-methylpentyl)-4-{3-(3-pyridylmethoxy)phenyl}-1,2-dihydro-2-oxo-1,8-naphthyridin-3-yl]-N'-(2,6-diisopropylphenyl)urea
N-[1-(4-methylpentyl)-4-[3-{3-(4-pyridyl)propoxy}phenyl]-1,2-dihydro-2-oxo-1,8-naphthyridin-3-yl]-N'-(2,6-diisopropylphenyl)urea
N-[1-(4-methylpentyl)-4-[3-{3-(1,2,4-triazol-1-yl)propoxy}-phenyl]-1,2-dihydro-2-oxo-1,8-naphthyridin-3-yl]-N'-(2,6-diisopropylphenyl)urea
N-[1-methyl-4-[3-{3-(1-pyrrolidinyl)-1-propynyl}phenyl]-1,2-dihydro-2-oxo-1,8-naphthyridin-3-yl]-N'-(2,6-diisopropylphenyl)urea
N-[1-(2-pyridylmethyl)-4-[3-{3-(1-pyrrolidinyl)-1-propynyl}-phenyl]-1,2-dihydro-2-oxo-1,8-naphthyridin-3-yl]-N'-(2,6-diisopropylphenyl)urea
N-[1-(3-pyridymethyl)-4-[3-{3-(1-pyrrolidinyl)-1-propynyl}-phenyl]-1,2-dihydro-2-oxo-1,8-naphthyridin-3-yl]-N'-(2,6-diisopropylphenyl)urea
N-[1-(4-pyridylmethyl)-4-[3-{3-( 1-pyrrolidinyl)-1-propynyl}-phenyl]-1,2-dihydro-2-oxo-1,8-naphthyridin-3-yl]-N'-(2,6-diisopropylphenyl)urea
N-[1-ethyl-4-[3-{3-(1-pyrrolidinyl)- 1-propynyl}phenyl]-1,2-dihydro-2-oxo-1,8-naphthyridin-3-yl]-N'-(2,6-diisopropylphenyl)urea
N-[1-propyl-4-[3-{3-(1-pyrrolidinyl)-1-propynyl}phenyl]-1,2-dihydro-2-oxo-1,8-naphthyridin-3-yl]-N'-(2,6-diisopropylphenyl)urea
N-(1-isobutyl-4-[3-{3-(1-pyrrolidinyl)-1-propynyl}phenyl]-1,2-dihydro-2-oxo-1,8-naphthyridin-3-yl]-N'-(2,6-diisopropylphenyl)urea
N-[1-pentyl-4-[3-{3-(1-pyrrolidinyl)-1-propynyl}phenyl]-1,2-dihydro-2-oxo-1,8-naphthyridin-3-yl]-N'-(2,6-diisopropylphenyl)urea
N-[1-(3-acetylaminopropyl)-4-[3-{3-(1-pyrrolidinyl)-1-propynyl}-phenyl]-1,2-dihydro-2-oxo-1,8-naphthyridin-3-yl]-N'-(2,6-diisopropylphenyl)urea
N-[1-(3-hydroxypropyl)-4-[3-{3-(1-pyrrolidinyl)-1-propynyl}-phenyl]-1,2-dihydro-2-oxo-1,8-naphthyridin-3-yl]-N'-(2,6-diisopropylphenyl)urea

### BEST MODE FOR CARRYING OUT THE INVENTION

The present invention is illustrated by Reference Examples and Examples, but should not be construed to be limited thereto.

### Reference Example 1

Preparation of 4-(2-chlorophenyl)-1,2-dihydro-1-methyl-2-oxo-1,8-naphthyridine-3-carboxylic acid

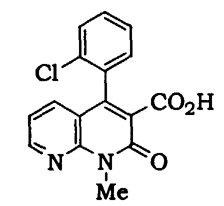

### (a) separation of ethyl 4-(2-chlorophenyl)-1,2-dihydro-2-oxo-1,8-naphthyridine-3-carboxylate

A mixture of 2-amino-3-(2-chlorobenzoyl)pyridine (3.91 g, 16.8 mmol), diethyl malonate (4.04 g, 25.2 mmol) and pyridine (0.33 g, 4.2 mmol) was heated with stirring at about 170°C for five hours. After allowed to stand for cooling, the precipitated crystals were recrystallized from ethanol to give the title compound (4.73 g, 14.4 mmol) as a colorless crystal.
m.p. 218-221°C
¹H-NMR δ (CDCl₃) 11.53 (1H, brs), 8.76 (1H, dd, J=5.0Hz, 1.32Hz), 7.26-7.57 (5H, m), 7.17 (1H, dd, J=7.9Hz, 5.0Hz), 4.04-4.17 (2H, m), 0.97 (3H, t, J=7.0Hz)
IR (KBr) 1739, 1667, 1613, 1568, 1466, 1425, 1375 cm⁻¹

### (b) Preparation of ethyl 1-methyl-4-(2-chlorophenyl)-1,2-dihydro-2-oxo-1,8-naphthyridine-3-carboxylate

To a solution of ethyl 4-(2-chlorophenyl)-1,2-dihydro-2-oxo-1,8-naphthyridine-3-carboxylate (4.50, 13.7 mmol) in N,N-dimethylformamide (50 ml) was added sodium hydride (60 % oily, 547 mg, 13.7 mg) at room temperature, and the mixture was stirred for 0.5 hour. To the mixture was added methyl iodide (1.9 g, 13.7 mmol) at 0°C to 5°C, and the mixture was stirred at the same temperature for 0.5 hour, and then stirred at room temperature for five hours. The mixture was poured into water, and extracted with ethyl acetate. The extract was washed with water, washed with a saturated aqueous sodium chloride solution, dried over anhydrous sodium sulfate, and concentrated under reduced pressure to give the title compound (4.60 g, 13.4 mmol), which was used without further isolation in the subsequent reaction.
¹H-NMR δ (CDCl₃) 8.65 (1H, dd, J=4.6Hz, 1.7Hz), 7.29-7.56 (5H, m), 7.10-7.15 (1H, m), 4.07-4.13 (2H, m), 3.92 (3H, s), 0.98 (3H, t, J=7.0Hz)

### (c) Preparation of 1-methyl-4-(2-chlorophenyl)-1,2-dihydro-2-oxo-1,8-naphthyridine-3-carboxylic acid

To a solution of ethyl 1-methyl-4-(2-chlorophenyl)-1,2-dihydro-2-oxo-1,8-naphthyridine-3-carboxylate (4.6 g, 13.4 mmol) in ethanol (20 ml) was added sodium hydroxide (2.1 g, 52.5 mmol), and the mixture was refluxed for 0.5 hour. The mixture was diluted with water, and the pH value thereof was adjusted to pH 4 with 2N aqueous hydrochloric acid. The mixture was extracted with ethyl acetate, and the extract was washed with a saturated aqueous sodium chloride solution, dried over anhydrous sodium sulfate, and concentrated under reduced pressure. The precipitated crystals were recrystallized from ethyl acetate to give the title compound (3.52 g, 11.2 mmol) as a colorless crystal.
m.p. 178- 180°C
¹H-NMR δ (CDCl₃) 8.80 (1H, dd, J=4.6Hz, 2.0Hz), 7.39-7.57 (4H, m), 7.24-7.29 (1H, dd, J=7.9Hz, 4.6Hz), 7.11 (1H, dd, J=7.9Hz, 2.0Hz), 4.07 (3H, s)
IR (KBr) 1747, 1612, 1576, 1472, 1446, 1342 cm⁻¹

### Reference Example 2

### Preparation of 4-(3-methoxyphenyl)-1,2-dihydro-2-oxo-1,8-naphthyridine-3-carboxylic acid

The title compound was obtained in the same manner as in Reference Example 1.
¹H-NMR δ (CDCl₃) 8.84 (1H, d, J=3.0Hz), 7.69 (1H, d, J=8.2Hz), 7.46 (1H, dd, J=7.9Hz, 7.9Hz), 7.28-7.33 (1H, m), 7.05 (1H, dd, J=8.3Hz, 1.7Hz), 6.73-6.80 (2H, m), 3.84 (3H, s)

### Reference Example 3

### Preparation of 1-butyl-4-(3-methoxyphenyl)-1,2-dihydro-2-oxo-1,8-naphthyridine-3-carboxylic acid

The title compound was obtained in the same manner as in Reference Example 1.
¹H-NMR δ (CDCl₃) 8.76 (1H, dd, J=4.6Hz, 2.0Hz), 7.65 (1H, dd, J=7.9Hz, 2.0Hz), 7.43 (1H, dd, J=7.9Hz, 7.9Hz), 7.22 (1H, dd, J=7.9Hz, 4.6Hz), 7.02 (1H, dd, J=7.6Hz, 1.6Hz), 6.70-6.78 (2H, m), 4.68-4.74 (2H, m), 3.82 (3H, s), 1.77-1.88 (2H, m), 1.45-1.59 (2H, m), 1.03 (3H, t, J=7.3Hz)

### Reference Example 4

### Preparation of 1-pentyl-4-(3-methoxyphenyl)-1,2-dihydro-2-oxo-1,8-naphthyridine-3-carboxylic acid

The title compound was obtained in the same manner as in Reference Example 1.
¹H-NMR δ (CDCl₃) 8.76 (1H, dd, J=4.6Hz, 1.7Hz), 7.64 (1H, dd, J=8.3Hz, 1.7Hz), 7.42 (1H, dd, J=7.9Hz, 7.9Hz), 7.22 (1H, dd, J=8.3Hz, 4.6Hz), 7.02 (1H, dd, J=7.9Hz, 2.3Hz), 6.73 (1H, d, J=7.9Hz), 6.71 (1H, s), 4.70 (2H, t, J=7.6Hz), 1.84 (2H, br), 1.46 (4H, br), 0.95 (3H, t, J=6.9Hz)

### Reference Example 5

### Preparation of 1-(3-methylbutyl)-4-(3-methoxyphenyl)-1,2-dihydro-2-oxo-1,8-naphthyridine-3-carboxylic acid

The title compound was obtained in the same manner as in Reference Example 1.
¹H-NMR δ (CDCl₃) 8.76 (1H, dd, J=4.6Hz, 2.0Hz), 7.64 (1H, dd, J=8.3Hz, 2.0Hz), 7.42 (1H, dd, J=7.9Hz, 7.9Hz), 7.22 (1H, dd, J=8.3Hz, 4.6Hz), 7.02 (1H, dd, J=8.3Hz, 2.0Hz), 6.76 (1H, d, J=7.6Hz), 6.71 (1H, d, J=2.0Hz), 4.73 (2H, t, J=7.9Hz), 3.82 (3H, s), 1.67-1.84 (3H, m), 1.06 (6H, d, J=6.6Hz)

### Reference Example 6

### Preparation of 1-(3-benzyloxypropyl)-4-(3-methoxyphenyl)-1,2-dihydro-2-oxo-1,8-naphthyridine-3-carboxylic acid

The title compound was obtained in the same manner as in Reference Example 1.
¹H-NMR δ (CDCl₃) 8.74 (1H, dd, J=4.6Hz, 1.7Hz), 7.62 (1H, dd, J=7.9Hz, 1.7Hz), 7.43 (1H, dd, J=7.9Hz, 7.9Hz), 7.20-7.37 (5H, m), 7.21 (1H, dd, J=7.9Hz, 4.6Hz), 7.02 (1H, dd, J=7.9Hz, 2.6Hz), 6.60-6.75 (2H, m), 4.87 (2H, t, J=7.3Hz), 4.51 (2H, s), 3.82 (3H, s), 3.69 (2H, t, J=5.9Hz), 2.19 (2H, dd, J=7.3Hz, 5.9Hz)

### Reference Example 7

### Preparation of 1-butyl-4-(3-bromophenyl)-1,2-dihydro-2-oxo-1,8-naphthyridine-3-carboxylic acid

The title compound was obtained in the same manner as in Reference Example 1.
¹H-NMR δ (CD₃Cl₃) 8.78 (1H, dd, J=4.6Hz, 1.7Hz), 7.56-7.64 (2H, m), 7.39 (1H, dd, J=7.9Hz, 7.9Hz), 7.33 (1H, dd, J=2.0Hz, 1.7Hz), 7.23-7.28 (1H, m), 7.13 (1H, d, J=7.69Hz), 4.72 (2H, t, J=7.6Hz), 1.77-1.88 (2H, m), 1.45-1.58 (2H, m), 1.03 (3H, t, J=7.3Hz)

### Reference Example 8

### Preparation of 1-butyl-4-(4-bromophenyl)-1,2-dihydro-2-oxo-1,8-naphthyridine-3-carboxylic acid

The title compound was obtained in the same manner as in Reference Example 1.
m.p. 158-160°C

### Reference Example 9

### Preparation of 1-butyl-4-(3-fluorophenyl)-1,2-dihydro-2-oxo-1,8-naphthyridine-3-carboxylic acid

The title compound was obtained in the same manner as in Reference Example 1.
¹H-NMR δ (DMSO-d₆) 13.28 (1H, brs), 8.71 (1H, dd, J=4.6Hz, 1.7Hz), 7.56-7.64 (2H, m), 7.21-7.40 (4H, m), 4.47 (2H, t, J=7.3Hz), 1.69 (2H, m), 1.39 (2H, m), 0.96 (3H, t, J=7.3Hz)

### Reference Example 10

### Preparation of N-[1-methyl-4-(2-chlorophenyl)-1,2-dihydro-2-oxo-1,7-naphthyridin-3-yl]-N'-(2,4,6-trimethylphenyl)urea

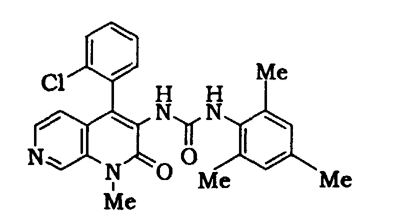

A solution of 1-methyl-4-(2-chlorophenyl)-1,2-dihydro-2-oxo-1,7-naphthyridine-3-carboxylic acid (315 mg, 1 mmol), diphenylphosphoryl azide (330 mg, 1.2 mmol) and triethylamine (101 mg, 1 mmol) in N,N-dimethylformamide (DMF, 5 ml) was stirred at room temperature for 0.5 hour, and stirred at 80-90°C for 0.5 hour. After allowed to stand for cooling, to the mixture was added 2,4,6-trimethylaniline (162 mg, 1.2 mmol), and the mixture was stirred at room temperature for 0.5 hour, and then stirred at 80-90°C for two hours. After allowed to stand for cooling, the mixture was diluted with ethyl acetate, washed with water, washed with a saturated aqueous sodium chloride solution, dried over anhydrous magnesium sulfate, and concentrated under reduced pressure. The resulting solid was recrystallized from ethanol to give the title compound (350 mg, 0.78 mmol) as a colorless crystal.
m.p. 222-224°C
¹H-NMR δ (CDCl₃) 8.83 (1H, s), 8.36 (1H, d, J=5.3Hz), 7.50-7.54 (1H, m), 7.38-7.43 (3H, m), 7.02 (1H, d, J=5.3Hz), 6.93 (1H, brs), 6.62 (0.5H, br), 5.68 (0.5H, br), 3.86 (3H, brs), 2.27 (6H, brs), 2.05 (3H, brs)
IR (KBr) 1658, 1638, 1545, 1432 cm⁻¹

### Reference Example 11

### Preparation of N-( 1-methyl-4-(3-hydroxyphenyl)- 1,2-dihydro-2-oxo-1,8-naphthyridin-3-yl)-N'-(2,6-diisopropylphenyl)urea

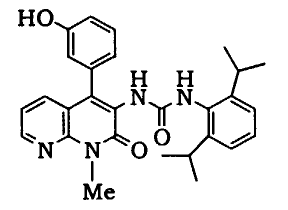

To a solution of N-[1-methyl-4-(3-methoxyphenyl)-1,2-dihydro-2-oxo-1,8-naphthyridin-3-yl]-N'-(2,6-diisopropylphenyl)urea (1310 mg, 2.7 mmol) in methylene chloride (20 ml) was added dropwise boron tribromide (1.7 g, 6.75 mmol) at 0°C, and the mixture was stirred for 6 hours. The mixture was poured into a saturated aqueous sodium hydrogen carbonate solution, and the mixture was extracted with methylene chloride. The extract was washed with water, washed with a saturated aqueous sodium chloride solution, and dried over anhydrous magnesium sulfate. The residue was concentrated under reduced pressure, purified by silica gel column chromatography (3 % methanol in chloroform), and crystallized from diethyl ether/hexane to give the title compound (830 mg, 1.76 mmol) as a colorless powder.
m.p. 152-155°C

### Reference Example 12

### Preparation of N-[1-butyl-4-{3-(3-dimethylaminopropoxy)phenyl}-1,2-dihydro-2-oxo-1,8-naphthyridin-3-yl]-N'-(2,6-diisopropylphenyl)urea

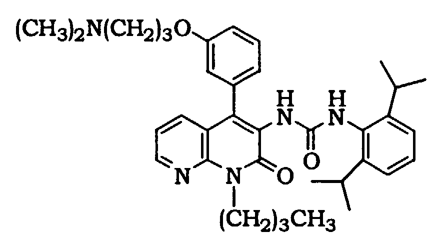

To a suspension of N-{1-butyl-4-(3-hydroxyphenyl)-1,2-dihydro-2-oxo-1,8-naphthyridin-3-yl}-N'-(2,6-diisopropylphenyl)urea (200 mg, 0.4 mmol), potassium carbonate (166 mg, 1.2 mmol), and sodium iodide (5 mg) in DMF (10 ml) was added 3-dimethylaminopropyl chloride hydrochloride (63 mg) at room temperature, and the mixture was stirred at 60-70°C for 10 hours. After allowed to stand for cooling, the mixture was poured into water, extracted with ethyl acetate, washed with water, washed with a saturated aqueous sodium chloride solution, and dried over anhydrous magnesium sulfate. The residue was concentrated under reduced pressure, and purified by silica gel column chromatography (10 % methanol in chloroform) to give the title compound (88 mg, 0.15 mmol).
¹H-NMR δ (DMSO-d₆) 8.59 (1H, d, J=3.3Hz), 7.76 (1H, s), 7.74 (1H, s), 7.61 (1H, d, J=6.6Hz), 7.38 (1H, dd, J=7.6Hz, 7.6Hz), 7.12-7.26 (2H, m), 6.98-7.04 (3H, m), 6.85-6.91 (2H, m), 4.52 (2H, br), 3.99 (2H, brt, J=6.9Hz), 2.85-2.95 (2H, m), 2.38 (2H, t, J=6.9Hz), 1.82-1.91 (2H, m), 1.65-1.75 (2H, m), 1.37-1.47 (2H, m), 0.95-1.00 (15H, m)

### Reference Example 13

### Preparation of N-[1-(3-phthalimidopropyl)-4-(3-methoxyphenyl)-1,2-dihydro-2-oxo-1,8-naphthyridin-3-yl]-N'-(2,6-diisopropylphenyl)urea

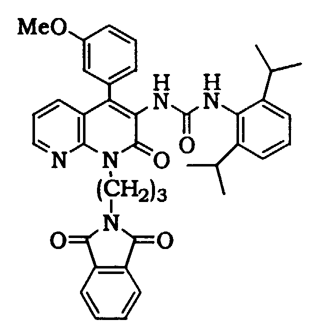

To a solution of N-[4-(3-methoxyphenyl)-1,2-dihydro-2-oxo-1,8-naphthyridin-3-yl]-N'-(2,6-diisopropylphenyl)urea (200 mg, 0.43 mmol) and N-(3-bromopropyl)phthalimide (133 mg, 0.50 mmol) in DMF (10 ml) was added potassium carbonate (114 mg, 0.83 mmol), and the mixture was stirred at 50-60°C for one hour. After allowed to stand for cooling, the mixture was poured into water, and the mixture was extracted with ethyl acetate. The extract was washed with water, washed with a saturated aqueous sodium chloride solution, and dried over anhydrous magnesium sulfate. The residue was concentrated under reduced pressure, purified by silica gel column chromatography (1-3 % methanol in chloroform), and crystallized from hexane to give the title compound (229 mg, 0.35 mmol).
¹H-NMR δ (CD₃OD) 8.40 (1H, dd, J=4.6Hz, 1.7Hz), 7.78-7.96 (4H, m), 7.72 (1H, dd, J=7.9Hz, 1.7Hz), 7.48 (1H, dd, J=8.2Hz, 7.9Hz), 7.04-7.27 (5H, m), 6.93 7.02 (2H, m), 4.66-4.78 (2H, m), 3.90 (2H, t, J=6.9Hz), 3.87 (3H, s), 3.02 (2H, sept, J=6.6Hz), 2.17-2.35 (2H, m), 1.13 (12H, brd, J=6.6Hz)

### Reference Example 14

### Preparation of N-[1-(3-hydroxypropyl)-4-(3-methoxyphenyl)-1,2-dihydro-2-oxo-1,8-naphthyridin-3-yl]-N'-(2,6-diisopropylphenyl)urea

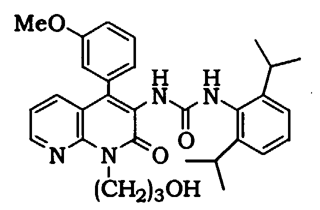

To a solution of N-[1-(3-benzyloxypropyl)-4-(3-methoxyphenyl)-1,2-dihydro-2-oxo-1,8-naphthyridin-3-yl]-N'-(2,6-diisopropylphenyl)urea (1.31 g, 2.12 mmol) in ethanol (80 ml) was added 10 % palladiumcarbon (150 mg), and the mixture was stirred at room temperature under hydrogen atmosphere for three hours. To the mixture was added 12N hydrochloric acid (1 ml), and the mixture was further stirred under hydrogen atmosphere for two hours, and then further stirred at room temperature for 3 hours. The mixture was filtered through a cerite pad, and the filtrate was concentrated under reduced pressure. The residue was purified by silica gel column chromatography (1-3 % methanol in chloroform) to give the title compound (1.12 g, 2.12 mmol).
¹H-NMR δ (CD₃OD) 8.65 (1H, dd, J=4.6Hz, 1.7Hz), 7.79 (1H, dd, J=7.9Hz, 1.7Hz), 7.49 (1H, dd, J=8.2Hz, 8.2Hz), 7.05-7.35 (5H, m), 6.95-7.04 (2H, m), 4.79 (2H, t, J=7.3Hz), 3.87 (3H, s), 3.71 (2H, t, J=6.3Hz), 3.03 (2H, sept, J=6.3Hz), 2.10 (2H, m), 1.15 (12H, brd, J=6.3Hz)

### Example 1

### Preparation of N-[1-(3-methylbutyl)-4-(3-hydroxyphenyl)-1,2-dihydro-2-oxo-1,8-naphthyridin-3-yl]-N'-(2,6-diisopropylphenyl)urea

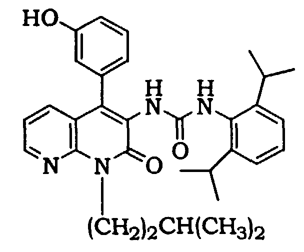

The title compound was obtained in the same manner as in Reference Example 11 from N-[1-(3-methylbutyl)-4-(3-methoxyphenyl)-1,2-dihydro-2-oxo-1,8-naphthyridin-3-yl]-N'-(2,6-diisopropylphenyl)urea.
¹H-NMR δ (DMSO-d₆) 9.52 (1H, s), 7.71 (1H, d, J=6.3Hz), 7.43 (1H, dd, J=8.3Hz, 7.9Hz), 7.71 (2H, brs), 7.63 (1H, dd, J=7.9Hz, 1.7Hz), 7.23-7.30 (2H, m), 7.15 (1H, t, J=7.3Hz), 7.04 (2H, d, J=7.3Hz), 6.85 (1H, dd, J=7.9Hz, 1.7Hz), 6.72-6.77 (3H, m), 4.54 (2H, t, J=7.3Hz), 2.95 (2H, sep, J=6.6Hz), 1.59-1.74 (3H, m), 0.99-1.05 (18H, m)

### Example 2

### Preparation of N-[1-(3-methylbutyl)-4-{3-(2-diethylaminoethoxy)phenyl}-1,2-dihydro-2-oxo-1,8-naphthyridin-3-yl]-N'-(2,6-diisopropylphenyl)urea

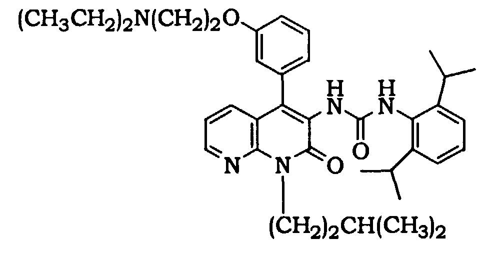

The title compound was obtained in the same manner as in Reference Example 12 from N-[ 1-(3-methylbutyl)-4-(3-hydroxyphenyl)-1,2-dihydro-2-oxo-1,8-naphthyridin-3-yl]-N'-(2,6-diisopropylphenyl)urea and 2-diethylaminoethyl chloride hydrochloride.
¹H-NMR δ (CD₃OD) 8.59 (1H, brs), 7.71 (1H, d, J=6.3Hz), 7.43 (1H, dd, J=8.3Hz, 7.9Hz), 7.05-7.20 (5H, m), 6.97 (2H, brs), 4.64 (2H, m), 4.13 (2H, m), 2.90-3.01 (4H, m), 2.63-2.69 (2H, m), 1.69-1.80 (3H, m), 1.03-1.11 (24H, m)

### Example 3

### Preparation of N-[1-isobutyl-4-(3-methoxyphenyl)-1,2-dihydro-2-oxo-1,8-naphthyridin-3-yl]-N'-(2,6-diisopropylphenyl)urea

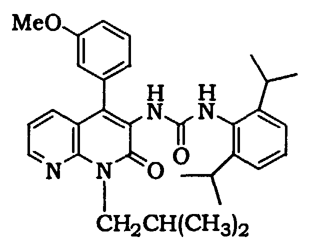

To a solution of N-[4-(3-methoxyphenyl)-1,2-dihydro-2-oxo-1,8-naphthyridin-3-yl]-N'-(2,6-diisopropylphenyl)urea (100 mg, 0.21 mmol) in DMF (5 ml) were added potassium carbonate (1.39 g, 10.1 mmol), potassium iodide (10 mg, 0.07 mmol) and 1-bromo-2-methylpropane (58 mg, 0.43 mmol), and the mixture was stirred at room temperature for five hours. The mixture was poured into water, and the mixture was extracted with ethyl acetate. The extract was washed with water, washed with a saturated aqueous sodium chloride solution, and dried over anhydrous magnesium sulfate. The resultant was concentrated under reduced pressure, and the residue was purified by silica gel column chromatography (1-3 % methanol in chloroform) to give the title compound (71 mg, 0.13 mmol).
¹H-NMR δ (CD₃OD) 8.61 (1H, dd, J=4.6Hz, 1.7Hz), 7.76 (1H, dd, J=7.9Hz, 1.7Hz), 7.49 (1H, dd, J=8.3Hz, 8.2Hz), 6.93-7.30 (7H, m), 4.56 (2H, d, J=7.6Hz), 3.87 (3H, s), 2.90-3.10 (2H, m), 2.30-2.52 (1H, m), 1.15 (12H, d, J=6.6Hz), 1.03 (6H, d, J=6.6Hz)

### Example 4

### Preparation of N-[1-(3-methyl-2-butenyl)-4-(3-methoxyphenyl)-1,2-dihydro-2-oxo-1,8-naphthyridin-3-yl]-N'-(2,6-diisopropylphenyl)urea

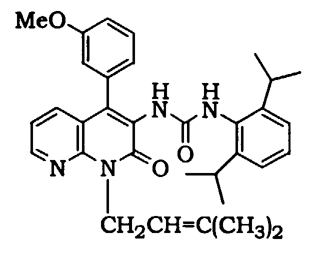

The title compound was obtained in the same manner as in Example 3 from N-[4-(3-methoxyphenyl)-1,2-dihydro-2-oxo-1,8-naphthyridin-3-yl]-N'-(2,6-diisopropylphenyl)urea and 4-bromo-2-methyl-2-butene.
¹H-NMR δ (CD₃OD) 8.63 (1H, dd, J=4.3Hz, 1.7Hz), 7.75 (1H, dd, J=7.9Hz, 1.7Hz), 7.49 (1H, dd, J=8.6Hz, 7.9Hz), 6.90-7.30 (7H, m), 5.36-5.50 (1H, m), 5.26-5.36 (2H, m), 3.87 (3H, s), 2.95-3.15 (2H, m), 2.01 (3H, s), 1.75 (3H, s), 1.15 (12H, d, J=6.6Hz)

### Example 5

### Preparation of N-[1-(4-methylpentyl)-4-(3-methoxyphenyl)-1,2-dihydro-2-oxo-1,8-naphthyridin-3-yl]-N'-(2,6-diisopropylphenyl)urea

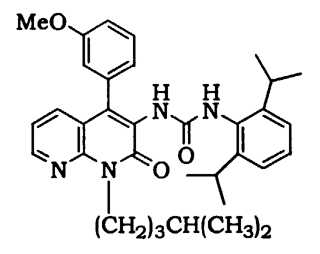

The title compound was obtained in the same manner as in Example 3 from N-[4-(3-methylphenyl)-1,2-dihydro-2-oxo-1,8-naphthyridin-3-yl]-N'-(2,6-diisopropylphenyl)urea and 1-bromo-4-methylpentane.
¹H-NMR δ (CD₃OD) 8.63 (1H, dd, J=4.6Hz, 1.7Hz), 7.75 (1H, dd, J=8.2Hz, 1.9Hz), 7.48 (1H, dd, J=8.2Hz, 7.9Hz), 6.90-7.30 (7H, m), 4.53-4.70 (2H, m), 3.87 (3H, s), 2.90-3.10 (2H, m), 1.76-1.95 (2H, m), 1.60-1.75 (1H, m), 1.30-1.50 (2H, m), 1.15 (12H, d, J=6.3Hz), 0.97 (6H, d, J=6.6Hz)

### Example 6

### Preparation of N-[1-butyl-4-[3-{2-(1-piperazinyl)ethoxy}phenyl]-1,2-dihydro-2-oxo- 1,8-naphthyridin-3-yl]-N'-(2,6-diisopropylphenyl)urea

### (a) Preparation of N-[1-butyl-4-[3-{2-(4-tert-butoxycarbonyl-1-piperazinyl)ethoxy}phenyl]-1,2-dihydro-2-oxo-1,8-naphthyridin-3-yl]-N'-(2,6-diisopropylphenyl)urea

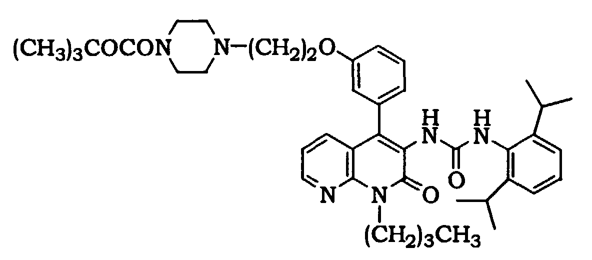

The title compound was obtained in the same manner as in Reference Example 12 from N-[4-(3-hydroxyphenyl)-1,2-dihydro-2-oxo-1,8-naphthyridin-3-yl]-N'-(2,6-diisopropylphenyl)urea and 1-tert-butoxycarbonyl-4-(2-chloroethyl)piperazine.
¹H-NMR δ (CD₃OD) 8.63 (1H, dd, J=4.6Hz, 1.7Hz), 7.75 (1H, dd, J=7.9Hz, 1.7Hz), 7.47 (1H, dd, J=8.2Hz, 7.9Hz), 7.04-7.30 (5H, m), 6.93-7.04 (2H, m), 4.60-4.73 (2H, m), 4.05-4.10 (2H, m), 3.38-3.53 (4H, m), 2.92-3.10 (2H, m), 2.55-2.68 (2H, m), 2.36-2.54 (4H, m), 1.95-2.10 (2H, m), 1.70-1.90 (2H, m), 1.45-1.62 (2H, m), 1.49 (9H, s), 1.15 (12H, d, J=6.6Hz), 1.05 (3H, t, J=7.3Hz)

### (b) Preparation of N-[1-butyl-4-[3-{2-(1-piperazinyl)ethoxy}phenyl]-1,2-dihydro-2-oxo-1,8-naphthyridin-3-yl]-N'-(2,6-diisopropylphenyl)urea

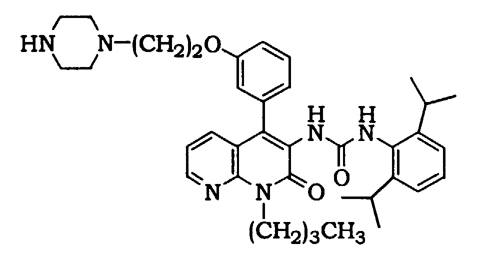

To a solution of N-[1-butyl-4-[3-{2-(4-tert-butoxycarbonyl-1-piperazinyl)ethoxy}phenyl]-1,2-dihydro-2-oxo-1,8-naphthyridin-3-yl]-N'-(2,6-diisopropylphenyl)urea (26 mg, 0.035 mmol) in methylene chloride (5 ml) was added trifluoroacetic acid (1 ml, 13 mmol), and the mixture was stirred at room temperature for two hours. The mixture was concentrated under reduced pressure to remove the solvent, and to the residue was added 5 % aqueous ammonia (50 ml), and the mixture was extracted with ethyl acetate. The extract was washed with water, washed with a saturated aqueous sodium chloride solution, and dried over anhydrous magnesium sulfate. The resultant was concentrated under reduced pressure, and the residue was purified by thin layer chromatography (20 % methanol in chloroform) to give the title compound (13 mg, 0.20 mmol).
¹H-NMR δ (CD₃OD) 8.65 (1H, dd, J=4.6Hz, 1.7Hz), 7.76 (1H, dd, J=7.9Hz, 1.7Hz), 7.48 (1H, dd, J=8.3Hz, 8.2Hz), 7.17-7.30 (2H, m), 7.04-7.16 (3H, m), 6.95-7.02 (2H, m), 4.60-4.73 (2H, m), 4.00-4.18 (2H, m), 2.98-3.10 (2H, m), 2.85-2.97 (4H, m), 2.40-2.68 (7H, m), 1.96-2.13 (2H, m), 1.72-1.92 (2H, m), 1.46-1.64 (2H, m), 1.15 (12H, brs, J=6.6Hz), 1.06 (3H, t, J=7.3Hz)

### Example 7

### Preparation of N-[1-butyl-4-(3-methoxyphenyl)-1,2-dihydro-2-oxo-1,8-naphthyridin-3-yl]-N'-(2,4,6-trifluorophenyl)urea

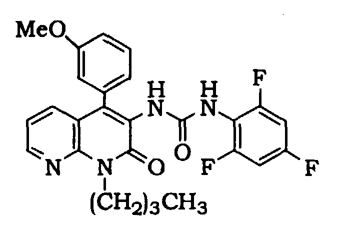

The title compound was obtained in the same manner as in Reference Example 10 from 1-butyl-4-(3-methoxyphenyl)-1,2-dihydro-2-oxo-1,8-naphthyridine-3-carboxylic acid and 2,4,6-trifluoroaniline.
m.p. 189- 190°C

### Example 8

### Preparation of N-[1-(4-pentenyl)-4-(3-methoxyphenyl)-1,2-dihydro-2-oxo-1,8-naphthyridin-3-yl]-N'-(2,6-diisopropylphenyl)urea

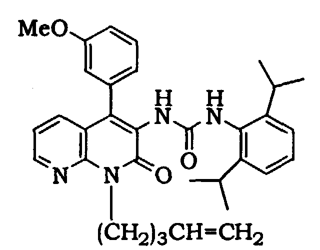

The title compound was obtained in the same manner as in Example 3 from N-[4-(3-methoxyphenyl)-1,2-dihydro-2-oxo-1,8-naphthyridin-3-yl]-N'-(2,6-diisopropylphenyl)urea and 5-bromo-1-pentene.
¹H-NMR δ (CD₃OD) 8.64 (1H, dd, J=4.6Hz, 1.7Hz), 7.77 (1H, dd, J=8.3Hz, 2.0Hz), 7.49 (1H, dd, J=8.3Hz, 7.9Hz), 7.05-7.30 (5H, m), 6.95-7.02 (2H, m), 5.85-6.05 (1H, m), 5.08-5.20 (1H, m), 4.96-5.09 (1H, m), 4.63-4.75 (2H, m), 3.87 (3H, s), 2.59-3.13 (2H, m), 2.16-2.37 (2H, m), 1.82-2.05 (2H, m), 1.15 (12H, brd, J=5.9Hz),

### Example 9

### Preparation of N-[1-(2-phenoxyethyl)-4-(3-methoxyphenyl)-1,2-dihydro-2-oxo-1,8-naphthyridin-3-yl]-N'-(2,6-diisopropylphenyl)urea

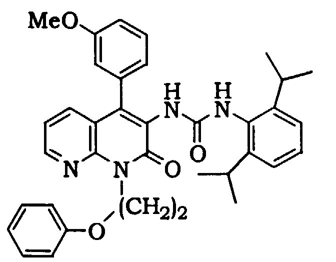

To a solution of N-[4-(3-methoxyphenyl)-1,2-dihydro-2-oxo-1,8-naphthyridin-3-yl]-N'-(2,6-diisopropylphenyl)urea (500 mg, 1.06 mmol) in DMF (10 ml) were added β-bromophenetole (256 mg, 1.28 mmol) and potassium carbonate (441 mg, 3.19 mmol), and the mixture was stirred at 40-50°C for 10 hours. The mixture was allowed to stand for cooling, and then poured into water. The mixture was extracted with ethyl acetate, and the extract was washed with water, washed with a saturated aqueous sodium chloride solution, and dried over anhydrous magnesium sulfate. The resultant was concentrated under reduced pressure, and the residue was purified by silica gel column chromatography (1-3 % methanol in chloroform), and crystallized from ether to give the title compound (446 mg, 0.77 mmol) as a colorless crystal.
m.p. 168- 169.5°C

### Example 10

### Preparation of N-[1-butyl-4-{3-(3-methylaminopropoxy)phenyl}-1,2-dihydro-2-oxo-1,8-naphthyridin-3-yl]-N'-(2,6-diisopropylphenyl)urea

### (a) Preparation of N-[1-butyl-4-[3-{3-(N-tert-butoxycarbonyl-N-methylamino)propoxy}phenyl]-1,2-dihydro-2-oxo-1,8-naphthyridin-3-yl]-N'-(2,6-diisopropylphenyl)urea

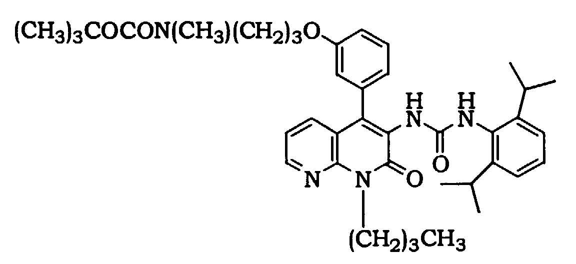

The title compound was obtained in the same manner as in Reference Example 12 from N-[4-(3-hydroxyphenyl)-1,2-dihydro-2-oxo-1,8-naphthyridin-3-yl]-N'-(2,6-diisopropylphenyl)urea and (N-tert-butoxycarbonyl-N-methylamino)propyl iodide.
¹H-NMR δ (CD₃OD) 8.64 (1H, dd, J=4.6Hz, 1.7Hz), 7.70-7.80 (1H, m), 7.48 (1H, dd, J=8.3Hz, 7.9Hz), 7.16-7.18 (2H, m), 7.04-7.16 (3H, m), 6.97-7.03 (2H, m), 4.60-4.73 (2H, m), 3.98-4.13 (2H, m), 3.40-3.55 (2H, m), 2.95-3.11 (2H, m), 2.90 (3H, m), 1.97-2.12 (2H, m), 1.73-1.90 (2H, m), 1.45-1.60 (2H, m), 1.45 (9H, s), 1.15 (12H, d, J=6.6Hz), 1.06 (3H, t, J=7.6Hz)

### (b) Preparation of N-[1-butyl-4-{3-(3-methylaminopropoxy)phenyl}-1,2-dihydro-2-oxo- 1,8-naphthyridin-3-yl]-N'-(2,6-diisopropylphenyl)urea

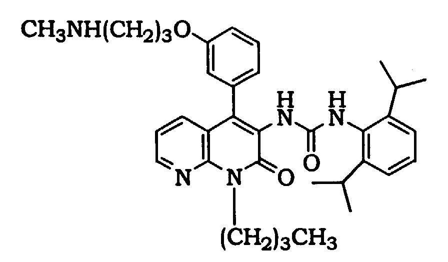

The title compound was obtained in the same manner as in Example 6-(b) from N-[1-butyl-4-[3-{3-(N-tert-butoxycarbonyl-N-methylamino)propoxy}phenyl]-1,2-dihydro-2-oxo-1,8-naphthyridin-3-yl]-N'-(2,6-diisopropylphenyl)urea.
¹H-NMR δ (DMSO-d₆) 8.61 (1H, dd, J=4.6Hz, 1.7Hz), 7.76 (2H, brs), 7.55-7.65 (1H, m), 7.39 (1H, dd, J=8.3Hz, 7.9Hz), 7.25 (1H, dd, J=7.6Hz, 4.6Hz), 7.10-7.19 (1H, m), 6.96-7.08 (3H, m), 6.85-6.94 (2H, m), 4.42-4.60 (2H, m), 3.90-4.10 (2H, m), 2.80-3.00 (2H, m), 2.68 (2H, t, J=6.9Hz), 2.32 (3H, s), 1.82-1.98 (2H, m), 1.60-1.80 (2H, m), 1.30-1.53 (2H, m), 1.02 (12H, brs), 0.96 (3H, d, J=7.3Hz)

### Example 11

### Preparation of N-[1-butyl-4-(3-hydroxyphenyl)-1,2-dihydro-2-oxo-1,8-naphthyridin-3-yl]-N'-(2,4,6-trifluorophenyl)urea

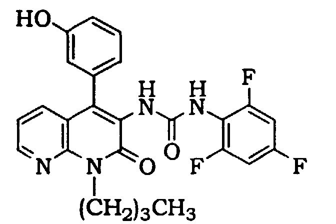

The title compound was obtained in the same manner as in Reference Example 11 from N-[1-butyl-4-(3-methoxyphenyl)-1,2-dihydro-2-oxo-1,8-naphthyridin-3-yl]-N'-(2,4,6-trifluorophenyl)urea.
¹H-NMR δ (CD₃OD) 8.62 (1H, dd, J=4.6Hz, 2.0Hz), 7.74 (1H, dd, J=7.9Hz, 2.0Hz), 7.34 (1H, dd, J=7.9Hz, 7.9Hz), 7.20-7.24 (1H, m), 6.78-6.92 (5H, m), 4.62 (2H, t, J=7.6Hz), 1.72-1.81 (2H, m), 1.42-1.52 (2H, m), 1.01 (3H, t, J=7.3Hz)

### Example 12

### Preparation of N-[1-butyl-4-{3-(2-diethylaminoethoxy)phenyl}-1,2-dihydro-2-oxo-1,8-naphthyridin-3-yl]-N'-(2,4,6-trifluorophenyl)urea

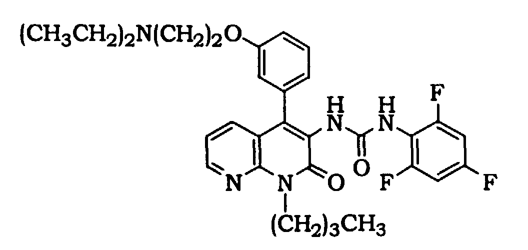

The title compound was obtained in the same manner as in Reference Example 12 from N-[1-butyl-4-(3-hydroxyphenyl)-1,2-dihydro-2-oxo-1,8-naphthyridin-3-yl]-N'-(2,4,6-trifluorophenyl)urea and 2-diethylaminoethyl chloride hydrochloride.
m.p. 132- 135°C

### Example 13

### Preparation of N-[1-butyl-4-{3-(2-diisopropylaminoethoxy)-phenyl}-1,2-dihydro-2-oxo-1,8-naphthyridin-3-yl]-N'-(2,6-diisopropylphenyl)urea

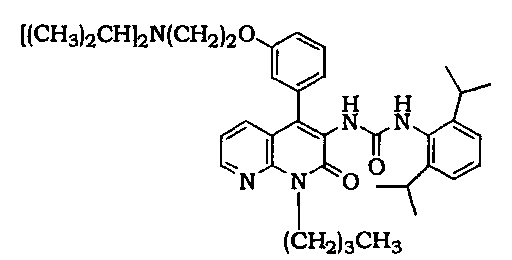

The title compound was obtained in the same manner as in Reference Example 12 from N-[1-butyl-4-(3-hydroxyphenyl)-1,2-dihydro-2-oxo-1,8-naphthyridin-3-yl]-N'-(2,6-diisopropylphenyl)urea and 2-diisopropylaminoethyl chloride hydrochloride.
¹H-NMR δ (DMSO-d₆) 8.60 (1H, dd, J=4.6Hz, 1.7Hz), 7.68-7.80 (2H, m), 7.55-7.65 (1H, m), 7.39 (1H, dd, J=7.9Hz, 7.9Hz), 7.24 (1H, dd, J=8.3Hz, 5.0Hz), 7.09-7.19 (1H, m), 6.97-7.08 (3H, m), 6.82-6.97 (2H, m), 4.44-4.58 (2H, m), 3.75-3.98 (2H, m), 2.83-3.09 (4H, m), 2.70-2.82 (2H, m), 1.60-1.80 (2H, m), 1.30-1.52 (2H, m), 0.80-1.17 (27H, m)

### Example 14

### Preparation of N-[1-(2-methyl-2-propenyl)-4-(3-methoxyphenyl)-1,2-dihydro-2-oxo- 1,8-naphthyridin-3-yl]-N'-(2,6-diisopropylphenyl)urea

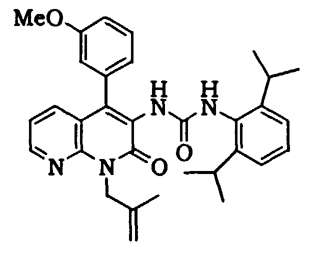

The title compound was obtained in the same manner as in Reference Example 13 from N-[4-(3-methoxyphenyl)- 1,2-dihydro-2-oxo-1,8-naphthyridin-3-yl]-N'-(2,6-diisopropylphenyl)urea and 3-bromo-2-methylpropene.
¹H-NMR δ (DMSO-d₆) 8.56 (1H, dd, J=4.6Hz, 1.9Hz), 7.76 (1H, brs), 7.73 (1H, brs), 7.64 (1H, dd, J=7.9Hz, 1.7Hz), 7.42 (1H, dd, J=8.3Hz, 8.3Hz), 7.24 (1H, dd, J=7.9Hz, 4.6Hz), 7.10-7.20 (1H, m), 6.88-7.09 (5H, m), 5.04 (2H, brs), 4.74 (1H, brs), 4.41 (1H, brs), 3.78 (3H, s), 2.84-3.02 (2H, m), 1.83 (3H, brs), 0.80-1.20 (12H, m)

### Example 15

### Preparation of N-[1-butyl-4-(3-bromophenyl)-1,2-dihydro-2-oxo-1,8-naphthyridin-3-yl]-N'-(2,6-diisopropylphenyl)urea

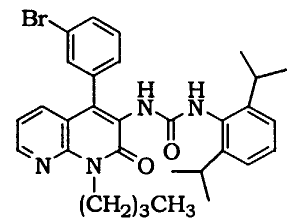

The title compound was obtained in the same manner as in Reference Example 10 from 1-butyl-4-(3-bromophenyl)-1,2-dihydro-2-oxo-1,8-naphthyridin-3-carboxylic acid and 2,6-diisopropylaniline.
m.p. 208-210°C

### Example 16

### Preparation of N-[1-cyclopropylmethyl-4-(3-methoxyphenyl)-1,2-dihydro-2-oxo-1,8-naphthyridin-3-yl]-N'-(2,6-diisopropylphenyl)urea

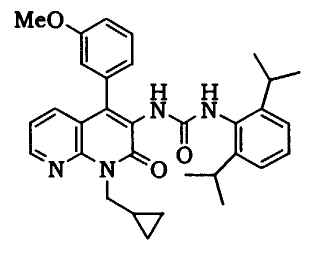

To a solution of N-[4-(3-methoxyphenyl)- 1,2-dihydro-2-oxo-1,8-naphthyridin-3-yl]-N'-(2,6-diisopropylphenyl)urea (400 mg, 0.85 mmol) in DMF (10 ml) were added potassium carbonate (141 mg, 1.02 mmol), potassium iodide (28 mg, 0.17 mmol), and (bromomethyl)cyclopropane (138 mg, 1.02 mmol), and the mixture was stirred at 40-50°C for 10 hours. After allowed to stand for cooling, the mixture was poured into water, and the mixture was extracted with ethyl acetate. The extract was washed with water, washed with a saturated aqueous sodium chloride solution, and dried over anhydrous magnesium sulfate. The resultant was concentrated under reduced pressure, and the residue was purified by silica gel column chromatography (1% methanol in chloroform), and crystallized from ether to give the title compound (354 mg, 0.67 mmol) as a colorless crystal.
m.p. 190- 190.5°C

### Example 17

### Preparation of N-[1-butyl-4-[3 {2-(N-benzyl-N-ethylamino)-ethoxy}phenyl]-1,2-dihydro-2-oxo-1,8-naphthyridin-3-yl]-N'-(2,6-diisopropylphenyl)urea

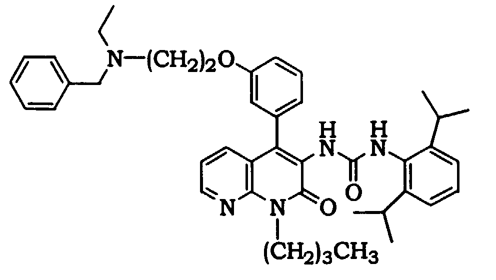

To a solution of N-[1-butyl-4-(3-hydroxyphenyl)-1,2-dihydro-2-oxo- 1,8-naphthyridin-3-yl]-N'-(2,6-diisopropylphenyl)urea (200 mg, 0.39 mmol) in DMSO (8 ml) were added 2-(N-benzyl-N-ethylamino)ethyl chloride hydrochloride (182 mg, 0.78 mmol) and potassium t-butoxide (132 mg, 1.17 mmol), and the mixture was stirred at room temperature for five hours. The mixture was poured into water, and the mixture was extracted with ethyl acetate. The extract was washed with water, washed with a saturated aqueous sodium chloride solution, and dried over anhydrous magnesium sulfate. The resultant was concentrated under reduced pressure, and the residue was purified by silica gel column chromatography (hexane:ethyl acetate = 1:1) to give the title compound (218 mg, 0.32 mmol).

### Hydrochloride:

¹H-NMR δ (DMSO-d₆) 11.51 (1H, brs), 8.62 (1H, dd, J=4.6Hz, 1.7Hz), 7.78-7.90 (2H, m), 7.55-7.70 (3H, m), 7.22-7.32 (1H, m), 6.90-7.20 (6H, m), 4.46-4.60 (2H, m), 430-4.45 (4H, m), 3.32-3.60 (2H, m), 3.05-3.25 (2H, m), 2.78-3.02 (2H, m), 1.60-1.80 (2H, m), 1.34-1.42 (2H, m), 1.27 (3H, t, J=7.3Hz), 0.80-1.15 (15H, m)

### Example 18

### Preparation of N-[1-butyl-4-{3-(2-ethylaminoethoxy)phenyl}-1,2-dihydro-2-oxo-1,8-naphthyridin-3-yl]-N'-(2,6-diisopropylphenyl)urea

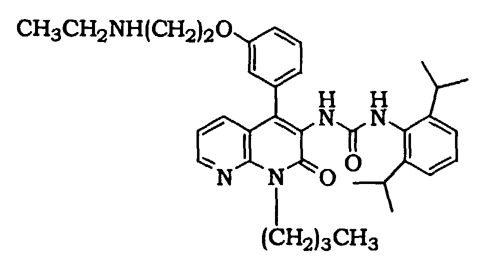

To a solution of N-[1-butyl-4-[3-{2-(N-benzyl-N-ethylamino)ethoxy}phenyl]-1,2-dihydro-2-oxo-1,8-naphthyridin-3-yl]-N'-(2,6-diisopropylphenyl)urea (129 mg, 0.191 mmol) in ethanol (10 ml) were added 12N aqueous hydrochloric acid solution (1 ml) and 10 % palladium-carbon (210 mg), and the mixture was stirred at room temperature under hydrogen atmosphere for five hours. The mixture was filtered through a cerite pad, and the filtrate was concentrated. To the concentrated resultant was added aqueous ammonia, and the mixture was extracted with ethyl acetate. The extract was washed with water, washed with a saturated aqueous sodium chloride solution, and dried over anhydrous magnesium sulfate. The reside was concentrated under reduced pressure, and the residue was purified by thin layer chromatography (10 % methanol in chloroform) to give the title compound (63 mg, 0.10 mmol).

### Hydrochloride:

¹H-NMR δ (DMSO-d₆) 8.80 (2H, brs), 8.58-8.66 (1H, m), 7.85 (1H, brs), 7.82 (1H, brs), 7.57-7.65 (1H, m), 7.44 (1H, dd, J=8.9Hz, 7.5Hz), 7.22-7.31 (1H, m), 6.90-7.20 (6H, m), 4.43-4.60 (2H, m), 4.15-4.30 (2H, m), 2.75-3.10 (4H, m), 1.60-1.80 (2H, m), 1.30-1.54 (2H, m), 1.18 (3H, t, J=7.3Hz), 0.90-1.15 (15H, m)

### Example 19

### Preparation of N-[1-butyl-4-{3-(3-hydroxy-1-propynyl)phenyl}-1,2-dihydro-2-oxo-1,8-naphthyridin-3-yl]-N'-(2,6-diisopropylphenyl)urea

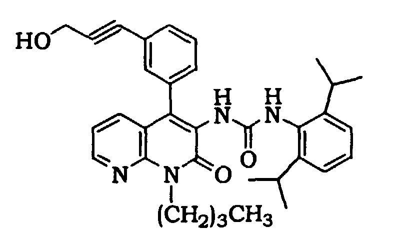

A mixture of N-[1-butyl-4-(3-bromophenyl)-1,2-dihydro-2-oxo-1,8-naphthyridin-3-yl]-N'-(2,6-diisopropylphenyl)urea (575 mg, 1 mmol), propargyl alcohol (168 mg, 3 mmol), copper (I) iodide (38 mg, 0.2 mmol), triphenylphosphine (52 mg, 0.2 mmol), bis(triphenylphosphine)-palladium (II) chloride (14 mg, 0.02 mmol), triethylamine (5 ml) and acetonitrile (5 ml) was heated under reflux for five hours. After allowed to stand for cooling, the mixture was diluted with a mixture of tetrahydrofuran and ethyl acetate, and the mixture was filtered through a cerite pad. The filtrate was concentrated under reduced pressure, and the residue was dissolved in ethyl acetate. The resultant was washed with 0.1N aqueous hydrochloric acid solution, washed with water, washed with a saturated aqueous sodium chloride solution, and dried over anhydrous magnesium sulfate. The resultant was concentrated under reduced pressure, and the residue was purified by silica gel column chromatography (hexane:ethyl acetate = 7:3 ∼ 5:5), and further purified by thin layer chromatography (hexane:ethyl acetate = 5:5) to give the title compound (16 mg, 0.03 mmol).
¹H-NMR δ (CD₃OD) 8.60 (1H, dd, J=4.6Hz, 1.7Hz), 7.66 (1H, dd, J=7.9Hz, 1.7Hz), 7.49-7.56 (3H, m), 7.37 (1H, d, J=7.3Hz), 7.15-7.23 (2H, m), 7.07 (2H, d, J=7.3Hz), 4.64 (2H, t, J=7.6Hz), 4.39 (2H, s), 2.91 (2H, br), 1.74-1.85 (2H, m), 1.46-1.54 (2H, m), 1.10 (12H, d, J=6.9Hz), 1.02 (3H, t, J=7.3Hz)

### Example 20

### Preparation of N-[1-butyl-4-{3-(3-hydroxypropyl)phenyl}-1,2-dihydro-2-oxo-1,8-naphthyridin-3-yl]-N'-(2,6-diisopropylphenyl)urea

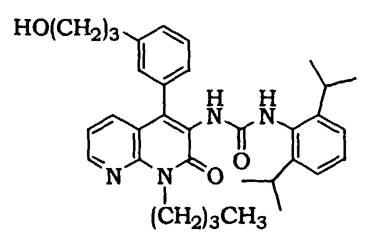

To a solution of N-[1-butyl-4-{3-(3-hydroxy-1-propynyl)phenyl}-1,2-dihydro-2-oxo-1,8-naphthyridin-3-yl]-N'-(2,6-diisopropylphenyl)urea (10 mg, 0.02 mmol) in methanol (3 ml) were added ammonium formate (5 mg, 0.08 mmol) and 10 % palladium-carbon (5 mg), and the mixture was heated under reflux for five hours. After allowed to stand for cooling, the mixture was filtered through a cerite pad, and the filtrate was concentrated. The concentrated residue was diluted with ethyl acetate, and washed with water, washed with a saturated aqueous sodium chloride solution, and dried over anhydrous magnesium sulfate. The residue was concentrated under reduced pressure, and further purified by thin layer chromatography (hexane:ethyl acetate = 5:5) to give the title compound (6 mg, 0.02 mmol).
m.p. 188-189°C

### Example 21

### Preparation of N-[1-butyl-4-{3-(3-diethylamino-1-propynyl)-phenyl}-1,2-dihydro-2-oxo-1,8-naphthyridin-3-yl]-N'-(2,6-diisopropylphenyl)urea

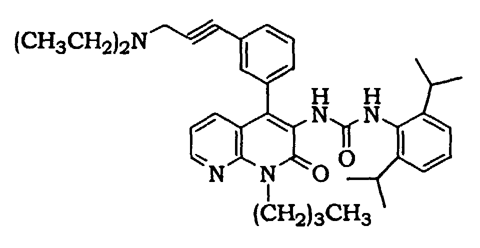

A mixture of N-[1-butyl-4-(3-bromophenyl)-1,2-dihydro-2-oxo-1,8-naphthyridin-3-yl]-N'-(2,6-diisopropylphenyl)urea (4.6 g, 8 mmol), 3-diethylamino-3-propyne (2.67 g, 24 mmol), copper (I) iodide (122 mg, 0.64 mmol), triphenylphosphine (335 mg, 1.28 mmol), 10 % palladium-carbon (336 mg), triethylamine (30 ml), acetonitrile (30 ml), and DMF (30 ml) was heated under reflux for six hours. After allowed to stand for cooling, the mixture was filtered through a cerite pad. The filtrate was concentrated under reduced pressure, and the residue was dissolved in ethyl acetate (500 ml). The mixture was washed with water, washed with a 0.5N aqueous hydrochloric acid solution, washed with water, washed with a saturated aqueous sodium chloride solution, and dried over anhydrous sodium sulfate. The resultant was concentrated under reduced pressure, and the residue was purified by silica gel column chromatography (0-4 % methanol in chloroform) to give the title compound (3.05g, 5.0 mmol).
¹H-NMR δ (CD₃OD) 8.60 (1H, d, J=3.3Hz), 7.06-7.68 (9H, m), 4.64 (2H, t, J=7.6Hz), 3.66 (2H, s), 2.85 (2H, m), 2.66 (4H, q, J=7.3Hz), 1.74-1.82 (2H, m), 1.43-1.54 (2H, m), 1.09-1.14 (18H, m), 1.02 (3H, t, J=7.3Hz)

### Example 22

### Preparation of N-[1-butyl-4-{3-(3-diethylaminopropyl)phenyl}-1,2-dihydro-2-oxo-1,8-naphthyridin-3-yl]-N'-(2,6-diisopropylphenyl)urea

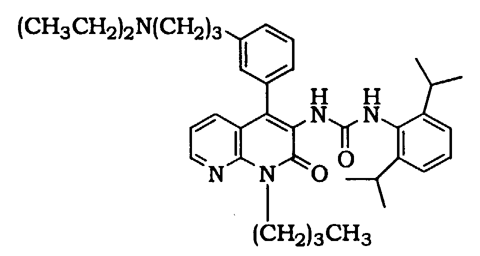

The title compound was obtained in the same manner as in Example 20 from N-[1-butyl-4-{3-(3-diethylamino-1-propynyl)phenyl}-1,2-dihydro-2-oxo-1,8-naphthyridin-3-yl]-N'-(2,6-diisopropylphenyl)-urea.
¹H-NMR δ (CD₃OD) 8.60 (1H, dd, J=4.6Hz, 1.7Hz), 7.71 (1H, dd, J=7.9Hz, 1.7Hz), 7.47 (1H, dd, J=7.9Hz, 7.6Hz), 7.35 (1H, d, J=7.9Hz), 7.07-7.26 (6H, m), 4.65 (2H, t, J=7.6Hz), 3.02 (2H, br), 2.73-2.93 (8H, m), 1.89-2.02 (2H, m), 1.74-1.86 (2H, m), 1.45-1.56 (2H, m), 0.99-1.19 (21H, m)

Simultaneously, N-[1-butyl-4-(3-propylphenyl)-1,2-dihydro-2-oxo-1,8-naphthyridin-3-yl]-N'-(2,6-diisopropylphenyl)urea was also prepared. 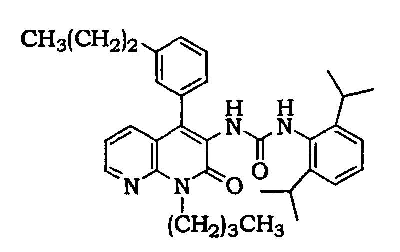
¹H-NMR δ (CD₃OD) 8.59 (1H, d, J=4.6Hz), 7.55-7.68 (2H, m), 7.44 (1H, dd, J=8.3Hz, 7.6Hz), 7.32 (1H, d, J=7.6Hz), 7.07-7.23 (5H, m), 4.63 (2H, t, J=7.6Hz), 2.98 (2H, br), 2.68 (2H, t, J=7.6Hz), 1.66-1.79 (4H, m), 1.45-1.54 (2H, m), 1.10 (12H, d, J=6.9Hz), 1.02 (3H, t, =7.3Hz), 0.98 (3H, t, J=7.3Hz)

### Example 23

### Preparation of N-[1-(N,N-diethylaminocarbonylmethyl)-4-(3-methoxyphenyl)-1,2-dihydro-2-oxo-1,8-naphthyridin-3-yl]-N'-(2,6-diisopropylphenyl)urea

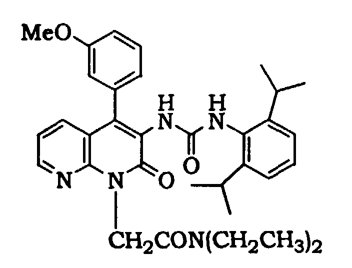

The title compound was obtained in the same manner as in Example 16 from N-[4-(3-methoxyphenyl)-1,2-dihydro-2-oxo-1,8-naphthyridin-3-yl]-N'-(2,6-diisopropylphenyl)urea and 2-chloro-N,N-diethylacetamide.
IR (KBr) 2996, 1662, 1597, 1537, 1486 cm⁻¹

### Example 24

### Preparation of N-[1-(2-methylpropyl)-4-(3-hydroxyphenyl)-1,2-dihydro-2-oxo-1,8-naphthyridin-3-yl]-N'-(2,6-diisopropylphenyl)urea

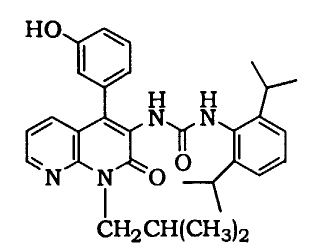

The title compound was obtained in the same manner as in Reference Example 11 from N-[1-(2-methylpropyl)-4-(3-methoxyphenyl)-1,2-dihydro-2-oxo-1,8-naphthyridin-3-yl]-N'-(2,6-diisopropylphenyl)-urea.
¹H-NMR δ (CD₃OD) 8.62 (1H, dd, J=4.6Hz, J=1.6Hz), 7.78 (1H, dd, J=7.9Hz, 1.7Hz), 7.39 (1H, dd, J=7.9Hz, 7.6Hz), 7.10-7.30 (4H, m), 6.80-7.00 (3H, m), 4.55 (2H, d, J=7.6Hz), 2.95-3.15 (2H, m), 2.30-2.55 (1H, m), 1.10-1.30 (12H, m), 1.03 (6H, d, J=6.9Hz)

### Example 25

### Preparation of N-[1-(4-methylpentyl)-4-(3-hydroxyphenyl)-1,2-dihydro-2-oxo-1,8-naphthyridin-3-yl]-N'-(2,6-diisopropylphenyl)urea

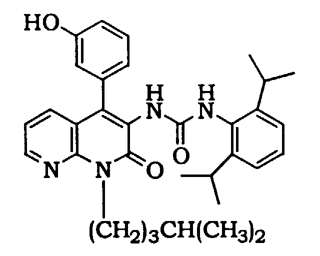

The title compound was obtained in the same manner as in Reference Example 11 from N-[1-(4-methylpentyl)-4-(3-methoxyphenyl)-1,2-dihydro-2-oxo-1,8-naphthyridin-3-yl]-N'-(2,6-diisopropylphenyl)-urea.
¹H-NMR δ (CD₃OD) 8.64 (1H, dd, J=4.6Hz, 2.0Hz), 7.78 (1H, dd, J=7.9Hz, 1.7Hz), 7.39 (1H, dd, J=8.2Hz, 7.6Hz), 7.18-7.30 (2H, m), 7.08-7.15 (2H, m), 6.80-7.00 (3H, m), 4.56-4.70 (2H, m), 3.00-3.15 (2H, m), 1.75-1.93 (2H, m), 1.60-1.74 (1H, m), 1.32-1.50 (2H, m), 1.05-1.30 (12H, m), 0.98 (6H, d, J=6.3Hz)

### Example 26

### Preparation of N-(1-(2-methylpropyl)-4-{3-(2-diethylamino-ethoxy)phenyl}-1,2-dihydro-2-oxo-1,8-naphthyridin-3-yl]-N'-(2,6-diisopropylphenyl)urea

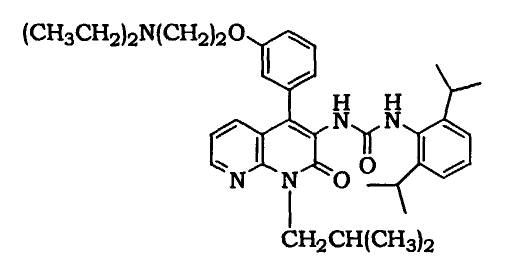

The title compound was obtained in the same manner as in Reference Example 12 from N-[1-(2-methylpropyl)-4-(3-hydroxyphenyl)-1,2-dihydro-2-oxo-1,8-naphthyridin-3-yl]-N'-(2,6-diisopropylphenyl)urea and 2-diethylaminoethyl chloride hydrochloride.

### Hydrochloride:

¹H-NMR δ (DMSO-d₆) 10.41 (1H, brs), 8.60 (1H, dd, J=4.6Hz, 1.7Hz), 7.85 (2H, brs), 7.61 (1H, dd, J=8.2Hz, 1.7Hz), 7.44 (1H, dd, J=7.9Hz, 7.9Hz), 7.24 (1H, dd, J=7.9Hz, 4.6Hz), 6.90-7.20 (6H, m), 4.26-4.50 (4H, m), 3.40-3.53 (2H, m), 3.05-3.30 (4H, m), 2.80-3.00 (2H, m), 2.20-2.40 (1H, m), 1.21 (6H, t, 3=7.3Hz), 0.91-1.10 (12H, m), 0.94 (6H, d, J=6.6Hz)

### Example 27

### Preparation of N-[1-(2-methylpropyl)-4-{3-(4-pyridylmethoxy)-phenyl}-1,2-dihydro-2-oxo-1,8-naphthyridin-3-yl]-N'-(2,6-diisopropylphenyl)urea

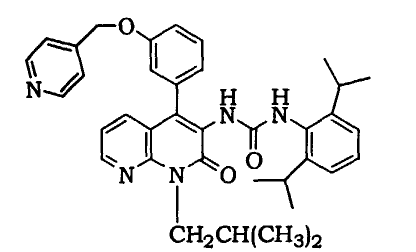

The title compound was obtained in the same manner as in Reference Example 12 from N-[1-(2-methylpropyl)-4-(3-hydroxyphenyl)-1,2-dihydro-2-oxo-1,8-naphthyridin-3-yl]-N'-(2,6-diisopropylphenyl)urea and 4-picolyl chloride hydrochloride.

### Hydrochloride:

¹H-NMR δ (DMSO-d₆) 8.80 (2H, brd, J=6.6Hz), 8.60 (1H, dd, J=4.6Hz, 1.7Hz), 7.94 (2H, brd, J=6.6Hz), 7.85 (1H, brs), 7.82 (1H, brs), 7.57 (1H, dd, J=7.9Hz, 1.3Hz), 7.46 (1H, dd, J=7.9Hz, J=7.9Hz), 7.21 (1H, dd, J=7.9Hz, 4.6Hz), 7.10-7.20 (2H, m), 6.95-7.08 (3H, m), 5.44 (2H, s), 4.41 (2H, d, J=6.9Hz), 2.90 (2H, brs), 2.20-2.40 (1H, m), 1.00 (12H, m), 0.94 (6H, d, J=6.6Hz)

### Example 28

### Preparation of N-[1-(4-methylpentyl)-4-{3-(4-pyridylmethoxy)phenyl}-1,2-dihydro-2-oxo-1,8-naphthyridin-3-yl]-N'-(2,6-diisopropylphenyl)urea

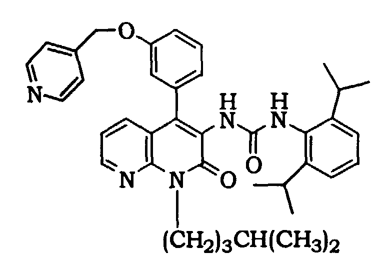

The title compound was obtained in the same manner as in Reference Example 12 from N-[1-(4-methylpentyl)-4-(3-hydroxyphenyl)-1,2-dihydro-2-oxo-1,8-naphthyridin-3-yl]-N'-(2,6-diisopropylphenyl)urea and 4-picolyl chloride hydrochloride.

### Hydrochloride:

¹H-NMR δ (DMSO-d₆) 8.79 (2H, brd, J=6.3Hz), 8.61 (1H, dd, J=4.6Hz, 1.7Hz), 7.93 (2H, brd, J=6.3Hz), 7.85 (1H, brs), 7.82 (1H, brs), 7.57 (1H, dd, J=7.9Hz, 1.9Hz), 7.46 (1H, dd, J=7.9Hz, 7.9Hz), 7.22 (1H, dd, J=7.9Hz, 4.6Hz), 7.10-7.20 (2H, m), 6.95-7.08 (4H, m), 5.43 (2H, s), 4.40-4.60 (2H, m), 2.80-3.00 (2H, m), 1.50-1.85 (3H, m), 1.20-1.40 (2H, m), 1.00 (12H, brs), 0.90 (6H, d, J=6.6Hz)

### Example 29

### Preparation of N-[1-(3-methylbutyl)-4-{3-(4-pyridylmethoxy)phenyl}-1,2-dihydro-2-oxo-1,8-naphthyridin-3-yl]-N'-(2,6-diisopropylphenyl)urea

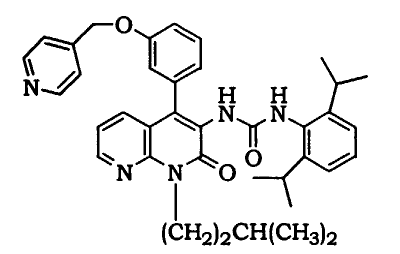

The title compound was obtained in the same manner as in Reference Example 12 from N-[1-(3-methylbutyl)-4-(3-hydroxyphenyl)-1,2-dihydro-2-oxo-1,8-naphthyridin-3-yl]-N'-(2,6-diisopropylphenyl)urea and 4-picolyl chloride hydrochloride.
¹H-NMR δ (CD₃OD) 8.59 (1H, d, J=2.6Hz), 8.50 (2H, d, J=6.3Hz), 7.60 (1H, d, J=6.6Hz), 7.51 (2H, d, J=6.3Hz), 7.46 (1H, d, J=7.9Hz), 6.99-7.17 (7H, m), 5.22 (2H, s), 4.66 (2H, t, J=7.6Hz), 2.95 (2H, br), 1.61-1.82 (3H, m), 1.04-1.12 (18H, m)

### Example 30

### Preparation of N-[1-propyl-4-(3-methoxyphenyl)-1,2-dihydro-2-oxo-1,8-naphthyridin-3-yl]-N'-(2,6-diisopropylphenyl)urea

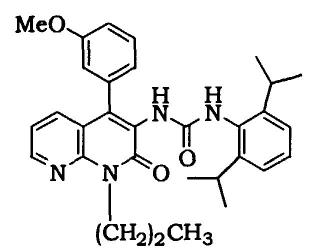

The title compound was obtained in the same manner as in Reference Example 13 from N-[4-(3-methoxyphenyl)-1,2-dihydro-2-oxo-1,8-naphthyridin-3-yl]-N'-(2,6-diisopropylphenyl)urea and 1-iodopropane.
m.p. 198.5-200°C

### Example 31

### Preparation of N-[1-hexyl-4-(3-methoxyphenyl)-1,2-dihydro-2-oxo-1,8-naphthyridin-3-yl]-N'-(2,6-diisopropylphenyl)urea

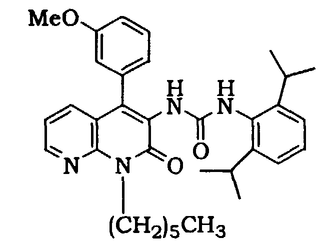

The title compound was obtained in the same manner as in Reference Example 13 from N-[4-(3-methoxyphenyl)-1,2-dihydro-2-oxo-1,8-naphthyridin-3-yl]-N'-(2,6-diisopropylphenyl)urea and 1-bromohexane.
m.p. 163.5- 165°C

### Example 32

### Preparation of N-[1-(3-phenylpropyl)-4-(3-methoxyphenyl)-1,2-dihydro-2-oxo-1,8-naphthyridin-3-yl]-N'-(2,6-diisopropylphenyl)urea

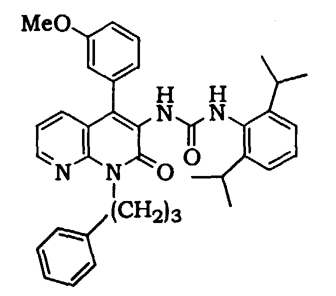

The title compound was obtained in the same manner as in Example 16 from N-[4-(3-methoxyphenyl)-1,2-dihydro-2-oxo-1,8-naphthyridin-3-yl]-N'-(2,6-diisopropylphenyl)urea and 1-bromo-3-phenylpropane.
m.p. 166-167°C

### Example 33

### Preparation of N-[1-ethyl-4-(3-methoxyphenyl)-1,2-dihydro-2-oxo-1,8-naphthyridin-3-yl]-N'-(2,6-diisopropylphenyl)urea

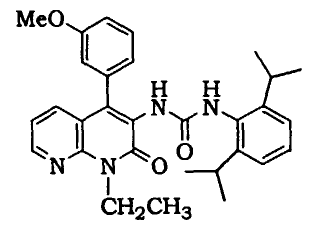

The title compound was obtained in the same manner as in Reference Example 13 from N-[4-(3-methoxyphenyl)-1,2-dihydro-2-oxo-1,8-naphthyridin-3-yl]-N'-(2,6-diisopropylphenyl)urea and 1-iodoethane.
m.p. 194-195°C

### Example 34

### Preparation of N-[1-(2-phenylethyl]-4-(3-methoxyphenyl)-1,2-dihydro-2-oxo-1,8-naphthyridin-3-yl]-N'-(2,6-diisopropylphenyl)urea

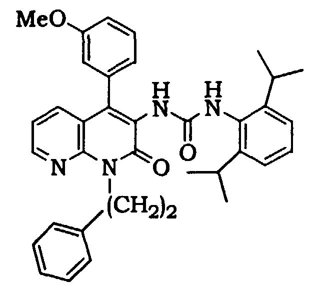

The title compound was obtained in the same manner as in Example 16 from N-[4-(3-methoxyphenyl)-1,2-dihydro-2-oxo-1,8-naphthyridin-3-yl]-N'-(2,6-diisopropylphenyl)urea and 1-bromo-3-phenylethane.
m.p. 149-150°C

### Example 35

### Preparation of N-[1-butyl-4-{3-(3-pyridylmethoxy)phenyl}-1,2-dihydro-2-oxo-1,8-naphthyridin-3-yl]-N'-(2,6-diisopropylphenyl)urea

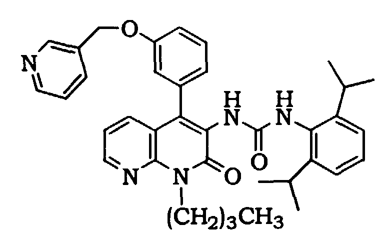

The title compound was obtained in the same manner as in Reference Example 12 from N-[1-butyl-4-(3-hydroxyphenyl)-1,2-dihydro-2-oxo-1,8-naphthyridin-3yl]-N'-(2,6-diisopropylphenyl)urea and 3-picolyl chloride hydrochloride.
¹H-NMR δ (CD₃OD) 8.63 (1H, brs), 8.59 (1H, d, J=4.6Hz), 8.50 (1H, d, J=4.6Hz), 7.42-7.50 (2H, m), 6.99-7.20 (7H, m), 5.19 (2H, s), 4.64 (2H, t, J=7.6Hz), 2.97 (2H, br), 1.70-1.83 (2H, m), 1.42-1.60 (2H, m), 1.09 (12H, d, J=6.6Hz), 1.02 (3H, t, J=7.3Hz)

### Example 36

### Preparation of N-[1-cyclohexylmethyl-4-(3-methoxyphenyl)-1,2-dihydro-2-oxo-1,8-naphthyridin-3-yl]-N'-(2,6-diisopropylphenyl)urea

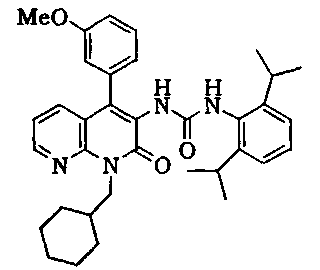

The title compound was obtained in the same manner as in Example 16 from N[4-(3-methoxyphenyl)-1,2-dihydro-2-oxo-1,8-naphthyridin-3-yl]-N'-(2,6-diisopropylphenyl)urea and bromomethylcyclohexane.
m.p. 209-210°C

### Example 37

### Preparation of N-[1-(4-methylpentyl)-4-{3-(2-diethylamino-ethoxy)phenyl}-1,2-dihydro-2-oxo-1,8-naphthyridin-3-yl]-N'-(2,6-diisopropylphenyl)urea

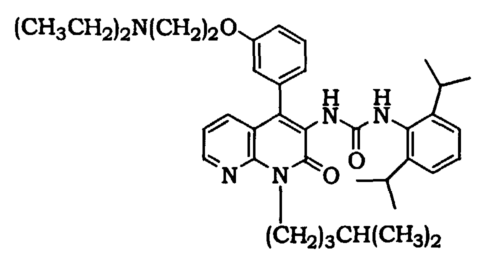

The title compound was obtained in the same manner as in Reference Example 12 from N-[1-(4-methylpentyl)-4-(3-hydroxyphenyl)-1,2-dihydro-2-oxo-1,8-naphthyridin-3-yl]-N'-(2,6-diisopropylphenyl)urea and 2-diethylaminoethyl chloride hydrochloride.

### Hydrochloride:

¹H-NMR δ (DMSO-d₆) 10.64 (1H, brs), 8.55-8.68 (1H, m), 7.79-8.02 (2H, m), 7.55-7.68 (1H, m), 7.44 (1H, dd, J=8.3Hz, 7.9Hz), 7.25 (1H, dd, J=7.9Hz, 4.6Hz), 6.86-7.20 (6H, m), 4.30-4.60 (4H, m), 3.38-3.61 (2H, m), 3.02-3.27 (6H, m), 2.80-3.00 (2H, m), 1.50-1.85 (3H, m), 1.35-1.40 (2H, m), 1.21 (6H, t, J=6.9Hz), 1.02 (12H, brs), 0.89 (6H, d, J=6.6Hz)

### Example 38

### Preparation of N-[1-(2-propenyl)-4-(3-methoxyphenyl)-1,2-dihydro-2-oxo- 1,8-naphthyridin-3-yl]-N'-(2,6-diisopropylphenyl)urea

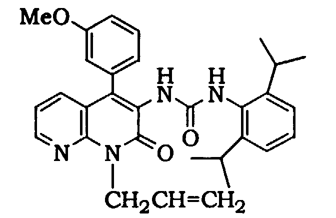

The title compound was obtained in the same manner as in Example 3 from N-[4-(3-methoxyphenyl)-1,2-dihydro-2-oxo-1,8-naphthyridin-3-yl]-N'-(2,6-diisopropylphenyl)urea and allyl bromide.
¹H-NMR δ (CD₃OD) 8.52-8.68 (1H, m), 7.70-7.82 (1H, m), 7.49 (1H, dd, J=8.3Hz, 7.9Hz), 6.95-7.30 (7H, m), 5.95-6.20 (1H, m), 5.10-5.40 (4H, m), 3.87 (3H, s), 2.90-3.15 (2H, m), 1.15 (12H, d, J=6.3Hz)

### Example 39

### Preparation of N-[1-(3-butenyl)-4-(3-methoxyphenyl)-1,2-dihydro-2-oxo-1,8-naphthyridin-3-yl]-N'-(2,6-diisopropylphenyl)urea

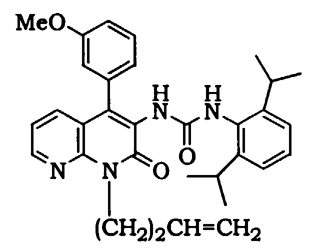

The title compound was obtained in the same manner as in Example 3 from N-[4-(3-methoxyphenyl)-1,2-dihydro-2-oxo-1,8-naphthyridin-3-yl]-N'(2,6-diisopropylphenyl)urea and 4-bromo-1-butene.
¹H-NMR δ (CD₃OD) 8.63 (1H, dd, J=4.6Hz, 1.3Hz), 7.76 (1H, dd, J=7.9Hz, 1.7Hz), 7.48 (1H, dd, J=8.2Hz, 7.9Hz), 6.94-7.30 (7H, m), 5.88-6.10 (1H, m), 5.00-5.21 (2H, m), 4.66-4.80 (2H, m), 3.87 (3H, s), 2.90-3.13 (2H, m), 2.50-2.70 (2H, m), 1.15 (12H, d, J=6.3Hz)

### Example 40

### Preparation of N-[1-butyl-4-[3-{(3,5-dimethyl-4-methoxypyridin-2-yl)methoxy}phenyl]-1,2-dihydro-2-oxo-1,8-naphthyridin-3-yl]-N'-(2,6-diisopropylphenyl)urea

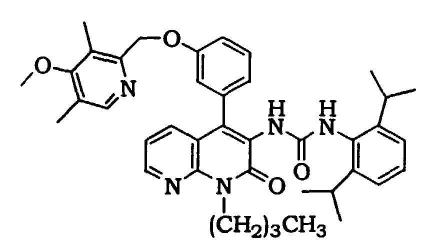

The title compound was obtained in the same manner as in Reference Example 12 from N-[1-butyl-4-(3-hydroxyphenyl)-1,2-dihydro-2-oxo-1,8-naphthyridin-3-yl]-N'-(2,6-diisopropylphenyl)urea and 2-chloromethyl-3,5-dimethyl-4-methoxypyridine.

### Hydrochloride:

¹H-NMR δ (DMSO-d₆) 8.60 (1H, d, J=4.6Hz), 8.53 (1H, s), 7.87 (1H, s), 7.81 (1H, s), 7.60 (1H, d, J=6.6Hz), 7.45 (1H, dd, J=7.9Hz, 7.9Hz), 7.00-7.25 (7H, m), 5.38 (2H, s), 4.54 (2H, brs), 3.97 (3H, s), 2.90 (2H, br), 2.37 (3H, s), 2.30 (3H, s), 1.72 (2H, m), 1.43 (2H, m), 0.95-1.01 (15H, m)

### Example 41

### Preparation of N-[1-decyl-4-(3-methoxyphenyl)-1,2-dihydro-2-oxo-1,8-naphthyridin-3-yl]-N'-(2,6-diisopropylphenyl)urea

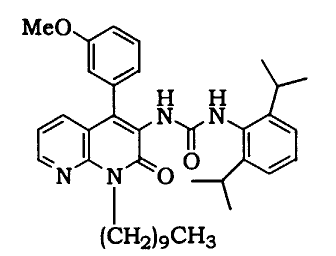

The title compound was obtained in the same manner as in Example 16 from N-[4-(3-methoxyphenyl)-1,2-dihydro-2-oxo-1,8-naphthyridin-3-yl]-N'-(2,6-diisopropylphenyl)urea and 1-bromodecane.
m.p. 119-122°C

### Example 42

### Preparation of N-[1-butyl-4-[3-{3-(3-pyridyl)propoxy}phenyl]-1,2-dihydro-2-oxo-1,8-naphthyridin-3-yl]-N'-(2,6-diisopropylphenyl)urea

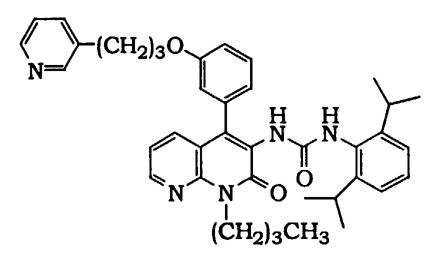

The title compound was obtained in the same manner as in Reference Example 12 from N-[1-butyl-4-(3-hydroxyphenyl)-1,2-dihydro-2-oxo-1,8-naphthyridin-3-yl]-N'-(2,6-diisopropylphenyl)urea and 1-iodo-3-(3-pyridyl)propane.
¹H-NMR δ (CD₃OD) 8.60 (1H, d, J=4.3Hz), 8.41 (1H, s), 8.34 (1H, d, J=2.3Hz), 7.70-7.73 (2H, m), 7.43 (1H, dd, J=8.3Hz, 7.6Hz), 7.32 (1H, dd, J=8.3Hz, 5.0Hz), 6.94-7.23 (7H, m), 4.64 (2H, t, J=7.6Hz), 4.08 (2H, m), 2.83-2.99 (4H, m), 2.14 (2H, m), 1.79 (2H, m), 1.54 (2H, m), 1.10 (12H, d, J=6.9Hz), 1.02 (3H, t, J=7.3Hz)

### Example 43

### Preparation of N-[1-butyl-4-(3-propoxyphenyl)-1,2-dihydro-2-oxo-1,8-naphthyridin-3-yl]-N'-(2,6-diisopropylphenyl)urea

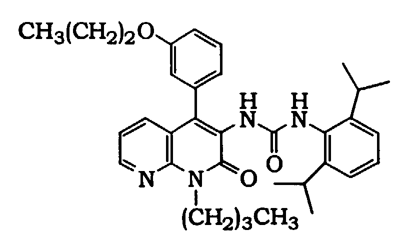

To a suspension of N-{1-butyl-4-(3-hydroxyphenyl)-1,2-dihydro-2-oxo-1,8-naphthyridin-3-yl}-N'-(2,6-diisopropylphenyl)urea (300 mg, 0.58 mmol), potassium carbonate (96 mg, 0.7 mmol) and potassium iodide (19 mg) in DMF (5 ml) was added 1-iodopropane (99 mg, 0.58 mmol) at room temperature, and the mixture was stirred at 40-50°C for five hours. Alter allowed to stand for cooling, the mixture was poured into water, and the mixture was extracted with ethyl acetate. The extracted was washed with water, washed with a saturated aqueous sodium chloride solution, and dried over anhydrous magnesium sulfate. The resultant was concentrated under reduced pressure, and the residue was purified by silica gel column chromatography (20 % ethyl acetate in hexane), and crystallised from ether/hexane to give the title compound (253 mg, 0.48 mmol).
m.p. 154-155.5°C

### Example 44

### Preparation of N-[1-butyl-4-(3-hexyloxyphenyl)-1,2-dihydro-2-oxo-1,8-naphthyridin-3-yl]-N'-(2,6-diisopropylphenyl)urea

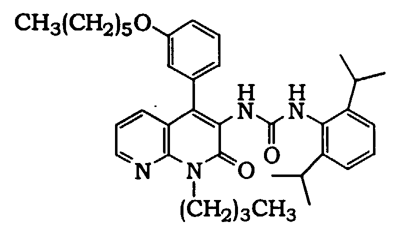

The title compound was obtained in the same manner as in Example 43 from N-[1-butyl-4-(3-hydroxyphenyl)-1,2-dihydro-2-oxo-1,8-naphthyridin-3-yl]-N'-(2,6-diisopropylphenyl)urea and 1-iodohexane.
m.p. 115-117°C

### Example 45

### Preparation of N-[1-butyl-4-(3-benzyloxyphenyl)-1,2-dihydro-2-oxo-1,8-naphthyridin-3-yl]-N'-(2,6-diisopropylphenyl)urea

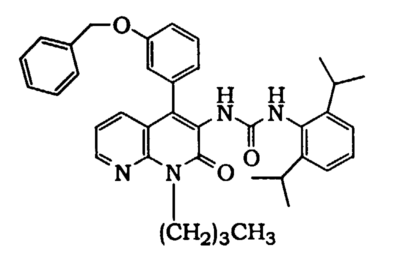

The title compound was obtained in the same manner as in Reference Example 12 from N-[1-butyl-4-(3-hydroxyphenyl)-1,2-dihydro-2-oxo-1,8-naphthyridin-3-yl]-N'-(2,6-diisopropylphenyl)urea and benzyl bromide.
m.p. 178- 179°C

### Example 46

### Preparation of N-[1-butyl-4-(3-cyclohexylmethoxyphenyl)-1,2-dihydro-2-oxo-1,8-naphthyridin-3-yl]-N'-(2,6-diisopropylphenyl)urea

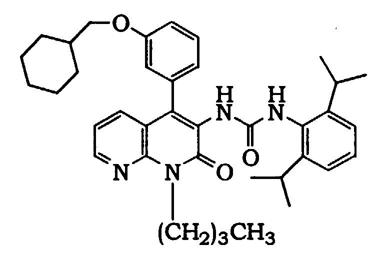

The title compound was obtained in the same manner as in Reference Example 12 from N-[1-butyl-4-(3-hydroxyphenyl)-1,2-dihydro-2-oxo-1,8-naphthyridin-3-yl]-N'-(2,6-diisopropylphenyl)urea and bromomethylcyclohexane.
m.p. 102-106°C

### Example 47

### Preparation of N-[1-(4-pentenyl)-4-(3-hydroxyphenyl)-1,2-dihydro-2-oxo-1,8-naphthyridin-3-yl]-N'-(2,6-diisopropylphenyl)urea

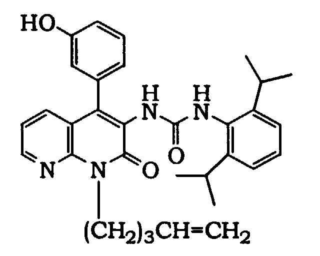

The title compound was obtained in the same manner as in Reference Example 11 from N-[1-(4-pentenyl)-4-(3-methoxyphenyl)-1,2-dihydro-2-oxo-1,8-naphthyridin-3-yl]-N'-(2,6-diisopropylphenyl)urea.
¹H-NMR δ (DMSO-d₆) 9.52 (1H, s), 8.61 (1H, dd, J=4.3Hz, 1.3Hz), 7.71 (1H, s), 7.70 (1H, s), 7.63 (1H, dd, J=7.9Hz, 1.3Hz), 7.20-7.35 (3H, m), 7.10-7.20 (1H, m), 7.00-7.08 (2H, m), 6.80-6.90 (1H, m), 6.67-6.79 (2H, m), 5.80-6.00 (1H, m), 4.90-5.20 (2H, m), 4.39-4.61 (2H, m), 2.85-3.06 (2H, m), 2.05-2.28 (2H, m), 1.72-1.93 (2H, m), 1.3 (12H, d, J=6.3Hz)

### Example 48

### Preparation of N-[1-(4-pentenyl)-4-{3-(4-pyridylmethoxy)phenyl}-1,2-dihydro-2-oxo-1,8-naphthyridin-3-yl]-N'-(2,6-diisopropylphenyl)urea

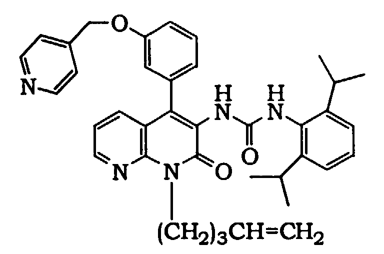

The title compound was obtained in the same manner as in Reference Example 12 from N-[1-(4-pentenyl)-4-(3-hydroxyphenyl)-1,2-dihydro-2-oxo-1,8-naphthyridin-3-yl]-N'-(2,6-diisopropylphenyl)urea and 4-picolyl chloride hydrochloride.

### Hydrochloride:

¹H-NMR δ (DMSO-d₆) 8.07-8.80 (2H, m), 8.62 (1H, dd, J=4.3Hz, 1.7Hz), 7.73-7.89 (4H, m), 7.52-7.62 (1H, m), 7.46 (1H, dd, J=8.6Hz, 7.9Hz), 7.22 (1H, dd, J=7.9Hz, 4.6Hz), 7.11-7.20 (2H, m), 6.95-7.09 (4H, m), 5.78-6.00 (1H, m), 5.38 (2H, s), 4.95-5.19 (2H, m), 4.45-4.61 (2H, m), 2.08-3.00 (2H, m), 2.10-2.28 (2H, m), 1.73-1.92 (2H, m), 0.85-1.17 (12H,m)

### Example 49

### Preparation of N-[1-(4-methylpentyl)-4-[3-{2-(N-benzyl-N-ethylamino)ethoxy}phenyl]-1,2-dihydro-2-oxo-1,8-naphthyridin-3-yl]-N'-(2,6-diisopropylphenyl)urea

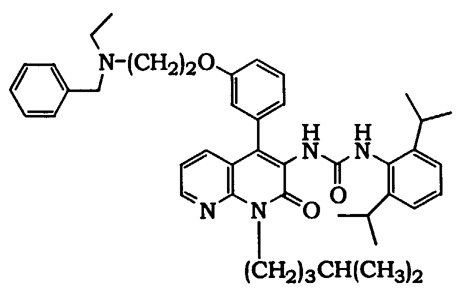

The title compound was obtained in the same manner as in Reference Example 12 from N-[1-(4-methylpentyl)-4-(3-hydroxyphenyl)-1,2-dihydro-2-oxo-1,8-naphthyridin-3-yl]-N'-(2,6-diisopropylphenyl)urea and 2-(N-benzyl-N-ethyl)ethyl chloride hydrochloride.

### Hydrochloride:

¹H-NMR δ (DMSO-d₆) 10.81 (1H, brs), 8.62 (1H, dd, J=4.6Hz, 1.3Hz), 7.79-7.91 (2H, m), 7.55-7.74 (3H, m), 7.35-7.51 (5H, m), 7.25 (1H, dd, J=7.6Hz, 4.6Hz), 6.90-7.20 (5H, m), 4.29-4.60 (6H, m), 3.33-3.55 (2H, m), 3.02-3.27 (2H, m), 2.80-3.00 (2H, m), 1.53-1.83 (3H, m), 1.20-1.40 (2H, m), 1.28 (3H, t, J=7.3Hz), 0.94-1.15 (12H, m), 0.90 (6H, d, J=6.3Hz)

### Example 50

### Preparation of N-[1-(4-pentenyl)-4-{3-(2-diethylaininoethoxy)-phenyl}-1,2-dihydro-2-oxo-1,8-naphthyridin-3-yl]-N'-(2,6-diisopropylphenyl)urea

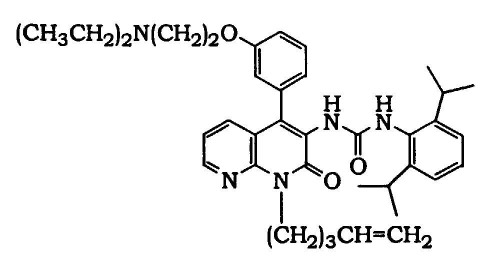

The title compound was obtained in the same manner as in Reference Example 12 from N-[1-(4-pentenyl)-4-(3-hydroxyphenyl)-1,2-dihydro-2-oxo-1,8-naphthyridin-3-yl]-N'-(2,6-diisopropylphenyl)urea and 2-diethylaminoethyl chloride hydrochloride.

### Hydrochloride:

¹H-NMR δ (DMSO-d₆) 9.99 (1H, brs), 8.62 (1H, dd, J=4.3Hz, 1.7Hz), 7.85 (1H, brs), 7.83 (1H, brs), 7.62 (1H, dd, J=8.3Hz, 1.7Hz), 7.45 (1H, dd, J=8.3Hz, 8.3Hz), 7.27 (1H, dd, J=7.9Hz, 4.6Hz), 6.90-7.11 (6H, m), 5.80-6.00 (1H, m), 4.95-5.19 (2H, m), 4.48-4.64 (2H, m), 4.28-4.44 (2H, m), 3.40-3.57 (2H, m), 3.06-3.27 (4H, m), 2.79-3.02 (2H, m), 2.09-2.29 (2H, m), 1.72-1.94 (2H, m), 1.20 (6H, t, J=7.3Hz), 1.85-1.14 (12H,m)

### Example 51

### Preparation of N-[1-butyl-4-(3-methylphenyl)-1,2-dihydro-2-oxo-1,8-naphthyridin-3-yl]-N'-(2,6-diisopropylphenyl)urea

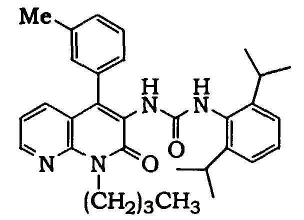

The title compound was obtained in the same manner as in Reference Example 10 from 1-butyl-4-(3-methylphenyl)-1,2-dihydro-2-oxo-1,8-naphthyridine-3-carboxylic acid and 2,6-diisopropylaniline.
m.p. 214-215°C

### Example 52

### Preparation of N-[1-octyl-4-(3-methoxyphenyl)-1,2-dihydro-2-oxo-1,8-naphthyridin-3-yl]-N'-(2,6-diisopropylphenyl)urea

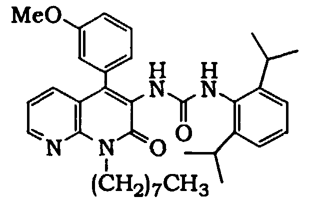

The title compound was obtained in the same manner as in Example 3 from N-[4-(3-methoxyphenyl)-1,2-dihydro-2-oxo-1,8-naphthyridin-3-yl]-N'-(2,6-diisopropylphenyl)urea and bromooctane.
¹H-NMR δ (DMSO-d₆) 8.59 (1H, dd, J=4.6Hz, 2.0Hz), 7.74 (2H, s), 7.63 (1H, d, J=7.9Hz), 7.41 (1H, dd, J=8.3Hz, 7.9Hz), 7.24 (1H, dd, J=8.3Hz, 4.6Hz), 7.15 (1H, t, J=7.3Hz), 7.04 (3H, m), 6.92 (2H, brs), 4.51 (2H, t, J=7.3Hz), 3.77 (3H, s), 2.92 (2H, sep, J=6.9Hz), 1.73 (2H, br), 1.27-1.37 (10H, br), 1.02 (12H, br), 0.85 (3H, brt, J=6.9Hz)

### Example 53

### Preparation of N-[1-butyl-4-{3-(quinolin-2-ylmethoxy)phenyl}-1,2-dihydro-2-oxo-1,8-naphthyridin-3-yl]-N'-(2,6-diisopropylphenyl)urea

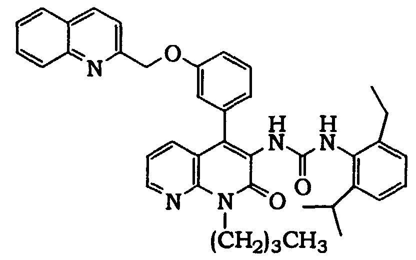

The title compound was obtained in the same manner as in Reference Example 12 from N-[1-butyl-4-(3-hydroxyphenyl)-1,2-dihydro-2-oxo-1,8-naphthyridin-3-yl]-N'-(2,6-diisopropylphenyl)urea and 2-(chloromethyl)quinoline hydrochloride.
¹H-NMR δ (DMSO-d₆) 8.48-8.60 (2H, m), 8.05 (1H, dd, J=8.3Hz, 3.3Hz), 7.62-7.86 (5H, m), 7.41-7.48 (2H, m), 6.95-7.26 (8H, m), 5.44 (2H, s), 4.51 (2H, t, J=7.3Hz), 2.94 (2H, br), 1.71 (2H, br), 1.44 (2H, m), 0.95-1.03 (15H, m)

### Example 54

### Preparation of N-[1-(4-pentenyl)-4-{3-(2-pyridylmethoxy)phenyl}-1,2-dihydro-2-oxo-1,8-naphthyridin-3-yl]-N'-(2,6-diisopropylphenyl)urea

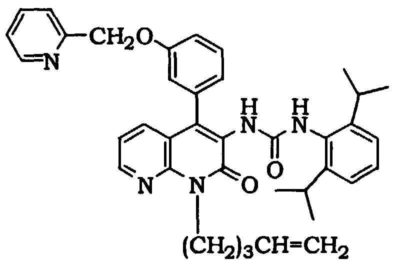

The title compound was obtained in the same manner as in Reference Example 12 from N-[1-(4-pentenyl)-4-(3-hydroxyphenyl)-1,2-dihydro-2-oxo-1,8-naphthyridin-3-yl]-N'-(2,6-diisopropylphenyl)urea and 2-picolyl chloride hydrochloride.

### Hydrochloride:

¹H-NMR δ (DMSO-d₆) 8.65-8.74 (1H, m), 8.61 (1H, dd, J=4.6Hz, 1.7Hz), 8.02-8.15 (1H, m), 7.82 (1H, brs), 7.81 (1H, brs), 7.72-7.80 (1H, m), 7.51-7.65 (2H, m), 7.45 (1H, dd, J=7.9Hz, 7.9Hz), 7.22 (1H, dd, J=7.9Hz, 4.6Hz), 7.10-7.20 (2H, m), 6.94-7.09 (1H, m), 5.76-6.00 (1H, m), 5.32 (2H, s), 4.92-5.20 (2H, m), 4.35-4.65 (2H, m), 2.79-3.01 (2H, m), 2.05-2.28 (2H, m), 1.70-1.91 (2H, m), 0.78-1.15 (12H, brs)

### Example 55

### Preparation of N-[1-(4-pentenyl)-4-{3-(3-pyridylmethoxy)phenyl}-1,2-dihydro-2-oxo-1,8-naphthyridin-3-yl]-N'-(2,6-diisopropylphenyl)urea

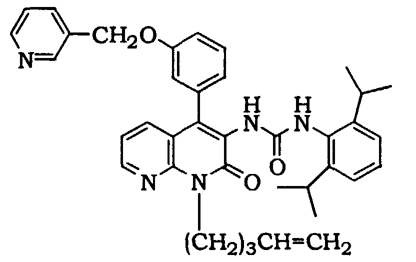

The title compound was obtained in the same manner as in Reference Example 12 from N-[1-(4-pentenyl)-4-(3-hydroxyphenyl)-1,2-dihydro-2-oxo-1,8-naphthyridin-3-yl]-N'-(2,6-diisopropylphenyl)urea and 3-picolyl chloride hydrochloride.

### Hydrochloride:

¹H-NMR δ (DMSO-d₆) 8.90-8.96 (1H, m), 8.75-8.84 (1H, m), 8.62 (1H, dd, J=4.6Hz, 2.0Hz), 8.38-8.48 (1H, m), 7.86 (1H, dd, J=7.9Hz, 5.6Hz), 7.84 (2H, brs), 7.57 (1H, dd, J=7.9Hz, 1.7Hz), 7.45 (1H, dd, J=7.9Hz, 7.9Hz), 7.24 (1H, dd, J=8.3Hz, 4.6Hz), 7.11-7.12 (2H, m), 6.90-7.10 (5H, m), 5.78-6.00 (1H, m), 5.32 (2H, s), 4.93-5.18 (2H, m), 4.41-4.61 (2H, m), 2.80-3.02 (2H, m), 2.08-2.28 (2H, m), 1.72-1.92 (2H, m), 0.70-1.15 (12H, m)

### Example 56

### Preparation of N-[1-(4-pentenyl)-4-{3-(3-piperidinopropoxy)-phenyl}-1,2-dihydro-2-oxo-1,8-naphthyridin-3-yl]-N'-(2,6-diisopropylphenyl)urea

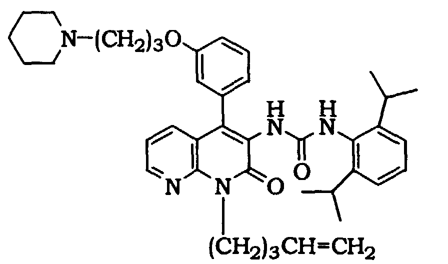

The title compound was obtained in the same manner as in Reference Example 12 from N-[1-(4-pentenyl)-4-(3-hydroxyphenyl)-1,2-dihydro-2-oxo-1,8-naphthyridin-3-yl]-N'-(2,6-diisopropylphenyl)urea and N-(3-chloropropyl)piperidine hydrochloride.

### Hydrochloride:

¹H-NMR δ (DMSO-d₆) 10.83 (1H, brs), 8.62 (1H, dd, J=4.6Hz, 1.3Hz), 7.83 (1H, brs), 7.82 (1H, brs), 7.58-7.68 (1H, m), 7.42 (1H, dd, J=7.9Hz, 7.6Hz), 7.26 (1H, dd, J=7.9Hz, 4.6Hz), 7.11-7.20 (1H, m), 6.99-7.09 (3H, m), 6.87-6.98 (2H, m), 5.80-6.00 (1H, m), 4.93-5.19 (2H, m), 4.42-4.62 (2H, m), 3.96-4.15 (2H, m), 3.35-3.52 (2H, m), 3.02-3.22 (2H, m), 2.70-3.00 (3H, m), 2.05-2.28 (4H, m), 1.55-1.94 (8H, m), 1.25-1.47 (1H, m), 1.02 (12H, brs)

### Example 57

### Preparation of N-[1-(4-methylpentyl)-4-{3-(2-ethylaminoethoxy)-phenyl}-1,2-dihydro-2-oxo-1,8-naphthyridin-3-yl]-N'-(2,6-diisopropylphenyl)urea

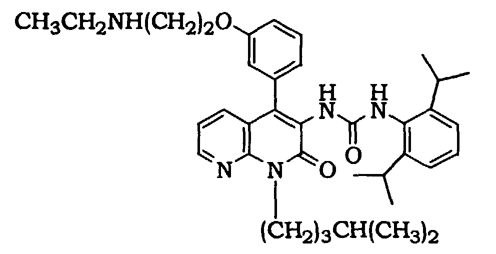

The title compound was obtained in the same manner as in Reference Example 14 from N-(1-(4-methylpentyl)-4-[3-{2-(N-benzyl-N-ethylamino)ethoxy}phenyl]-1,2-dihydro-2-oxo-1,8-naphthyridin-3-yl]-N'-(2,6-diisopropylphenyl)urea.

### Hydrochloride:

¹H-NMR δ (DMSO-d₆) 8.92 (2H, brs), 8.62 (1H, dd, J=4.6Hz, 1.7Hz), 7.72 (1H, brs), 7.71 (1H, brs), 7.61 (1H, dd, J=7.9Hz, 1.7Hz), 7.44 (1H, dd, J=8.3Hz, 7.9Hz), 7.25 (1H, dd, J=7.9Hz, 4.6Hz), 6.85-7.20 (6H, m), 4.38-4.58 (2H, m), 4.16-4.33 (2H, m), 3.20-3.40 (2H, m), 2.78-3.10 (4H, m), 1.50-1.83 (3H, m), 1.25-1.40 (2H, m), 1.19 (3H, t, J=7.3Hz), 0.93-1.13 12H, m), 0.90 (6H, d, J=6.6Hz)

### Example 58

### Preparation of N-[1-butyl-4-{3-(2-methyl-3-pyridylmethoxy)phenyl}-1,2-dihydro-2-oxo-1,8-naphthyridin-3-yl]-N'-(2,6-diisopropylphenyl)urea

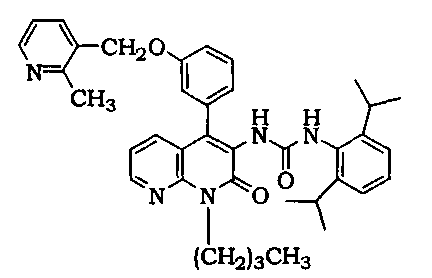

The title compound was obtained in the same manner as in Reference Example 12 from N-[1-butyl-4-(3-hydroxyphenyl)-1,2-dihydro-2-oxo-1,8-naphthyridln-3-yl]-N'-(2,6-diisopropylphenyl)urea and 2-methyl-3-picolyl chloride hydrochloride.
¹H-NMR δ (DMSO-d₆) 8.67 (1H, d, J=4.3Hz), 8.61 (1H, dd, J=4.6Hz, 1.7Hz), 8.44 (1H, d, J=7.6Hz), 7.86 (1H, s), 7.82 (1H, s), 7.74 (1H, dd, J=6.9Hz, 6.6Hz), 7.59 (1H, d, J=6.3Hz), 7.45 (1H, d, J=7.9Hz, 7.9Hz), 6.98-7.26 (7H, m), 5.30 (2H, s), 4.53 (2H, t, J=7.6Hz), 2.90 (2H, m), 2.74 (3H, s), 1.72 (2H, m), 1.45 (2H, m), 0.95-1.12 (15H, m)

### Example 59

### Preparation of N-[1-butyl-4-[3-{3-(4-pyridyl)propoxy}phenyl]-1,2-dihydro-2-oxo-1,8-naphthyridin-3-yl]-N'-(2,6-diisopropylphenyl)urea

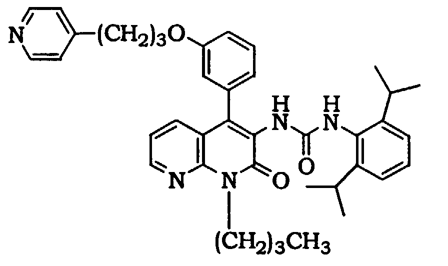

The title compound was obtained in the same manner as in Reference Example 12 from N-[1-butyl-4-(3-hydroxyphenyl)-1,2-dihydro-2-oxo-1,8-naphthyridin-3-yl]-N'-(2,6-diisopropylphenyl)urea and 3-(4-pyridyl)propyl bromide hydrochloride.
m.p. 138- 140°C

### Example 60

### Preparation of N-[1-butyl-4-[3-{2-(2-pyridyl)ethoxy}phenyl]-1,2-dihydro-2-oxo-1,8-naphthyridin-3-yl]-N'-(2,6-diisopropylphenyl)urea

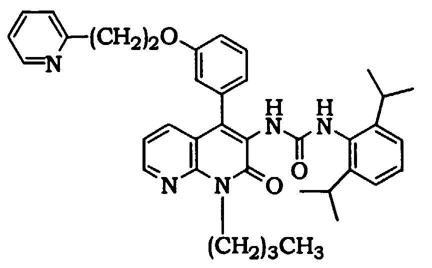

To a solution of N-[1-butyl-4-(3-hydroxyphenyl)-1,2-dihydro-2-oxo-1,8-naphthyridin-3-yl]-N'-(2,6-diisopropylphenyl)urea (300 mg, 0.58 mmol), 2-pyridinethanol (71 mg, 0.58 mmol) in THF (5 ml) were added triphenylphosphine (152 mg, 0.58 mmol), and diethyl azodicarboxylate (101 mg, 0.58 mmol), and the mixture was stirred at room temperature for 40 hours. The mixture was poured into water, and extracted with ethyl acetate. The extract was washed with water, washed with a saturated aqueous sodium chloride solution, and dried over anhydrous magnesium sulfate. The resultant was concentrated under reduced pressure, and the residue was purified by silica gel column chromatography (ethyl acetate:hexane = 5:5) and thin layer chromatography to give the title compound (43 mg, 0.07 mmol).
¹H-NMR δ (CD₃OD) 8.58 (1H, d, J=4.6Hz), 8.44 (1H, d, J=4.3Hz), 7.68-7.76 (2H, m), 7.41 (2H, m), 6.93-7.28 (8H, m), 4.62 (2H, t, J=7.3Hz), 4.37 (2H, t, J=6.3Hz), 3.24 (2H, t, J=6.3Hz), 2.93 (2H, br), 1.75 (2H, m), 1.47 (2H, m), 1.26 (12H, br), 1.00 (3H, t, J=7.3Hz)

### Example 61

### Preparation of N-[1-butyl-4-[3-{(2,4-dimethylpyridin-3-yl)-methoxy)phenyl]-1,2-dihydro-2-oxo-1,8-naphthyridin-3-yl]-N'-(2,6-diisopropylphenyl)urea

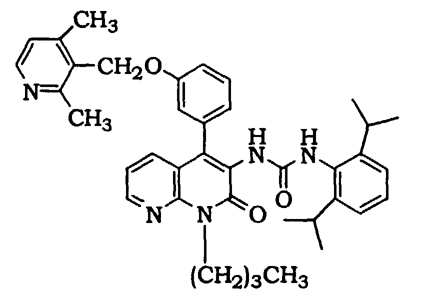

The title compound was obtained in the same manner as in Reference Example 12 from N-[1-butyl-4-(3-hydroxyphenyl)-1,2-dihydro-2-oxo-1,8-naphthyridin-3-yl]-N'-(2,6-diisopropylphenyl)urea and 3-chloromethyl-2,4-dimethylpyridine hydrochloride.

### Hydrochloride:

¹H-NMR δ (DMSO-d₆) 8.65 (1H, d, J=5.9Hz), 8.62 (1H, dd, J=4.6Hz, 1.7Hz), 7.78-7.86 (3H, m), 7.64 (1H, dd, J=7.9Hz, 1.7Hz), 7.47 (1H, dd, J=7.9Hz, 7.9Hz), 7.02-7.28 (7H, m), 5.26 (2H, brs), 4.53 (2H, t, J=7.3Hz), 2.92 (2H, m), 2.76 (3H, s), 2.60 (3H, s), 1.69 (2H, m), 1.44 (2H, m), 1.00 (12H, brs), 0.97 (3H, t, J=7.3Hz)

### Example 62

### Preparation of N-(1-butyl-4-[3-{2-(3-pyridyl)ethoxy}phenyl]-1,2-dihydro-2-oxo-1,8-naphthyridin-3-yl]-N'-(2,6-diisopropylphenyl)urea

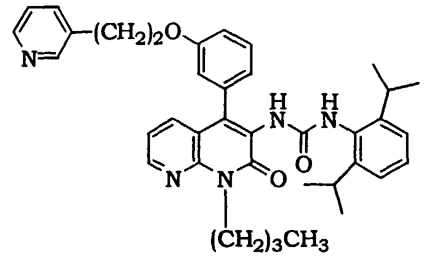

The title compound was obtained in the same manner as in Example 60 from N-[1-butyl-4-(3-hydroxyphenyl)-1,2-dihydro-2-oxo-1,8-naphthyridin-3-yl]-N'-(2,6-diisopropylphenyl)urea and 3-pyridinethanol.
¹H-NMR δ (CD₃OD) 8.58 (1H, dd, J=4.6Hz, J=1.7Hz), 8.49 (1H, s), 8.37 (1H, d, J=4.0Hz), 7.80 (1H, d, J=7.9Hz), 7.52-7.60 (3H, m), 7.37 (1H, m), 6.93-7.21 (6H, m), 4.62 (2H, t, J=6.3Hz), 2.91 (2H, m), 1.78 (2H, m), 1.47 (2H, m), 1.04 (15H, br)

### Example 63

### Preparation of N-[1-(4-pentenyl)-4-{3-(3-dimethylamino-propoxy)phenyl}-1,2-dihydro-2-oxo-1,8-naphthyridin-3-yl]-N'-(2,6-diisopropylphenyl)urea

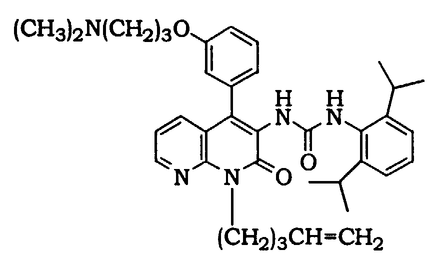

The title compound was obtained in the same manner as in Reference Example 12 from N-[1-(4-pentenyl)-4-(3-hydroxyphenyl)-1,2-dihydro-2-oxo-1,8-naphthyridin-3-yl]-N'-(2,6-diisopropylphenyl)urea and 3-dimethylaminopropyl chloride hydrochloride.

### Hydrochloride:

¹H-NMR δ (DMSO-d₆) 10.35 (1H, brs), 8.62 (1H, dd, J=4.6Hz, 1.7Hz), 7.83 (1H, brs), 7.82 (1H, brs), 7.62 (1H, dd, J=7.9Hz, 1.7Hz), 7.41 (1H, dd, J=8.3Hz, 7.6Hz), 7.26 (1H, dd, J=7.9Hz, 4.6Hz), 7.11-7.20 (1H, m), 7.00-7.09 (3H, m), 6.85-6.99 (2H, m), 5.78-6.00 (1H, m), 4.93-5.20 (2H, m), 4.40-4.66 (2H, m), 3.09-3.28 (2H, m), 2.81-3.02 (2H, m), 2.73 (6H, d, J=5.0Hz), 2.03-2.28 (4H, m), 1.71-1.94 (2H, m), 0.75-1.20 (12H, m)

### Example 64

### Preparation of N-[1-(4-pentenyl)-4-[3-{2-(N-benzyl-N-ethylamino)ethoxy}phenyl]-1,2-dihydro-2-oxo-1,8-naphthyridin-3-yl]-N'-(2,6-diisopropylphenyl)urea

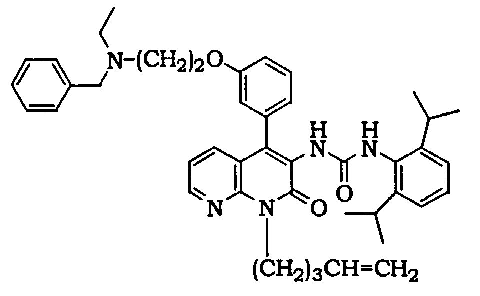

The title compound was obtained in the same manner as in Reference Example 12 from N-[1-(4-pentenyl)-4-(3-hydroxyphenyl)-1,2-dihydro-2-oxo-1,8-naphthyridin-3-yl]-N'-(2,6-diisopropylphenyl)urea and 2-(N-benzyl-N-ethylamino)ethyl chloride hydrochloride.

### Hydrochloride:

¹H-NMR δ (DMSO-d₆) 10.75 (1H, brs), 8.62 (1H, dd, J=4.6Hz, 1.7Hz), 7.85 (1H, brs), 7.55-7.72 (3H, m), 7.37-7.52 (4H, m), 7.26 (1H, dd, J=7.9Hz, J=4.6Hz), 6.89-7.20 (7H, m), 5.80-6.00 (1H, m), 4.93-5.19 (2H, m), 4.30-4.62 (6H, m), 3.35-3.54 (2H, m), 3.05-3.25 (2H, m), 2.80-3.00 (2H, m), 2.08-2.28 (2H, m), 1.72-1.93 (2H, m), 1.83 (3H, brt, J=6.9Hz), 0.80-1.16 (12H, brs)

### Example 65

### Preparation of N-[1-(4-pentenyl)-4-[3-{2-(pyrrolidin-1-yl)-ethoxy}phenyl]-1,2-dihydro-2-oxo-1,8-naphthyridin-3-yl]-N'-(2,6-diisopropylphenyl)urea

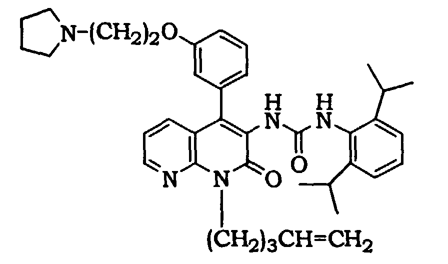

The title compound was obtained in the same manner as in Reference Example 12 from N-[1-(4-pentenyl)-4-(3-hydroxyphenyl)-1,2-dihydro-2-oxo-1,8-naphthyridin-3-yl]-N'-(2,6-diisopropylphenyl)urea and N-(2-chloroethyl)pyrrolidine hydrochloride.

### Hydrochloride:

¹H-NMR δ (DMSO-d₆) 10.62 (1H, brs), 8.57-8.69 (1H, m), 7.86 (2H, brs), 7.58-7.70 (1H, m], 7.38-7.51 (1H, m), 7.22-7.32 (1H, m), 6.88-7.21 (6H, m), 5.80-6.00 (1H, m), 4.92-5.20 (2H, m), 4.45-4.62 (2H, m), 4.26-4.42 (2H, m), 3.45-3.66 (4H, m), 2.78-3.20 (4H, m), 2.06-2.29 (2H, m), 1.69-2.05 (4H, m), 1.02 (12H, brs)

### Example 66

### Preparation of N-[1-(4-pentynyl)-4-(3-methoxyphenyl)-1,2-dihydro-2-oxo-1,8-naphthyridin-3-yl]-N'-(2,6-diisopropylphenyl)urea

### (a) Preparation of N-[1-(5-trimethylsilyl-4-pentynyl)-4-(3-methoxyphenyl)-1,2-dihydro-2-oxo-1,8-naphthyridin-3-yl]-N'(2,6-diisopropylphenyl)urea

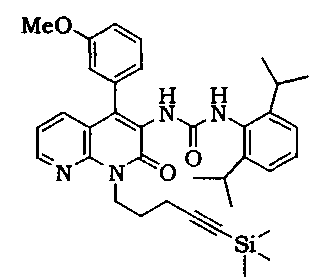

The title compound was obtained in the same manner as in Example 16 from N-[4-(3-methoxyphenyl)-1,2-dihydro-2-oxo-1,8-naphthyridin-3-yl]-N'-(2,6-diisopropylphenyl)urea and 5-(p-toluene-sulfonyloxy)-1-trimethylsilyl-1-pentyne.
¹H-NMR δ (CD₃OD) 8.65 (1H, dd, J=4.3Hz, 1.7Hz), 7.76 (1H, dd, J=7.9Hz, 1.7Hz), 7.49 (1H, dd, J=8.3Hz, 7.9Hz), 6.95-7.35 (7H, m), 4.70-7.83 (2H, m), 3.87 (3H, s), 2.95-3.13 (2H, m), 2.44 (2H, t, J=7.3Hz), 1.98-2.16 (2H, m), 1.15 (12H, brd, J=6.3Hz), 0.17 (9H, s)

### (b) Preparation of N-[1-(4-pentynyl)-4-(3-methoxyphenyl)-1,2-dihydro-2-oxo-1,8-naphthyridin-3-yl]-N'-(2,6-diisopropylphenyl)urea

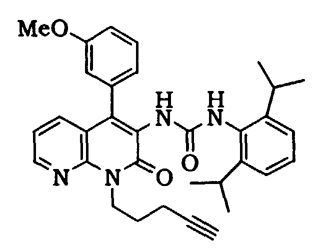

To a solution of N-[1-(5-trimethylsilyl-4-pentynyl)-4-(3-methoxyphenyl)-1,2-dihydro-2-oxo-1,8-naphthyridin-3-yl]-N'-(2,6-diisopropyl phenyl)urea (81 mg, 0.133 mmol) in DMF (4 ml) was added potassium fluoride (138 mg, 2.38 mmol), and the mixture was stirred at room temperature for 7 hours. The mixture was poured into water, and extracted with ethyl acetate. The extract was washed with a saturated aqueous sodium chloride solution, and dried over anhydrous magnesium sulfate. The resultant was concentrated under reduced pressure, and purified by preparative thin layer chromatography to give the title compound (58 mg, 0.14 mmol).
¹H-NMR δ (DMSO-d₆) 8.61 (1H, dd, J=8.1Hz, 1.5Hz), 7.75 (2H, brs), 7.62 (1H, dd, J=8.1Hz, 1.1Hz), 7.42 (1H, dd, J=8.1Hz, 8.1Hz), 7.26 (1H, dd, J=7.8Hz, 4.5Hz), 7.10-7.21 (1H, m), 6.98-7.10 (3H, m), 6.85-6.97 (2H, m), 4.42-4.70 (2H, m), 2.77-3.00 (2H, m), 2.82 (1H, t, J=2.4Hz), 2.24-2.40 (2H, m), 1.77-2.05 (2H, m), 1.02 (12H, brs)

### Example 67

### Preparation of N-[1-butyl-4-(3-fluorophenyl)- 1,2-dihydro-2-oxo-1,8-naphthyridin-3-yl]-N'-(2,6-diisopropylphenyl)urea

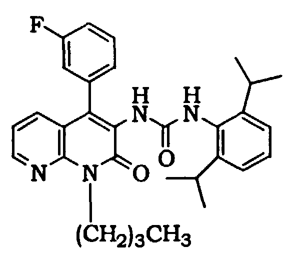

The title compound was obtained in the same manner as in Reference Example 10 from 1-butyl-4-(3-fluorophenyl)-1,2-dihydro-2-oxo-1,8-naphthyridine-3-carboxylic acid and 2,6-diisopropylaniline.
m.p. 201-202°C

### Example 68

### Preparation of N-[1-butyl-4-{3-(3-dipropylamino-1-propynyl)-phenyl}-1,2-dihydro-2-oxo-1,8-naphthyridin-3-yl]-N'-(2,6-diisopropylphenyl)urea

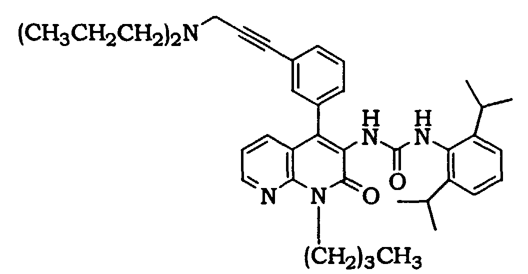

The title compound was obtained in the same manner as in Example 21 from N-[1-butyl-4-(3-bromophenyl)-1,2-dihydro-2-oxo-1,8-naphthyridin-3-yl]-N'-(2,6-diisopropylphenyl)urea and 3-dipropylamino-1-propyne.
Hydrochloride: m.p. 201-202°C

### Example 69

### Preparation of N-[1-butyl-4-{3-(3-amino-3-methyl-1-butynyl)-phenyl}-1,2-dihydro-2-oxo-1,8-naphthyridin-3-yl]-N'-(2,6-diisopropylphenyl)urea

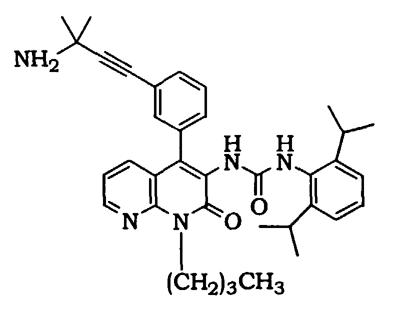

The title compound was obtained in the same manner as in Example 21 from N-[1-butyl-4-(3-bromophenyl)-1,2-dihydro-2-oxo-1,8-naphthyridin-3-yl]-N'-(2,6-diisopropylphenyl)urea and 3-amino-3-methyl-1-butyne.
Hydrochloride: m.p. 170-172°C

### Example 70

### Preparation of N-[1-butyl-4-[3-{3-(N-benzyl-N-methylamino)-1-propynyl)phenyl]-1,2-dihydro-2-oxo-1,8-naphthyridin-3-yl]-N'-(2,6-diisopropylphenyl)urea

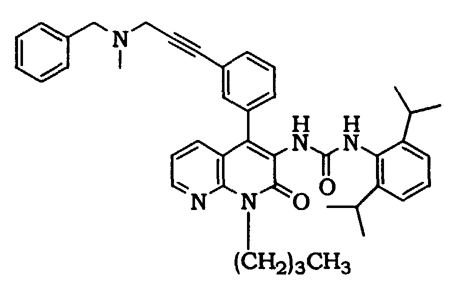

The title compound was obtained in the same manner as in Example 21 from N-[1-butyl-4-(3-bromophenyl)-1,2-dihydro-2-oxo-1,8-naphthyridin-3-yl]-N'-(2,6-diisopropylphenyl)urea and 3-(N-benzyl-N-methylamino)-1-propyne.
Hydrochloride: m.p. 140-142°C

### Example 71

### Preparation of N-[1-butyl-4-[3-{2-(aminocyclohexan-1-yl)-ethynyl)phenyl]-1,2-dihydro-2-oxo-1,8-naphthyridin-3-yl]-N'-(2,6-diisopropylphenyl)urea

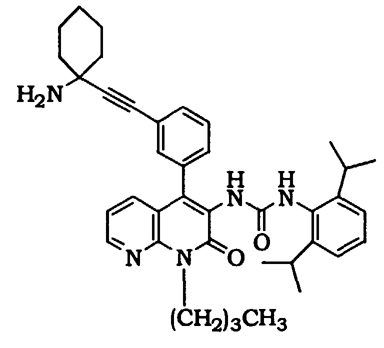

The title compound was obtained in the same manner as in Example 21 from N-[1-butyl-4-(3-bromophenyl)-1,2-dihydro-2-oxo-1,8-naphthyridin-3-yl]-N'(2,6-diisopropylphenyl)urea and 1-ethynyl-cyclohexylamine.
Hydrochloride: m.p. 171-172°C

### Example 72

### Preparation of N-[1-butyl-4-(3-(3-amino-3-ethyl-1-pentynyl)-phenyl}-1,2-dihydro-2-oxo-1,8-naphthyridin-3-yl]-N'-(2,6-diisopropylphenyl)urea

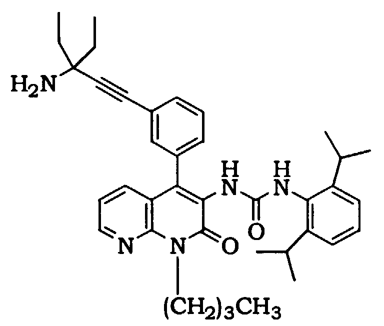

The title compound was obtained in the same manner as in Example 21 from N-[1-butyl-4-(3-bromophenyl)-1,2-dihydro-2-oxo-1,8-naphthyridin-3-yl]-N'-(2,6-diisopropylphenyl)urea and 3-amino-3-ethyl-1-pentyne.
Hydrochloride: m.p. 164- 165°C

### Example 73

### Preparation of N-[1-butyl-4-{3-(3-tert-butoxycarbonylamino-1-propynyl)phenyl}-1,2-dihydro-2-oxo-1,8-naphthyridin-3-yl]-N'-(2,6-diisopropylphenyl)urea

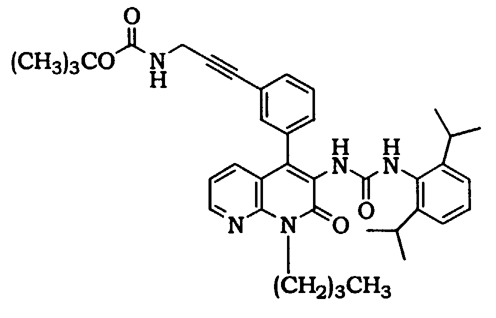

The title compound was obtained in the same manner as in Example 21 from N-[1-butyl-4-(3-bromophenyl)-1,2-dihydro-2-oxo-1,8-naphthyridin-3-yl]-N'-(2,6-diisopropylphenyl)urea and 3-tert-butoxy-carbonylamino-1-propyne.
¹H-NMR δ (CDCl₃) 8.61 (1H, dd, J=4.6Hz, 1.6Hz), 7.89 (1H, br), 7.81 (1H, br), 7.01-7.56 (9H, m), 4.50-4.53 (2H, m), 3.95-3.97 (2H, m), 2.71-2.92 (2H, m), 1.71-1.80 (2H, m), 1.30-1.51 (11H, m), 0.94-1.07 (12H, m)

### Example 74

### Preparation of N-[1-butyl-4-{3-(3-amino-1-propynyl)phenyl}-1,2-dihydro-2-oxo-1,8-naphthyridin-3-yl]-N'-(2,6-diisopropylphenyl)urea

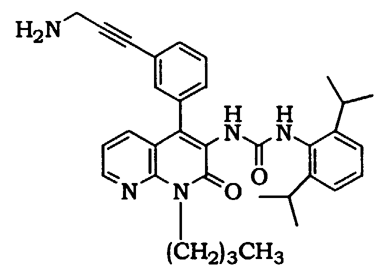

To a solution of N-[1-butyl-4-{3-(3-tert-butoxycarbonylamino-1-propynyl)phenyl}-1,2-dihydro-2-oxo-1,8-naphthyridin-3-yl]-N'-(2,6-diisopropylphenyl)urea (104 mg, 0.16 mmol) in chloroform (5 ml) was added trifluoroacetic acid (1 ml) under ice-cooling. The mixture was warmed to room temperature, and then stirred for 1.5 hour. The mixture was concentrated under reduced pressure to remove the solvent, and to the residue was added 5 % aqueous sodium chloride solution. The mixture was treated with aqueous ammonia to be converted into a free form, and then extracted with ethyl acetate. The oily layer was washed with 5 % aqueous sodium chloride solution, and dried over anhydrous magnesium sulfate. The resultant was concentrated under reduced pressure, and the residue was purified by silica gel column chromatography (4 % methanol in chloroform) to give the title compound (64 mg, 0.12 mmol).

### Hydrochloride:

¹H-NMR δ (DMSO-d₆) 8.62 (1H, d, J=3.7Hz), 8.35 (2H, brs), 8.03 (1H, s), 7.90 (1H, s), 7.54 (1H, s), 7.52 (2H, s), 7.41-7.42 (2H, m), 7.27 (1H, dd, J=8. 1Hz, 4.8Hz), 7.13-7.19 (1H, m), 7.03 (2H, d, J=7.9Hz), 4.52-4.57 (2H, m), 3.98 (2H, d, J=4.8Hz), 2.85 (1H, brs), 2.76 (1H, brs), 1.68-1.78 (2H, m), 1.40-1.47 (2H, m), 0.92-1.04 (15H, m)

### Example 75

### Preparation of N-[1-butyl-4-{3-(3-methylamino-3-methyl-1-butynyl)phenyl}-1,2-dihydro-2-oxo-1,8-naphthyridin-3-yl]-N'-(2,6-diisopropylphenyl)urea

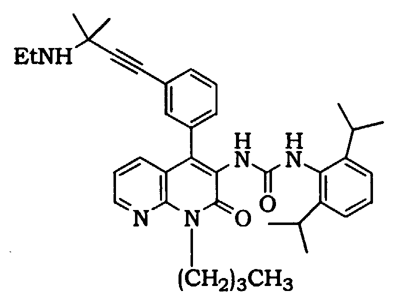

To a solution of N-[1-butyl-4-{3-(3-amino-3-methyl-1-butynyl)-phenyl}-1,2-dihydro-2-oxo-1,8-naphthyridin-3-yl]-N'-(2,6-diisopropylphenyl)urea (30 mg, 0.05 mmol) in ethanol (1 ml) were added acetaldehyde (22 mg, 0.49 mmol) and sodium cyanoborohydride (9 mg, 0.147 mmol), and the mixture was stirred for two hours. To the mixture was added ice-water, and the mixture was basified with conc. aqueous ammonia, and extracted with ethyl acetate. The extract was washed with a saturated aqueous sodium chloride solution, and dried over anhydrous magnesium sulfate. The resultant was concentrated under reduced pressure, purified by silica gel column chromatogpraphy (2 % methanol in chloroform), and then converted into a hydrochloride thereof to give the title compound (24 mg, 0.04 mmol).

### Hydrochloride:

¹H-NMR δ (DMSO-d₆) 9.36 (2H, brs), 8.62 (1H, d, J=3.9Hz), 8.03 (1H, s), 7.89 (1H, s), 7.51-7.55 (3H, m), 7.40-7.44 (2H, m), 7.25-7.29 (1H, m), 7.13-7.19 (1H, m), 7.04 (2H, d, J=7.5Hz), 4.54 (2H, t, J=7.5Hz), 3.12 (2H, brs), 2.76 (2H, brs), 1.66-1.75 (2H, m), 1.66 (6H, s), 1.40-1.47 (2H, m), 1.25 (3H, t, J=7.2Hz), 0.95-1.14 (15H, m)

### Example 76

### Preparation of N-[1-butyl-4-{3-(3-dimethylamino-1-propynyl)-phenyl}-1,2-dihydro-2-oxo-1,8-naphthyridin-3-yl]-N'-(2,6-diisopropylphenyl)urea

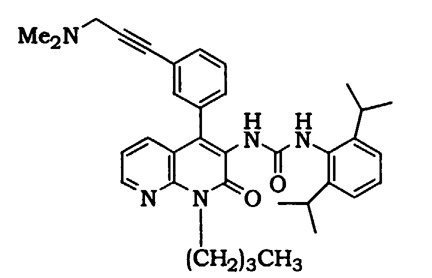

The title compound was obtained in the same manner as in Example 75 from N-[1-butyl-4-{3-(3-amino-1-propynyl)phenyl}-1,2-dihydro-2-oxo-1,8-naphthyridin-3-yl]-N'-(2,6-diisopropylphenyl)urea and formaldehyde.

### Hydrochloride:

¹H-NMR δ (DMSO-d₆) 10.62 (1H, brs), 8.62-8.63 (1H, m), 8.00 (1H, s), 7.89 (1H, s), 7.50-7.63 (4H, m), 7.43 (1H, d, J=7.3Hz), 7.27 (1H, dd, J=7.7Hz, J=4.4Hz), 7.13-7.18 (1H, m), 7.03 (2H, d, J=7.5Hz), 4.52-4.57 (2H, m), 4.32 (2H, s), 2.84 (2H, s), 2.73-2.84 (2H, m), 1.70-1.75 (2H, m), 1.39-1.47 (2H, m), 0.95-1.11 (15H, m)

### Example 77

### Preparation of N-[1-butyl-4-{3-(3-diethylamino-3-methyl-1-butynyl)phenyl}-1,2-dihydro-2-oxo-1,8-naphthyridin-3-yl]-N'-(2,6-diisopropylphenyl)urea

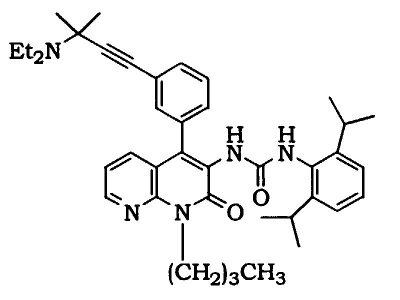

To a solution of N-[1-butyl-4-{3-(3-amino-3-methyl-1-butynyl)-phenyl}-1,2-dihydro-2-oxo-1,8-naphthyridin-3-yl]-N'-(2,6-diisopropylphenyl)urea (126 mg, 0.22 mmol) in DMF were added potassium carbonate (300 mg, 2.18 mmol) and iodoethane (102 mg, 0.654 mmol), and the mixture was stirred at room temperature for one hour. The mixture was warmed to 50°C, and then stirred for five hours. The mixture was further stirred at room temperature for 12 hours, and poured into water. The mixture was extracted with ethyl acetate, and the extract was washed with 5 % aqueous sodium hydrogen carbonate solution, 5 % aqueous sodium chloride solution, and concentrated under reduced pressure. The residue was purified by silica gel column chromatography (1 % methanol in chloroform) to give the title compound (25 mg, 0.04 mmol).
Hydrochloride: m.p. 144-146°C

### Example 78

### Preparation of N-[1-butyl-4-{3-(3-diethylamino-3-ethyl-1-pentynyl)phenyl}-1,2-dihydro-2-oxo-1,8-naphthyridin-3-yl]-N'-(2,6-diisopropylphenyl)urea

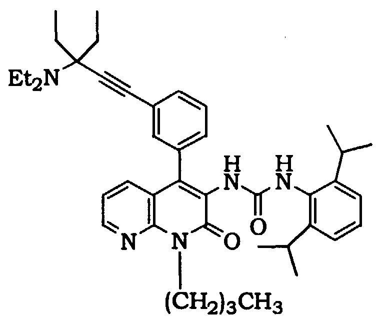

The title compound was obtained in the same manner as in Example 77 from N-[1-butyl-4-{3-(3-amino-3-ethyl-1-pentynyl)phenyl}-1,2-dihydro-2-oxo-1,8-naphthyridin-3-yl]-N'-(2,6-diisopropylphenyl)urea and iodoethane.
Hydrochloride: m.p. 137- 139°C

### Example 79

### Preparation of N-[1-butyl-4-[3-{2-(N,N-diethylaminocyclohexan-1-yl)ethynyl}phenyl]-1,2-dihydro-2-oxo-1,8-naphthyridin-3-yl]-N'-(2,6-diisopropylphenyl)urea

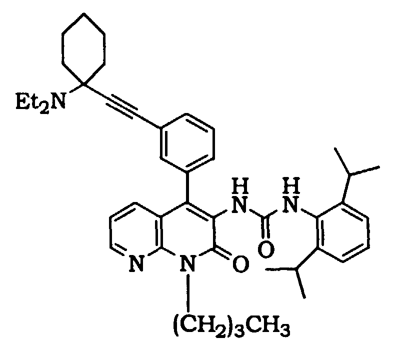

The title compound was obtained in the same manner as in Example 77 from N-[1-butyl-4-[3-{2-(aminocyclohexan-1-yl)ethynyl}phenyl]-1,2-dihydro-2-oxo-1,8-naphthyridin-3-yl]-N'-(2,6-diisopropylphenyl)urea and iodoethane.
Hydrochloride: m.p. 208-212°C

### Example 80

### Preparation of N-[1-butyl-4-{3-(3-methylamino-1-propynyl)phenyl]-1,2-dihydro-2-oxo-1,8-naphthyridin-3-yl]-N'-(2,6-diisopropylphenyl)urea

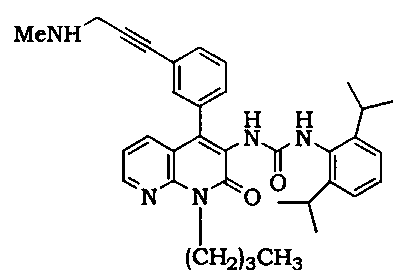

The title compound was obtained in the same manner as in Example 21 from N-[1-butyl-4-(3-bromophenyl)-1,2-dihydro-2-oxo-1,8-naphthyridin-3-yl]-N'-(2,6-diisopropylphenyl)urea and 3-methylamino-1-propyne.

### Hydrochloride:

¹H-NMR δ (DMSO-d₆) 9.19 (2H, brs), 8.61-8.63 (1H, m), 8.02 (1H, s), 7.90 (1H, s), 7.55 (2H, s), 7.53 (1H, s), 7.44 (1H, s), 7.42 (1H, brs), 7.25-7.29 (1H, m), 7.13-7.18 (1H, m), 7.03 (2H, d, J=7.7Hz), 4.52-4.57 (2H, m), 4.11-4.15 (2H, m), 2.73-2.76 (2H, m), 2.62 (3H, t, J=5.5Hz), 1.71-1.76 (2H, m), 1.40-1.47 (2H, m), 0.96-1.12 (15H, m)

### Example 81

### Preparation of N-[1-butyl-4-{3-(4-chlorobutoxy)phenyl}-1,2-dihydro-2-oxo-1,8-naphthyridin-3-yl]-N'-(2,6-diisopropylphenyl)urea

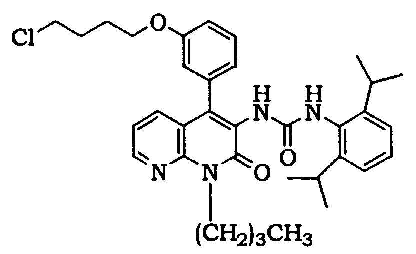

The title compound was obtained in the same manner as in Reference Example 12 from N-[1-butyl-4-(3-hydroxyphenyl)-1,2-dihydro-2-oxo-1,8-naphthyridin-3-yl]-N'-(2,6-diisopropylphenyl)urea and 1-bromo-3-chlorobutane.
¹H-NMR δ (DMSO-d₆) 8.59 (1H, dd, J=4.6Hz, 1.7Hz), 7.76 (1H, brs), 7.74 (1H, brs), 7.62 (1H, d, J=6.6Hz), 7.24 (1H, dd, J=7.9Hz, 4.6Hz), 7.12-7.18 (1H, m), 6.97-7.02 (3H, m), 6.89 (2H, br), 4.52 (2H, t, J=7.3Hz), 4.00 (2H, br), 3.67 (2H, br), 2.91 (2H, m), 1.86 (2H, m), 1.72 (2H, br), 1.42 (2H, m), 1.02 (12H, br), 0.97 (3H, t, J=7.3Hz)

### Example 82

### Preparation of N-[1-butyl-4-[3-{4-(1,2,4-triazol-1-yl)butoxy}phenyl]-1,2-dihydro-2-oxo-1,8-naphthyridin-3-yl]-N'-(2,6-diisopropylphenyl)urea

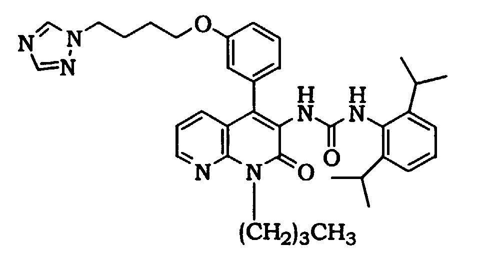

To a solution of N-[1-butyl-4-{3-(4-chlorobutoxy)phenyl}-1,2-dihydro-2-oxo-1,8-naphthyridin-3-yl]-N'-(2,6-diisopropylphenyl)urea (5.5 g, 9.13 mmol) in DMF (100 ml) were added potassium carbonate (1.89 g, 13.7 mmol), potassium iodide (0.30 g, 1.83 mmol) and 1,2,4-triazole (0.94 g, 13.7 mmol), and the mixture was stirred at 40-50°C for 11 hours. The mixture was poured into water, and the mixture is extracted with ethyl acetate. The extract was washed with water and a saturated aqueous sodium chloride solution, and concentrated under reduced pressure. The residue was purified by silica gel column chromatography to give the title compound in a free form (5.37 g, 8.46 mmol).

### Hydrochloride:

¹H-NMR δ (DMSO-d₆) 8.60 (1H, dd, J=4.6Hz, 1.7Hz), 8.50 (1H, s), 7.93 (1H, s), 7.77 (1H, brs), 7.74 (1H, brs), 7.61 (1H, dd, J=8.1Hz, 1.7Hz), 7.39 (1H, dd, J=7.9Hz, 7.9Hz), 7.24 (1H, dd, J=7.9Hz, 4.6Hz), 7.13 (1H, m), 6.98-7.04 (3H, m), 6.86-6.91 (2H, m), 4.52 (2H, t, J=7.3Hz), 4.23 (2H, t, J=6.8Hz), 3.98 (2H, t, J=6. 1Hz), 2.90 (2H, m), 1.93 (2H, m), 1.65-1.72 (4H, m), 1.43 (2H, m), 1.00 (12H, br), 0.97 (3H, t, J=7.5Hz)

### Example 83

### Preparation of N-[1-butyl-4-[3-{2-(p-toluenesulfonyloxy)ethoxy}-phenyl]-1,2-dihydro-2-oxo-1,8-naphthyridin-3-yl]-N'-(2,6-diisopropylphenyl)urea

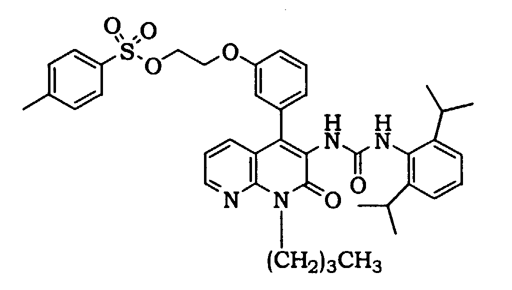

To a solution of N-[1-butyl-4-{3-(2-hydroxyethoxy)phenyl}-1,2-dihydro-2-oxo-1,8-naphthyridin-3-yl]-N'-(2,6-diisopropylphenyl)urea (2.02 g, 3.63 mmol) in THF (20 ml) were added p-toluenesulfonyl chloride (0.69 g, 3.63 mmol) and triethylamine (0.40 g, 3.99 mmol), and the mixture was stirred at 40-50°C for 8 hours. The mixture was poured into water, and extracted with ethyl acetate. The extract was washed with water, washed with a saturated aqueous sodium chloride solution, and dried over anhydrous magnesium sulfate. The resultant was concentrated under reduced pressure, and the residue was purified by silica gel column chromatography (ethyl acetate:hexane = 1:1) to give the title compound (1.40 g, 1.97 mmol).
¹H-NMR δ (DMSO-d₆) 8.60 (1H, dd, J=4.6Hz, 1.5Hz), 7.74-7.80 (4H, m), 7.57 (1H, d, J=7.0Hz), 7.45 (2H, d, J=8.4Hz), 7.37 (1H, dd, J=8.1Hz, 7.9Hz), 7.24 (1H, dd, J=7.9Hz, 4.8Hz), 7.15 (1H, d, J=7.5Hz), 7.03 (2H, d, J=7.5Hz), 6.90-6.94 (2H, m), 6.75 (1H, br), 4.52 (2H, t, J=7.3Hz), 4.34 (2H, br), 4.15 (2H, br), 2.88 (2H, m), 1.70 (2H, m), 1.42 (2H, m), 0.99 (12H, br), 0.97 (3H, t, J=7.2Hz)

### Example 84

### Preparation of N-[1-butyl-4-[3-{2-(1,2,4-triazol-1-yl)ethoxy}-phenyl]-1,2-dihydro-2-oxo-1,8-naphthyridin-3-yl]-N'-(2,6-diisopropylphenyl)urea

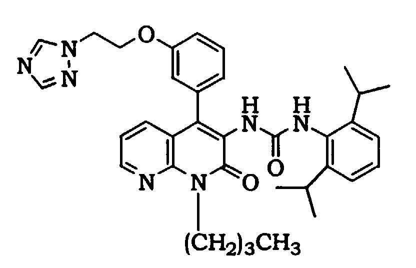

The title compound was obtained in the same manner as in Example 82 from N-[1-butyl-4-[3-{2-(p-toluenesulfonyloxy)ethoxy}-phenyl]-1,2-dihydro-2-oxo-1,8-naphthyridin-3-yl]-N'-(2,6-diisopropylphenyl)urea and 1,2,4-triazole.

### Hydrochloride:

¹H-NMR δ (DMSO-d₆) 8.64 (1H, brs), 8.60 (1H, dd, J=4.6Hz, 1.7Hz), 8.04 (1H, brs), 7.79 (1H, brs), 7.76 (1H, brs), 7.58 (1H, dd, J=8.1Hz, 1.7Hz), 7.38 (1H, dd, J=7.9Hz, 7.9Hz), 7.24 (1H, dd, J=8.1Hz, 4.6Hz), 7.14 (1H, t, J=7.5Hz), 6.98-7.03 (3H, m), 6.88-6.93 (2H, m), 4.59 (2H, m), 4.52 (2H, t, J=7.5Hz), 2.85 (2H, m), 1.70 (2H, m), 1.43 (2H, m), 0.97 (15H, br)

### Example 85

### Preparation of N-[1-butyl-4-{3-(2-propynyloxy)phenyl}-1,2-dihydro-2-oxo-1,8-naphthyridin-3-yl]-N'-(2,6-diisopropylphenyl)urea

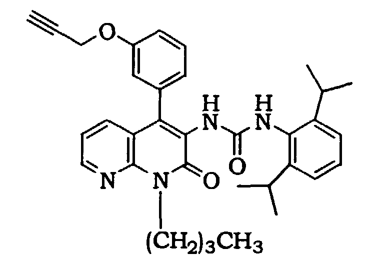

The title compound was obtained in the same manner as in Reference Example 12 from N-[1-butyl-4-(3-hydroxyphenyl)-1,2-dihydro-2-oxo-1,8-naphthyridin-3-yl]-N'-(2,6-diisopropylphenyl)urea and propargyl bromide.
¹H-NMR δ (DMSO-d₆) 8.61 (1H, d, J=4.6Hz), 7.77 (1H, s), 7.75 (1H, s), 7.64 (1H, d, J=6.3Hz), 7.43 (1H, dd, J=8.3Hz, 7.9Hz), 7.25 (1H, dd, J=8.3Hz, 4.6Hz), 6.96-7.18 (6H, m), 4.79 (2H, d, J=2.3Hz), 4.52 (2H, t, J=7.3Hz), 3.60 (1H, s), 2.92 (2H, m), 1.70 (2H, m), 1.46 (2H, m), 1.03 (12H, br), 1.00 (3H, t, J=7.3Hz)

### Example 86

### Preparation of N-[1-butyl-4-]3-{(4-diethylamino-2-butynyl)oxy}phenyl]-1,2-dihydro-2-oxo-1,8-naphthyridin-3-yl]-N'-(2,6-diisopropylphenyl)urea

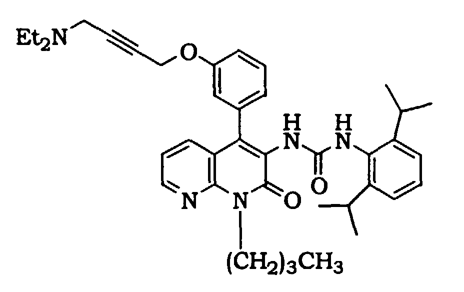

To a solution of N-[1-butyl-4-{3-(2-propynyloxy)phenyl}-1,2-dihydro-2-oxo-1,8-naphthyridin-3-yl]-N'-(2,6-diisopropylphenyl)urea (1.0 g, 1.82 mmol) in dioxane (10 ml) were added paraformaldehyde (0.37 g), diethylamine (0.26 g, 3.63 mmol) and copper (I) iodide, and the mixture was stirred at room temperature for two hours. To the mixture was added ether, and the mixture was filtered through a cerite pad. The filtrate was concentrated under reduced pressure, and the residue was purified by silica gel column chromatography (2% methanol in chloroform) to give the title compound (1.05 g, 1.65 mmol).
¹H-NMR δ (DMSO-d₆) 8.61 (1H, dd, J=4.2Hz, 1.7Hz), 7.78 (1H, brs), 7.76 (1H, brs), 7.62 (1H, dd, J=8. 1Hz, 1.5Hz), 7.42 (1H, dd, J=7.9Hz, 7.9Hz), 7.24 (1H, dd, J=7.9Hz, 4.6Hz), 6.94-7.18 (5H, m), 4.81 (2H, brs), 4.53 (2H, t, J=7.4Hz), 3.37 (2H, brs), 2.94 (2H, m), 2.33 (4H, q, J=7.2Hz), 1.70 (2H, m), 1.44 (2H, m), 1.03 (12H, br), 0.97 (3H, t, J=7.3Hz), 0.86 (6H, t, J=7.2Hz)

### Example 87

### Preparation of N-[1-butyl-4-[3-{(cis-4-diethylamino-2-butenyl)oxy)phenyl]-1,2-dihydro-2-oxo-1,8-naphthyridin-3-yl]-N'-(2,6-diisopropylphenyl)urea

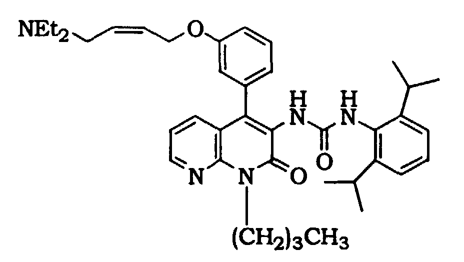

A suspension of N-[1-butyl-4-[3-{(4-diethylamino-2-butynyl)oxy}phenyl]-1,2-dihydro-2-oxo-1,8-naphthyridin-3-yl]-N'-(2,6-diisopropylphenyl)urea (300 mg, 0.45 mmol), a Lindlar catalyst (distributed by Sigma Alderich Japan, 20 mg) in methanol (20 ml) was stirred at room temperature under hydrogen atmosphere for six hours. The mixture was filtered through a cerite pad, and the filtrate was concentrated under reduced pressure. The resultant was dissolved in chloroform, and the mixture was washed with a dilute aqueous ammonia, washed with water, washed with a saturated aqueous sodium chloride solution, dried over anhydrous magnesium sulfate, and concentrated under reduced pressure. The residue was purified by silica gel column chromatography (3-8 % methanol in chloroform) to give the title compound (280 mg, 0.43 mmol).
¹H-NMR δ (DMSO-d₆) 8.62 (1H, dd, J=4.6Hz, 1.8Hz), 7.75 (1H, s), 7.62 (1H, dd, J=8.1Hz, 1.8Hz), 7.40 (1H, dd, J=8.1Hz, 8.1Hz), 7.25 (1H, dd, J=8.8Hz, 4.6Hz), 7.15 (1H, t, J=7.6Hz), 7.02-7.05 (3H, m), 6.90-6.93 (2H, m), 5.75 (1H, br), 5.67 (1H, dt, J=11.2Hz, J=6.2Hz), 4.65 (2H, br), 4.52 (2H, t, J=7.4Hz), 3.13 (2H, br), 2.91 (2H, m), 2.45 (4H, br), 1.71 (2H, m), 1.42 (2H, m), 0.90-1.08 (2H, m)

### Example 88

### Preparation of N-[1-butyl-4-{3-(cis-3-diethylamino-1-propenyl)phenyl}-1,2-dihydro-2-oxo-1,8-naphthyridin-3-yl]-N'-(2,6-diisopropylphenyl)urea

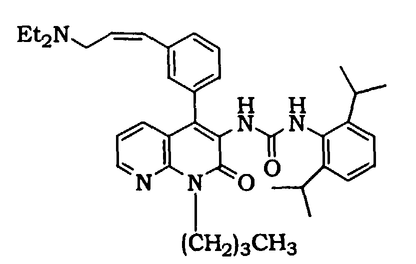

The title compound was obtained in the same manner as in Example 87 from N-[1-butyl-4-(3-diethylamino-1-propynylphenyl)-1,2-dihydro-2-oxo-1,8-naphthyridin-3-yl]-N'-(2,6-diisopropylphenyl)urea.
¹H-NMR δ (CD₃OD) 8.60 (1H, m), 7.66 (1H, d, J=6.6Hz), 7.53 (1H, dd, J=7.7Hz, 7.7Hz), 7.38 (1H, d, J=7.5Hz), 7.36 (2H, m), 7.15-7.22 (2H, m), 7.06 (2H, d, J=7.3Hz), 6.72 (1H, d, J=11.6Hz), 5.81 (1H, dt, J=11.6Hz, 5.1Hz), 4.64 (2H, b, J=7.5Hz), 3.47 (2H, d, J=5.1Hz), 2.90 (2H, br), 2.50 (4H, m), 1.80 (2H, m), 1.50 (2H, m), 0.92-1.09 (21H, m)

### Example 89

### Preparation of N-[1-butyl-4-{3-(3-bromo-1-propynyl)phenyl}-1,2-dihydro-2-oxo-1,8-naphthyridin-3-yl]-N'-(2,6-diisopropylphenyl)urea

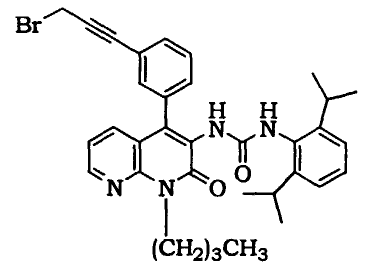

To a solution of N-[1-butyl-4-{3-(3-hydroxy-1-propynyl)phenyl}-1,2-dihydro-2-oxo-1,8-naphthyridin-3-yl]-N'-(2,6-diisopropylphenyl)urea (90 mg, 0.16 mmol) in methylene chloride (1.5 ml) were added carbon tetrabromide (81 mg, 0.245 mmol) and triphenylphosphine (51 mg, 0.196 mmol) under ice-cooling, and the mixture was stirred for 30 minutes. To the mixture was added water, and the mixture was extracted with ethyl acetate. The extract was washed with a saturated aqueous sodium chloride solution, dried over anhydrous magnesium sulfate, and concentrated under reduced pressure. The residue was purified by silica gel column chromatography (ethyl acetate:hexane: 1:5) to give the title compound (53 mg, 0.86 mmol).
¹H-NMR δ (CD₃OD) 8.58 (1H, d, J=3.9Hz), 7.66 (1H, d, J=7.9Hz), 7.38-7.56 (4H, m), 7.15-7.17 (2H, m), 7.07 (2H, d, J=7.5Hz), 4.64 (2H, t, J=6.8Hz), 4.27 (2H, s), 1.73-1.81 (2H, m), 1.42-1.52 (2H, m), 1.10-1.11 (12H, m), 1.01 (3H, t, J=7.3Hz)

### Example 90

### Preparation of N-[1-butyl-4-[3-{3-(1-pyrrolidinyl)-1-propynyl)phenyl}-1,2-dihydro-2-oxo-1,8-naphthyridin-3-yl)-N'-(2,6-diisopropylphenyl)urea

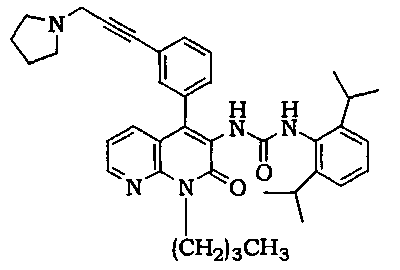

To a solution of N-[1-butyl-4-{3-(3-bromo-1-propynyl)phenyl}-1,2-dihydro-2-oxo-1,8-naphthyridin-3-yl]-N'-(2,6-diisopropylphenyl)urea (400 mg, 0.65 mmol) in THF (5 ml) was added pyrrolidine (139 mg, 1.96 mmol), and the mixture was stirred at room temperature for four hours. To the mixture was added water, and the mixture was extracted with ethyl acetate. The extract was washed with water, washed with a saturated aqueous sodium chloride solution, dried over anhydrous magnesium sulfate, and concentrated under reduced pressure. The residue was purified by silica gel column chromatography (ethyl acetate) to give the title compound (340 mg, 0.56 mmol).

### Hydrochloride:

¹H-NMR δ (DMSO-d₆) 11.04 (1H, br), 8.62 (1H, d, J=4.6Hz), 8.01 (1H, brs), 7.90 (1H, brs), 7.41-7.61 (5H, m), 7.27 (1H, dd, J=7.9Hz, 4.6Hz), 7.16 (1H, t, J=7.5Hz), 7.03 (2H, d, J=7.5Hz), 4.54 (2H, t, J=7.5Hz), 4.38 (2H, s), 3.53 (2H, br), 3.14 (2H, br), 2.89 (2H, br), 2.00 (4H, br), 1.73 (2H, m), 1.43 (2H, m), 0.95-1.09 (15H, m)

### Example 91

### Preparation of N-[1-butyl-4-[3-{3-(N,N-dihexylamino)-1-propynyl)phenyl}-1,2-dihydro-2-oxo-1,8-naphthyridin-3-yl]-N'-(2,6-diisopropylphenyl)urea

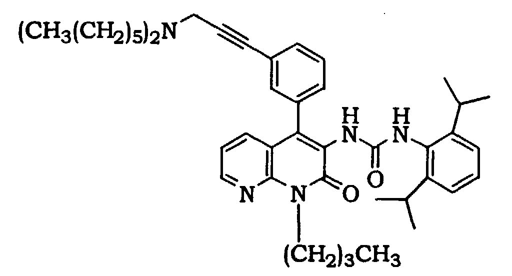

The title compound was obtained in the same manner as in Example 90 from N-[1-butyl-4-{3-(3-bromo-1-propynyl)phenyl}-1,2-dihydro-2-oxo-1,8-naphthyridin-3-yl]-N'-(2,6-diisopropylphenyl)urea and dihexylamine.

### Hydrochloride:

¹H-NMR δ (DMSO-d₆) 8.61 (1H, m), 8.04 (1H, brs), 7.90 (1H, brs), 7.42-7.59 (5H, m), 7.26 (1H, m), 7.15 (1H, t, J=7.2Hz), 7.02 (2H, d, J=7.2Hz), 4.54 (2H, t, J=7.7Hz), 4.35 (2H, br), 3.14 (4H, br), 2.77 (2H, br), 1.70 (6H, br), 1.43 (2H, m), 1.28 (12H, br), 0.95-1.03 (15H, m), 0.83 (6H, br)

### Example 92

### Preparation of N-[1-butyl-4-[3-{3-(N-benzyl-N-ethylamino)-1-propynyl}phenyl]-1,2-dihydro-2-oxo-1,8-naphthyridin-3-yl]-N'-(2,6-diisopropylphenyl)urea

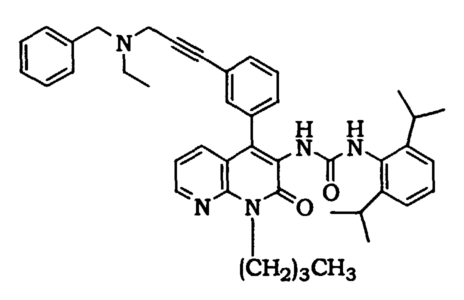

The title compound was obtained in the same manner as in Example 90 from N-[1-butyl-4-{3-(3-bromo-1-propynyl)phenyl})-1,2-dihydro-2-oxo-1,8-naphthyridin-3-yl]-N'-(2,6-diisopropylphenyl)urea and N-ethylbenzylamine.

### Hydrochloride:

¹H-NMR δ (DMSO-d₆) 11.16 (1H, br), 8.62 (1H, d, J=4.2Hz), 8.03 (1H, brs), 7.91 (1H, brs), 7.45-7.63 (10H, m), 7.37 (1H, dd, J=7.7Hz, J=4.4Hz), 7.15 (1H, t, J=7.5Hz), 7.03 (2H, d, J=7.5Hz), 4.55 (2H, t, J=7.4Hz), 4.26-4.43 (3H, m), 4.10 (1H, brd, J=18Hz), 3.21 (2H, br), 2.80 (2H, m), 1.73 (2H, m), 1.43 (2H, m), 1.32 (3H, br), 0.96-1.08 (15H, m)

### Example 93

### Preparation of N-[1-butyl-4-[3-{3-(1-piperidinyl)-1-propynyl)-phenyl}-1,2-dihydro-2-oxo-1,8-naphthyridin-3-yl]-N'-(2,6-diisopropylphenyl)urea

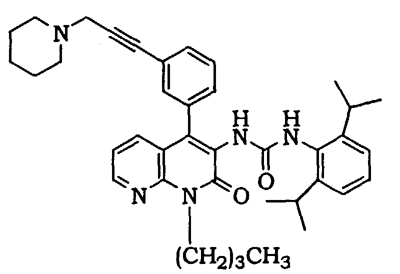

The title compound was obtained in the same manner as in Example 90 from N-[1-butyl-4-{3-(3-bromo-1-propynyl)phenyl}-1,2-dihydro-2-oxo-1,8-naphthyridin-3-yl]-N'-(2,6-diisopropylphenyl)urea and piperidine.
Hydrochloride: m.p. 160- 162°C

### Example 94

### Preparation of N-[1-butyl-4-[3-{3-(4-methyl-1-piperazinyl)-1-propynyl}phenyl]-1,2-dihydro-2-oxo-1,8-naphthyridin-3-yl]-N'-(2,6-diisopropylphenyl)urea

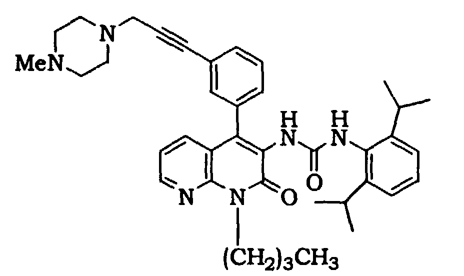

The title compound was obtained in the same manner as in Example 90 from N-[1-butyl-4-{3-(3-bromo-1-propynyl)phenyl}-1,2-dihydro-2-oxo-1,8-naphthyridin-3-yl]-N'-(2,6-diisopropylphenyl)urea and 1-methylpiperazine.
Hydrochloride: m.p. 157-161°C

### Example 95

### Preparation of N-[1-butyl-4-[3-{3-(N,N-dibenzylamino)-1-propynyl}phenyl]-1,2-dihydro-2-oxo-1,8-naphthyridin-3-yl]-N'-(2,6-diisopropylphenyl)urea

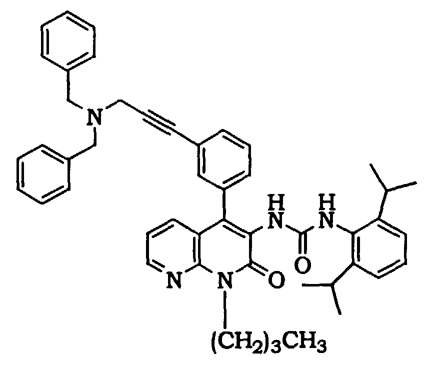

The title compound was obtained in the same manner as in Example 90 from N-[1-butyl-4-{3-(3-bromo-1-propynyl)phenyl}-1,2-dihydro-2-oxo-1,8-naphthyridin-3-yl]-N'-(2,6-diisopropylphenyl)urea and dibenzylamine.
Hydrochloride: IR (KBr) 2962, 2870, 1704, 1646, 1585, 1500, 1456 cm⁻¹

### Example 96

### Preparation of N-[1-butyl-4-[3-{3-(1-homopiperidinyl)-1-propynyl}phenyl]-1,2-dihydro-2-oxo-1,8-naphthyridin-3-yl]-N'-(2,6-diisopropylphenyl)urea

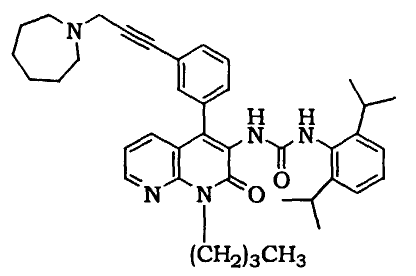

The title compound was obtained in the same manner as in Example 90 from N-[1-butyl-4-{3-{3-bromo-1-propynyl)phenyl}-1,2-dihydro-2-oxo-1,8-naphthyridin-3-yl]-N'-(2,6-diisopropylphenyl)urea and homopiperidine.

### Hydrochloride:

¹H-NMR δ (DMSO-d₆) 10.88 (1H, br), 8.62 (1H, dd, J=4.6Hz, 1.7Hz), 8.02 (1H, brs), 7.91 (1H, brs), 7.51-7.62 (3H, m), 7.49 (1H, br), 7.42 (1H, d, J=7.5Hz), 7.26 (1H, dd, J=8.1Hz, 4.6Hz), 7.15 (1H, t, J=7.5Hz), 7.03 (2H, d, J=7.5Hz), 4.54 (2H, t, J=7.3Hz), 4.34 (2H, d, J=4.2Hz), 3.48 (2H, m), 3.20 (3H, m), 2.79 (2H, br), 1.85 (4H, br), 1.57-1.78 (6H, m), 1.43 (2H, m), 0.96-1.03 (15H, m)

### Example 97

### Preparation of N-[1-butyl-4-[3-{3-(4-benzyl-1-piperazinyl)-1-propynyl}phenyl]-1,2-dihydro-2-oxo-1,8-naphthyridin-3-yl]-N'-(2,6-diisopropylphenyl)urea

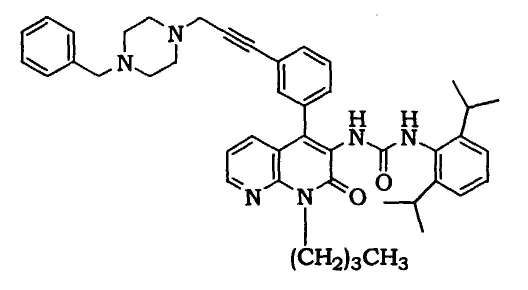

The title compound was obtained in the same manner as in Example 90 from N-[1-butyl-4-{3-(3-bromo-1-propynyl)phenyl}-1,2-dihydro-2-oxo-1,8-naphthyridin-3-yl]-N'-(2,6-diisopropylphenyl)urea and 1-benzylpiperazine.

### Hydrochloride:

¹H-NMR δ (DMSO-d₆) 8.61 (1H, dd, J=4.6Hz, 1.3Hz), 7.96 (1H, brs), 7.91 (1H, brs), 7.37-7.64 (11H, m), 7.26 (1H, dd, J=7.9Hz, 4.6Hz), 7.15 (1H, t, J=7.7Hz), 7.03 (2H, d, J=7.7Hz), 4.53 (2H, t, J=7.2Hz), 4.33 (2H, br), 4.04 (2H, br), 3.23-3.37 (8H, br), 2.81 (2H, m), 1.74 (2H, m), 1.42 (2H, m), 0.95-1.03 (15H, m)

### Example 98

### Preparation of N-[1-butyl-4-[3-{3-(3-azabicyclo[3.3.2]nonan-3-yl)-1-propynyl}phenyl]-1,2-dihydro-2-oxo-1,8-naphthyridin-3-yl]-N'-(2,6-diisopropylphenyl)urea

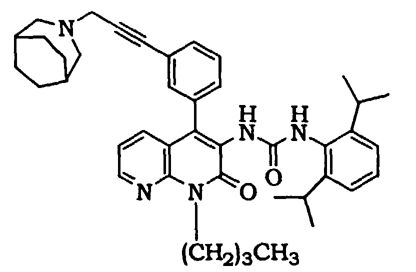

The title compound was obtained in the same manner as m Example 90 from N-[1-butyl-4-{3-(3-bromo-1-propynyl)phenyl}-1,2-dihydro-2-oxo-1,8-naphthyridin-3-yl]-N'-(2,6-diisopropylphenyl)urea and 3-azabicyclo[3.3.2]nonane.
Hydrochloride: m.p. 171-176°C

### Example 99

### Preparation of N-[1-butyl-4-[3-{3-(N-ethyl-N-propylamino)-1-propynyl)phenyl]-1,2-dihydro-2-oxo-1,8-naphthyridin-3-yl]-N'-(2,6-diisopropylphenyl)urea

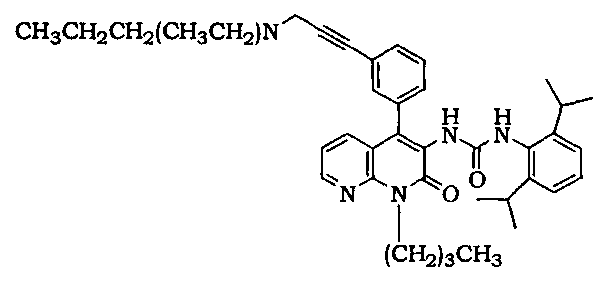

The title compound was obtained in the same manner as in Example 90 from N-[1-butyl-4-{3-(3-bromo-1-propynyl)phenyl}-1,2-dihydro-2-oxo-1,8-naphthyridin-3-yl]-N'-(2,6-diisopropylphenyl)urea and N-ethylpropylamine.
Hydrochloride: m.p. 163- 166.5°C

### Example 100

### Preparation of N-[1-butyl-4-[3-{3-(dibutylamino)-1-propynyl}phenyl]-1,2-dihydro-2-oxo-1,8-naphthyridin-3-yl]-N'-(2,6-diisopropylphenyl)urea

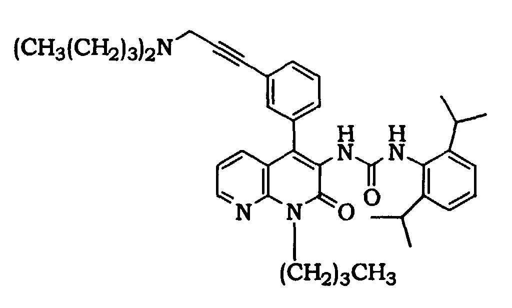

The title compound was obtained in the same manner as in Example 90 from N-[1-butyl-4-{3-(3-bromo-1-propynyl)phenyl}-1,2-dihydro-2-oxo-1,8-naphthyridin-3-yl]-N'-(2,6-diisopropylphenyl)urea and dibutylamine.
Hydrochloride: m.p. 138-141.5°C

### Example 101

### Preparation of N-[1-butyl-4-[3-{3-(diisopropylamino)-1-propynyl}phenyl]-1,2-dihydro-2-oxo-1,8-naphthyridln-3-yl]-N'-(2,6-diisopropylphenyl)urea

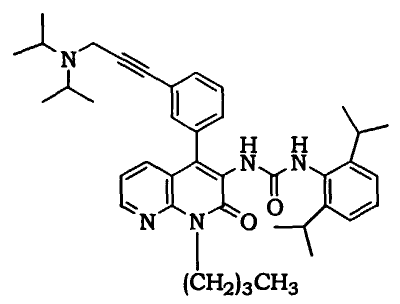

The title compound was obtained in the same manner as in Example 90 from N-[1-butyl-4-{3-(3-bromo-1-propynyl)phenyl}-1,2-dihydro-2-oxo-1,8-naphthyridin-3-yl]-N'-(2,6-diisopropylphenyl)urea and diisopropylamine.
Hydrochloride: m.p. 152-154°C

### Example 102

### Preparation of N-[1-butyl-4-[3-{3-di(2-ethoxyethyl)amino-1-propynyl}phenyl]-1,2-dihydro-2-oxo-1,8-naphthyridin-3-yl]-N'-(2,6-diisopropylphenyl)urea

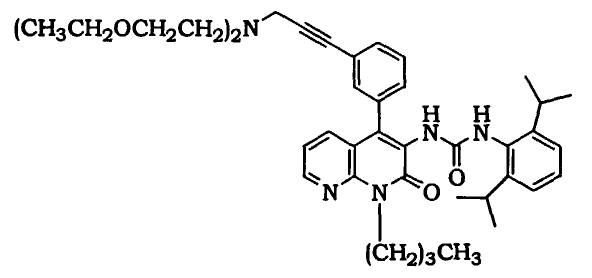

The title compound was obtained in the same manner as in Example 90 from N-[1-butyl-4-{3-(3-bromo-1-propynyl)phenyl}-1,2-dihydro-2-oxo-1,8-naphthyridin-3-yl]-N'-(2,6-diisopropylphenyl)urea and di(2-ethoxyethyl)amine.

### Hydrochloride:

¹H-NMR δ (DMSO-d₆) 10.68 (1H, br), 8.62 (1H, d, J=4.6Hz), 8.03 (1H, brs), 7.91 (1H, brs), 7.51-7.61 (3H, m), 7.49 (1H, brs), 7.43 (1H, d, J=7.0Hz), 7.26 (1H, dd, J=7.9Hz, 4.6Hz), 7.15 (1H, t, J=7.7Hz), 7.03 (2H, d, J=7.7Hz), 4.54 (2H, t, J=7.3Hz), 4.41 (2H, brs), 3.76 (4H, brs), 3.45-3.52 (8H, m), 2.80 (2H, br), 1.73 (2H, m), 1.45 (2H, m), 1.12 (6H, t, J=7.0Hz), 0.95-1.03 (15H, m)

### Example 103

### Preparation of N-[1-butyl-4-(4-bromophenyl)-1,2-dihydro-2-oxo-1,8-naphthyridin-3-yl]

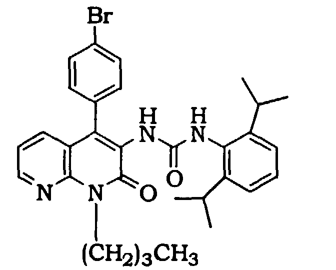

-N'-(2,6-diisopropylphenyl)urea

The title compound was obtained in the same manner as in Reference Example 10 from 1-butyl-4-(4-bromophenyl)-1,2-dihydro-2-oxo-1,8-naphthyridine-3-carboxylic acid and 2,6-diisopropylaniline.
m.p. 193- 195°C

### Example 104

### Preparation of N-[1-butyl-4-{4-(3-diethylamino-1-propynyl)phenyl}-1,2-dihydro-2-oxo-1,8-naphthyridin-3-yl]-N'-(2,6-diisopropylphenyl)urea

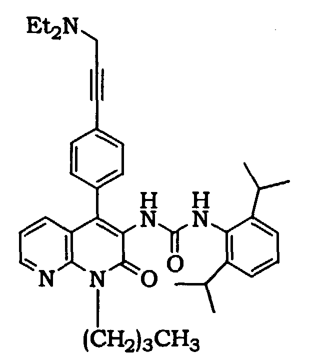

The title compound was obtained in the same manner as in Example 21 from N-[1-butyl-4-(4-bromophenyl)-1,2-dihydro-2-oxo-1,8-naphthyridin-3-yl]-N'-(2,6-diisopropylphenyl)urea and 3-diethylainino-1-propyne.
Hydrochloride: m.p. 208-210°C

### Example 105

### Preparation of N-[1-butyl-4-[3-{3-(morpholin-4-yl)-1-propynyl)phenyl}-1,2-dihydro-2-oxo-1,8-naphthyridin-3-yl]-N'-(2,6-diisopropylphenyl)urea

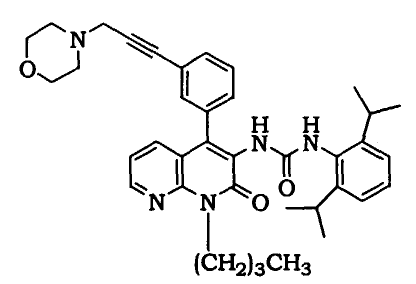

The title compound was obtained in the same manner as in Example 90 from N-[1-butyl-4-{3-(3-bromo-1-propynyl)phenyl}-1,2-dihydro-2-oxo-1,8-naphthyridin-3-yl]-N'-(2,6-diisopropylphenyl)urea.
Hydrochloride: m.p. 212-214°C

### Example 106

### Preparation of N-[1-butyl-4-{3-(3-hexylamino-1-propynyl)phenyl}-1,2-dihydro-2-oxo-1,8-naphthyridin-3-yl]-N'-(2,6-diisopropylphenyl)urea

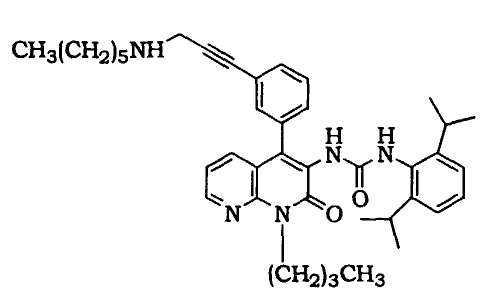

The title compound was obtained in the same manner as in Example 90 from N-[1-butyl-4-{3-(3-bromo-1-propynyl)phenyl}-1,2-dihydro-2-oxo-1,8-naphthyridin-3-yl]-N'-(2,6-diisopropylphenyl)urea and hexylamine.
Hydrochloride: m.p. 187-189°C

### Example 107

### Preparation of N-[1-butyl-4-{3-(4-phthalimido-1-butynyl)phenyl}-1,2-dihydro-2-oxo-1,8-naphthyridin-3-yl]-N'-(2,6-diisopropylphenyl)urea

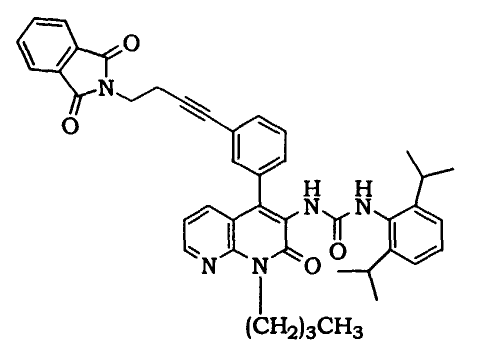

The title compound was obtained in the same manner as in Example 21 from N-[1-butyl-4-(3-bromophenyl)-1,2-dihydro-2-oxo-1,8-naphthyridin-3-yl]-N'-(2,6-diisopropylphenyl)urea and 4-phthalimido-1-butyne.
¹H-NMR δ (OD₃OD) 8.61 (1H, dd, J=4.8Hz, 1.7Hz), 7.78-7.85 (3H, m), 7.71-7.74 (1H, m), 7.62 (1H, dd, J=8.1Hz, 1.7Hz), 7.39-7.42 (2H, m), 7.14-7.31 (5H, m), 7.03 (2H, d, J=7.5Hz), 4.64 (2H, t, J=7.7Hz), 3.94 (2H, t, J=6.8Hz), 2.84 (2H, t, J=5.0Hz), 1.77-1.80 (2H, m), 1.46-1.54 (2H, m). 1.00-1.18 (15H, m)

### Example 108

### Preparation of N-[1-butyl-4-{3-(4-amino-1-butynyl)phenyl}-1,2-dihydro-2-oxo-1,8-naphthyridin-3-yl]-N'-(2,6-diisopropylphenyl)urea

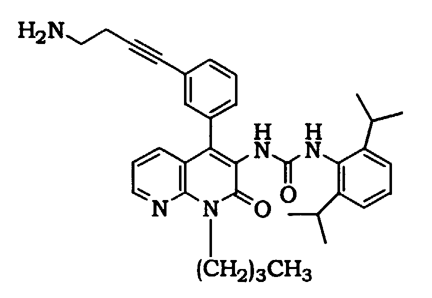

To a solution of N-[1-butyl-4-{3-(4-phthalimido-1-butynyl)phenyl}-1,2-dihydro-2-oxo-1,8-naphthyridin-3-yl]-N'-(2,6-diisopropylphenyl)urea (50 mg, 0.072 mmol) in ethanol (1 ml) was added 30 % methylamine ethanol solution (1.0 ml), and the mixture was stirred at room temperature. The mixture was concentrated under reduced pressure to remove the solvent, and the residue was purified by silica gel column chromatography (5 % methanol in chloroform) to give the title compound (33 mg, 0.058 mmol).
¹H-NMR δ (OD₃OD) 8.49 (1H, dd, J=4.6Hz, 1.7Hz), 7.56 (1H,dd, J=8.1Hz, 1.7Hz), 7.31-7.43 (3H, m), 7.23 (1H, d, J=7.3Hz, 7.01-7.12 (2H, m), 6.97 (2H, d, J=7.3Hz), 4.52 (2H, t, J=7.5Hz), 2.47 (2H, t, J=6.4Hz), 1.63-1.73 (2H, m), 1.33-1.45 (2H, m), 1.00 (12H, d, J=6.4Hz), 0.91 (3H, t, J=7.3Hz)

### Example 109

### Preparation of N-[1-butyl-4-{3-(3-cyclohexylamino-1-propynyl)phenyl}-1,2-dihydro-2-oxo-1,8-naphthyridin-3-yl]-N'-(2,6-diisopropylphenyl)urea

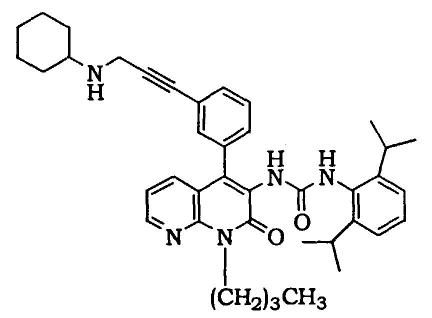

The title compound was obtained in the same manner as in Example 90 from N-[1-butyl-4-{3-(3-bromo-1-propynyl)phenyl}-1,2-dihydro-2-oxo-1,8-naphthyridin-3-yl]-N'-(2,6-diisopropylphenyl)urea and cyclohexylamine.
Hydrochloride: m.p. 186- 187°C

### Example 110

### Preparation of N-[1-butyl-4-[3-{3-(3-pyridylmethylamino)-1-propynyl}phenyl]-1,2-dihydro-2-oxo-1,8-naphthyridin-3-yl]-N'-(2,6-diisopropylphenyl)urea

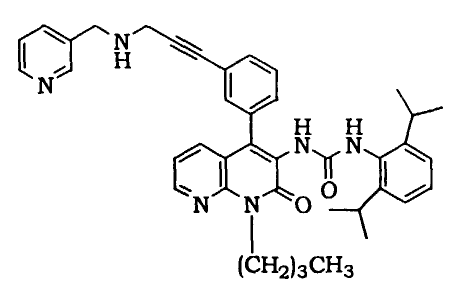

The title compound was obtained in the same manner as in Example 90 from N-[1-butyl-4-{3-(3-bromo-1-propynyl)phenyl}-1,2-dihydro-2-oxo-1,8-naphthyridin-3-yl]-N'-(2,6-diisopropylphenyl)urea and 3-(aminomethyl)pyridine.

### Hydrochloride:

¹H-NMR δ (DMSO-d₆) 10.29 (2H, brs), 8.98 (1H, s), 8.79 (1H, s), 8.63 (1H, d, J=3.9Hz), 8.46 (1H, d, J=7.3Hz), 8.03 (1H, s), 7.97 (1H, s), 7.83 (1H, dd, J=7.0Hz, 7.0Hz), 7.53-7.59 (3H, m), 7.48 (1H, s), 7.42 (1H, d, J=7.2Hz), 7.28 (1H, dd, J=7.9Hz, 5.0Hz), 7.16 (1H, dd, J=7.3Hz, 7.3Hz), 7.03 (2H, d, J=7.3Hz), 4.54 (2H, t, J=7.5Hz), 4.42 (2H, s), 4.21 (2H, s), 4.20 (2H, s), 2.80 (2H, brs), 1.76 (2H, m), 1.40-1.47 (2H, m), 0.95-1.03 (15H, m)

### Example 111

### Preparation of N-[1-butyl-4-[3-{3-(2-diethylaminoethyl)amino-1-propynyl}phenyl]-1,2-dihydro-2-oxo-1,8-naphthyridin-3-yl]-N'-(2,6-diisopropylphenyl)urea

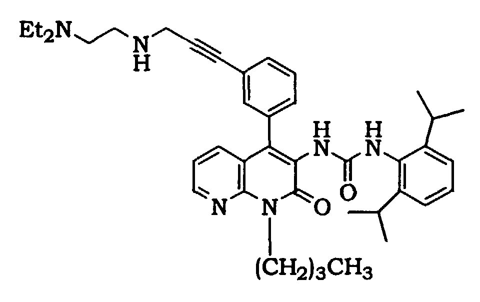

The title compound was obtained in the same manner as in Example 90 from N-[1-butyl-4-{3-(3-bromo-1-propynyl)phenyl}-1,2-dihydro-2-oxo-1,8-naphthyridin-3-yl]-N'-(2,6-diisopropylphenyl)urea and N,N-diethylethylenediamine.

### Hydrochloride:

¹H-NMR δ (CD₃OD) 8.62 (1H, dd, J=4.6Hz, 1.7Hz), 7.54-7.66 (4H, m), 7.47 (1H, d, J=7.7Hz), 7.22 (1H, dd, J=8.1Hz, 4.6Hz), 7.17 (1H, d, J=7.7Hz), 7.08 (2H, d, J=7.5Hz), 4.65 (2H, t, J=7.5Hz), 4.30 (2H, s), 3.65 (2H, t, J=7.9Hz), 3.51 (2H, t, J=7.7Hz), 2.87 (2H, brd), 1.77-1.87 (2H, m), 1.46-1.54 (2H, m), 1.29-1.40 (6H, m), 1.02-1.10 (15H, m)

### Example 112

### Preparation of N-[1-butyl-4-{3-(3-ethylamino-1-propynyl)phenyl}-1,2-dihydro-2-oxo-1,8-naphthyridin-3-yl]-N'-(2,6-diisopropylphenyl)urea

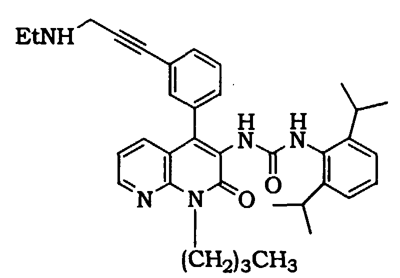

The title compound was obtained in the same manner as in Example 90 from N-[1-butyl-4-{3-(3-bromo-1-propynyl)phenyl}-1,2-dihydro-2-oxo-1,8-naphthyridin-3-yl]-N'-(2,6-diisopropylphenyl)urea and ethylamine.
Hydrochloride: m.p. 158-160°C

### Example 113

### Preparation of N-[1-butyl-4-{3-(4-diethylamino-1-butynyl)phenyl}-1,2-dihydro-2-oxo-1,8-naphthyridin-3-yl]-N'-(2,6-diisopropylphenyl)urea

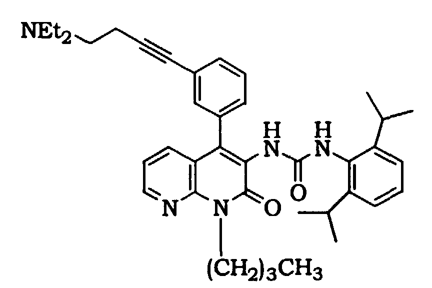

The title compound was obtained in the same manner as in Example 77 from N-[1-butyl-4-{3-(4-amino-1-butynyl)phenyl}-1,2-dihydro-2-oxo-1,8-naphthyridin-3-yl]-N'-(2,6-diisopropylphenyl)urea and ethyl iodide.

### Hydrochloride:

¹H-NMR δ (DMSO-d₆) 10.44 (1H, brs), 8.62 (1H, d, J=4.4Hz), 7.97 (1H, s), 7.89 (1H, s), 7.55 (1H, d, J=7.7Hz), 7.49 (2H, d, J=4.4Hz), 7.26 (1H, dd, J=7.7Hz, 4.4Hz), 7.16 (1H, dd, J=7.7Hz, 7.7Hz), 7.03 (2H, d, J=7.7Hz), 4.54 (2H, t, J=7.2Hz), 3.29 (2H, m), 3.15-3.17 (4H, m), 3.00 (2H, t, J=7.3Hz), 2.82 (2H, brs), 1.73 (2H, m), 1.40-1.47 (2H, m), 1.22 (6H, t, J=7.3Hz), 0.95-1.04 (15H, m)

### Example 114

### Preparation of N-[1-butyl-4-[3-{3-(2-pyridylmethylamino)-1-propynyl}phenyl]-1,2-dihydro-2-oxo-1,8-naphthyridin-3-yl]-N'-(2,6-diisopropylphenyl)urea

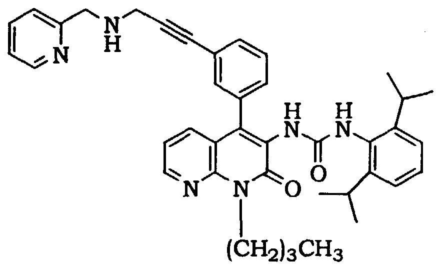

The title compound was obtained in the same manner as in Example 90 from N-[1-butyl-4-{3-(3-bromo-1-propynyl)phenyl}-1,2-dihydro-2-oxo-1,8-naphthyridin-3-yl]-N'-(2,6-diisopropylphenyl)urea and 2-(aminomethyl)pyridine.

### Hydrochloride:

¹H-NMR δ (CD₃OD) 8.59-8.64 (2H, m), 7.85 (1H, ddd, J=7.9Hz, 7.9Hz, J=1.84Hz), 7.63 (1H, d, J=6.4Hz), 7.39-7.62 (6H, m), 7.22 (1H, dd, J=7.9Hz, J=5.5Hz), 7.18 (1H, dd, J=7.7Hz, 7.7Hz), 7.07 (2H, d, J=5.5Hz), 4.65 (2H, t, J=7.7Hz), 4.50 (2H, s), 4.28 (2H, s), 2.88 (2H, brd), 1.72-1.82 (2H, m), 1.44-1.54 (2H, m), 1.08 (12H, m), 1.03 (3H, t, J=7.5Hz)

### Example 115

### Preparation of N-[1-butyl-4-[3-{3-(4-pyridylmethylamino)-1-propynyl}phenyl]-1,2-dihydro-2-oxo-1,8-naphthyridin-3-yl]-N'-(2,6-diisopropylphenyl)urea

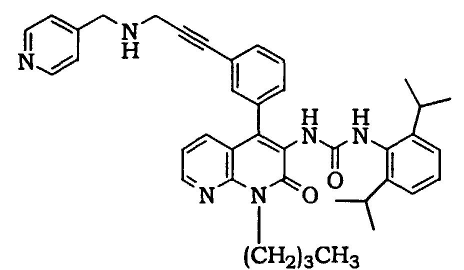

The title compound was obtained in the same manner as in Example 90 from N-[1-butyl-4-{3-(3-bromo-1-propynyl)phenyl}-1,2-dihydro-2-oxo-1,8-naphthyridin-3-yl]-N'-(2,6-diisopropylphenyl)urea and 4-(aminomethyl)pyridine.

### Hydrochloride:

¹H-NMR δ (CD₃OD) 8.88 (2H, d, J=4.6Hz), 8.61 (1H, d, J=9.4Hz), 8.18 (2H, d, J=4.6Hz), 7.55-7.63 (4H, m), 7.48 (1H, d, J=7.7Hz), 7.22 (1H, dd, J=7.9Hz, J=4.4Hz), 7.17 (2N, d, J=7.9Hz), 7.07 (2H, d, J=7.9Hz), 4.70 (2H, s), 4.65 (2H, t, J=7.9Hz), 4.35 (2H, s), 2.86 (2H, br), 1.80 (2H, m), 1.49-1.52 (2H, m), 1.09 (12H, brs), 1.03 (3H, t, J=7.3Hz)

### Example 116

### Preparation of N-[1-butyl-4-[3-{3-(N-(3-pyridylmethyl)-N-methyl-amino)-1-propynyl}phenyl]-1,2-dihydro-2-oxo-1,8-naphthyridin-3-yl]-N'-(2,6-diisopropylphenyl)urea

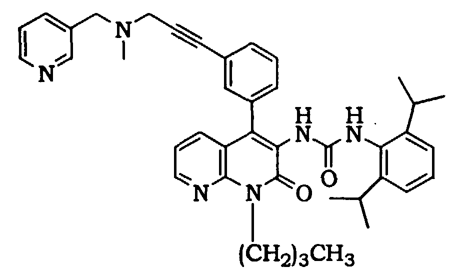

The title compound was obtained in the same manner as in Example 75 from N-[1-butyl-4-[3-{3-(3-pyridylmethylamino)-1-propynyl}phenyl]-1,2-dihydro-2-oxo-1,8-naphthyridin-3-yl]-N'-(2,6-diisopropylphenyl)urea and formaldehyde.

### Hydrochloride:

¹H-NMR δ (CD₃OD) 9.04 (1H, m), 8.73 (1H, m), 8.62 (1H, d, J=5.1Hz), 7.94 (1H, m), 7.57-7.67 (4H, m), 7.49 (1H, d, J=8.3Hz), 7.16-7.25 (3H, m), 7.09 (2H, m), 4.64-4.68 (4H, m), 4.38 (2H, s), 2.85 (2H, br), 3.02 (3H, s), 1.80-1.87 (2H, m), 1.46-1.52 (2H, m), 1.00-1.09 (12H, m), 1.03 (3H, t, J=7.3Hz)

### Example 117

### Preparation of N-[1-butyl-4-[3-{3-(N-(2-pyridylmethyl)-N-methylamino)-1-propynyl}phenyl]-1,2-dihydro-2-oxo-1,8-naphthyridin-3-yl]-N'-(2,6-diisopropylphenyl)urea

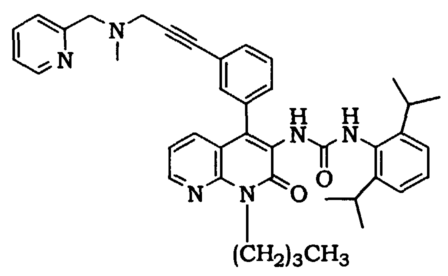

The title compound was obtained in the same manner as in Example 75 from N-[1-butyl-4-[3-{3-(2-pyridylmethylamino)-1-propynyl}phenyl]-1,2-dihydro-2-oxo-1,8-naphthyridin-3-yl]-N'-(2,6-diisopropylphenyl)urea and formaldehyde.

### Hydrochloride:

¹H-NMR δ (CD₃OD) 8.69 (1H, d, J=4.2Hz), 8.62 (1H, d, J=3.3Hz), 7.92 (1H, d, J=7.9Hz), 7.49-7.68 (7H, m), 7.16-7.21 (2H, m), 7.08 (2H, m), 4.66 (2H, t, J=7.7Hz), 4.62 (2H, s), 4.40 (2H, s), 3.02 (2H, br), 3.02 (3H, s), 1.80 (2H, m), 1.49-1.52 (2H, m), 1.08 (12H, brs), 1.03 (3H, t, J=7.3Hz)

### Example 118

### Preparation of N-[1-butyl-4-[3-{3-(N-(4-pyridylmethyl)-N-methylamino)-1-propynyl}phenyl]-1,2-dihydro-2-oxo-1,8-naphthyridin-3-yl]-N'-(2,6-diisopropylphenyl)urea

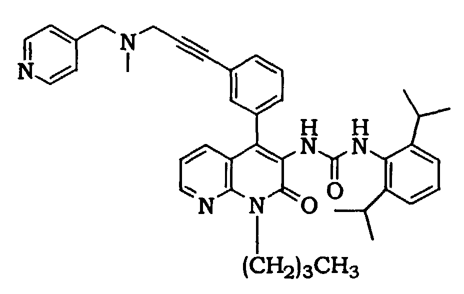

The title compound was obtained in the same manner as in Example 75 from N-[1-butyl-4-[3-{3-(4-pyridylmethylamino)-1-propynyl}phenyl]-1,2-dihydro-2-oxo-1,8-naphthyridin-3-yl]-N'-(2,6-diisopropylphenyl)urea and formaldehyde.

### Hydrochloride:

¹H-NMR δ (CD₃OD) 8.87 (2H, d, J=5.7Hz), 8.62 (1H, d, J=4.8Hz), 8.27 (2H, d, J=5.7Hz), 7.56-7.70 (4H, m), 7.49 (1H, d, J=7.5Hz), 7.23 (1H, dd, J=9.2Hz, 7.2Hz), 7.21 (1H, dd, J=6.8Hz, 6.8Hz), 7.07 (2H, d, J=8.4Hz), 4.75 (2H, s), 4.66 (2H, t, J=7.2Hz), 4.38 (2H, s), 3.03 (3H, s), 1.80 (2H, m), 1.52 (2H, m), 1.05-1.08 (12H, m), 1.03 (3H, t, J=7.3Hz)

### Example 119

### Preparation of N-[1-butyl-4-{3-(3-diethylamino-1-propynyl)phenyl}-1,2-dihydro-2-oxo-1,8-naphthyridin-3-yl]-N'-(2-isopropylphenyl)urea

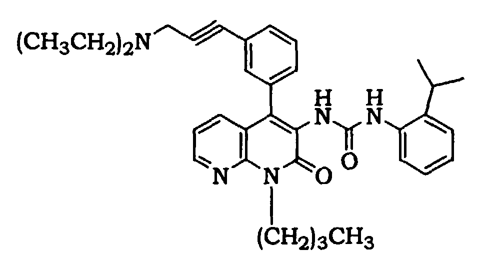

The title compound was obtained in the same manner as in Example 21 from N-[1-butyl-4-(3-bromophenyl)-1,2-dihydro-2-oxo-1,8-naphthyridin-3-yl]-N'-(2-isopropylphenyl)urea and 3-diethylamino-1-propyne.
Hydrochloride: m.p. 138-140°C

### Example 120

### Preparation of N-[1-butyl-4-{3-(3-diethylamino-1-propynyl)phenyl}-1,2-dihydro-2-oxo-1,8-naphthyridin-3-yl]-N'-(2,4,6-trimethylphenyl)urea

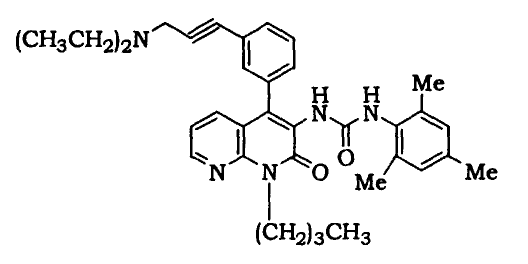

The title compound was obtained in the same manner as in Example 21 from N-[1-butyl-4-(3-bromophenyl)-1,2-dihydro-2-oxo-1,8-naphthyridin-3-yl]-N'-(2,4,6-trimethylphenyl)urea and 3-diethylainino-1-propyne.
¹H-NMR δ (DMSO-d₆) 8.61 (1H, dd, J=4.8Hz, 1.8Hz), 7.76 (1H, s), 7.56-7.63 (2H, m), 7.47 (2H, d, J=5.1Hz), 7.32-7.38 (2H, m), 7.25 (1H, dd, J=9.8Hz, 7.9Hz), 6.77 (2H, s), 4.51 (2H, brs), 3.59 (2H, s), 2.17 (3H, s), 1.92 (6H, s), 1.71 (2H, brs), 1.38-1.46 (2H, m), 1.01 (6H, t, J=7.2Hz), 0.97 (3H, t, J=7.2Hz)

### Example 121

### Preparation of N-[1-butyl-4-{3-(3-diethylamino-1-propynyl)phenyl}-1,2-dihydro-2-oxo-1,8-naphthyridin-3-yl]-N'-(2,4,6-trifluorophenyl)urea

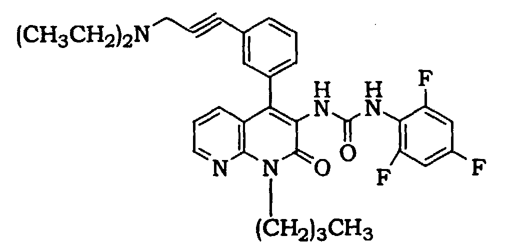

The title compound was obtained in the same manner as in Example 21 from N-[1-butyl-4-(3-bromophenyl)-1,2-dihydro-2-oxo-1,8-naphthyridin-3-yl]-N'-(2,4,6-trifluorophenyl)urea and 3-diethylamino-1-propyne.
¹H-NMR δ (CD₃OD) 8.61 (1H, dd, J=4.8Hz, 1.8Hz), 7.67 (1H, dd, J=8.1Hz, 1.8Hz), 7.44-7.55 (4H, m), 7.35 (1H, ddd, J=7.0Hz, 2.0Hz, 2.0Hz), 7.22 (1H, dd, J=8.1Hz, 4.8Hz), 6.84 (1H, dd, J=9.0Hz, 7.5Hz), 4.61 (2H, t, J=7.5Hz), 3.67 (2H, s), 2.67 (2H, q, J=7.2Hz), 1.72-1.82 (2H, m), 1.41-1.54 (2H, m), 1.12 (6H, t, J=7.3Hz), 1.00 (3H, t, J=7.2Hz)

### Example 122

### Preparation of N-[1-butyl-4-{3-(3-diethylamino-1-propynyl)phenyl}-1,2-dihydro-2-oxo-1,8-naphthyridin-3-yl]-N'-(2,4-difluorophenyl)urea

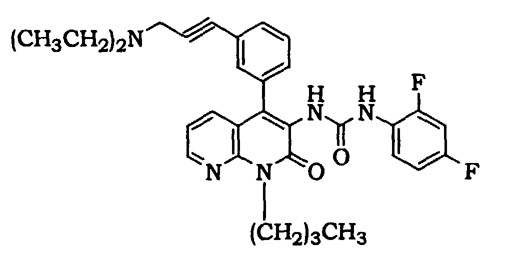

The title compound was obtained in the same manner as in Example 21 from N-[1-butyl-4-(3-bromophenyl)-1,2-dihydro-2-oxo-1,8-naphthyridin-3-yl]-N'-(2,4-difluorophenyl)urea and 3-diethylanuno-1-propyne.
¹H-NMR δ (CD₃OD) 8.60 (1H, dd, J=4.6Hz, 1.7Hz), 7.76 (1H, ddd, J=9.2Hz, 9.2Hz, 5.9Hz), 7.65 (1H, 8.1Hz, 1.8Hz), 7.45-7.51 (3H, m), 7.33-7.37 (1H, m), 7.21 (1H, dd, J=7.9Hz, 4.6Hz), 6.89 (1H, ddd, J=8.6Hz, 8.6Hz, 2.8Hz), 6.77 (1H, dd, J=8.1Hz, 8.1Hz), 4.56 (2H, t, J=7.9Hz), 3.61 (2H, s), 2.60 (4H, q, J=7.3Hz), 1.69-1.76 (2H, m), 1.41-1.48 (2H, m), 1.07 (6H, t, J=7.2Hz), 0.98 (3H, t, J=7.5Hz)

### Example 123

### Preparation of N-[1-butyl-4-(3-bromophenyl)-1,2-dihydro-2-oxo-1,8-naphthyridin-3-yl]-N'-(2-isopropylphenyl)urea

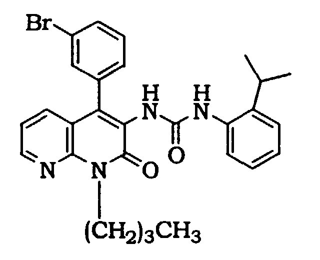

The title compound was obtained in the same manner as in Reference Example 10 from 1-butyl-4-(3-bromophenyl)-1,2-dihydro-2-oxo-1,8-naphthyridine-3-carboxylic acid and 2-isopropylaniline.
¹H-NMR δ (DMSO-d₆) 8.63 (1H, dd, J=4.8Hz, 1.7Hz), 8.13 (1H, s), 8.08 (1H, s), 7.60-7.65 (2H, m), 7.57 (1H, s), 7.47 (1H, dd, J=7.7Hz, 7.7Hz), 7.73 (1H, d, J=7.7Hz), 7.28 (1H, dd, J=8.1Hz, 4.8Hz), 7.17-7.22 (2H, m), 7.04-7.07 (2H, m), 4.53 (2H, t, J=7.2Hz), 2.92 (1H, sep, J=6.8Hz), 1.66-1.68 (2H, m), 1.28-1.41 (2H, m), 1.00 (12H, brs), 0.94 (3H, t, J=7.2Hz)

### Example 124

### Preparation of N-[1-butyl-4-(3-bromophenyl)-1,2-dihydro-2-oxo-1,8-naphthyridin-3-yl]-N'-(2,4,6-trimethylphenyl)urea

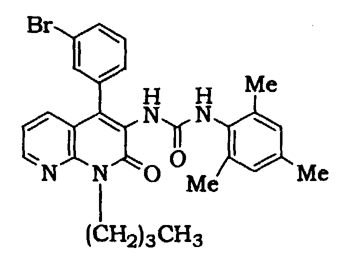

The title compound was obtained in the same manner as in Reference Example 10 from 1-butyl-4-(3-bromophenyl)-1,2-dihydro-2-oxo-1,8-naphthyridine-3-carboxylic acid and 2,4,6-trimethylaniline.
¹N-NMR δ (DMSO-d₆) 8.63 (1H, s), 7.93 (1H, s), 7.82 (1H, s), 7.55-7.65 (3H, m), 7.43-7.48 (1H, m), 7.34-7.36 (1H, m), 7.24-7.29 (1H, m), 7.11 (2H, d, J=5.9Hz), 6.78 (2H, s), 4.52 (2H, s), 3.33 (2H, m), 2.18 (3H, s), 1.92 (6H, s), 1.71 (2H, m), 1.41 (2H, m), 0.97 (3H, t, J=7.3Hz)

### Example 125

### Preparation of N-[1-butyl-4-(3-bromophenyl)-1,2-dihydro-2-oxo-1,8-naphthyridin-3-yl]-N'-(2,4,6-trifluorophenyl)urea

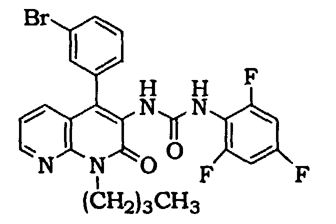

The title compound was obtained in the same manner as in Reference Example 10 from 1-butyl-4-(3-bromophenyl)-1,2-dihydro-2-oxo-1,8-naphthyridine-3-carboxylic acid and 2,4,6-trifluoroaniline.
¹H-NMR δ (DMSO-d₆) 8.63 (1H, dd, J=4.8Hz, 1.7YHz), 8.18 (1H, s), 8.05 (1H, s), 7.64 (1H, d, J=7.9Hz), 7.57 (1H, dd, J=7.9Hz, 1.7Hz), 7.54 (1H, dd, J=1.7Hz, 1.7Hz), 7.45 (1H, dd, J=7.9Hz, 7.9Hz), 7.34 (1H, d, J=7.9Hz), 7.26 (1H, dd, J=8. 1Hz, 4.8Hz), 7.15 (2H, dd, J=9.0Hz, 7.7Hz), 4.49 (2H, t, J=7.5Hz), 1.64-1.71 (2H, m), 1.36-1.43 (2H, m), 0.95 (3H, t, J=7.2Hz)

### Example 126

### Preparation of N-[1-butyl-4-(3-bromophenyl)-1,2-dihydro-2-oxo-1,8-naphthyridin-3-yl]-N'-(2,4-difluorophenyl)urea

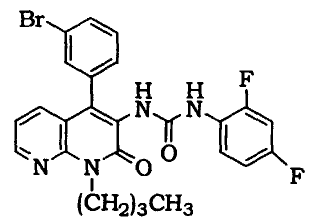

The title compound was obtained in the same manner as in Reference Example 10 from 1-butyl-4-(3-bromophenyl)-1,2-dihydro-2-oxo-1,8-naphthyridine-3-carboxylic acid and 2,4-difluoroaniline.
¹H-NMR δ (DMSO-d₆) 8.68 (1H, dd, J=1.7Hz), 8.63 (1H, dd, J=4.6Hz, 1.7Hz), 8.19 (1H, s), 7.76 (1H, ddd, J=9.2Hz, 9.2Hz, 6. 1Hz), 7.63 (1H, d, J=8. 1Hz), 7.55-7.58 (2H, m), 7.47 (1H, dd, J=7.7Hz, 7.7Hz), 7.73 (1H, d, J=7.5Hz), 7.27 (1H, dd, J=8.1Hz, 4.8Hz), 7.23 (1H, ddd, J=11.6Hz, 9.0Hz, 2.0Hz), 6.95 (1H, dd, J=8.4Hz, 8.4Hz), 4.50 (2H, t, J=7.3Hz), 1.64-1.74 (2H, m), 1.33-1.46 (1H, m), 0.95 (3H, t, J=7.3Hz)

The ACAT inhibitory activity of the present compounds were evaluated as follows.

### Experiment

### 1. Assay of ACAT inhibitory activity in a specimen prepared from rabbit liver

An enzyme specimen ACAT was prepared according to the method disclosed in the literature: J. Lipid. Research, 30, 681-690, 1989, from the liver of New Zealand white rabbit, which had been fed with 1 % cholesterol feed for one month. The ACAT activity was determined according to the method disclosed in the literature: J. Lipid Research, 24, 1127-1134, 1983, i.e., using radioactive [1-14C]oleoyl-CoA and endogenous cholesterol contained in the liver microsome, and calculated from the radioactivity of the labeled cholesterol oleate. The results are shown in Table 1.

**Table 1**

| Test compound (Example No.) | ACAT inhibitory rate (%) 10⁻⁷ M |
|---|---|
| 90 | 39 |

### 2. Assay of ACAT inhibitory activity in the macrophage derived from rat peritoneal

The rat peritoneal-derived macrophage was prepared according to the method disclosed in the literature: Biochimica et Biophysica Acta, 1126, 73-80, 1992. The ACAT activity was determined by a modified method of the method disclosed in the above literature: Biochimica et Biophysica Acta, 1126, 73-80, 1992, i.e., using radioactive [9, 10-H]oleic acid and exogenous cholesterol contained in the liposome which was reconstituted according to the method disclosed in the literature: Biochimica et Biophysica Acta, 1213, 127-134, 1994, and calculated from the radioactivity of the labeled cholesterolyl oleate. The results are shown in Table 2.

**Table 2**

| Test compound (Example No.) | ACAT inhibitory rate (%) 10⁻⁷ M |
|---|---|
| 90 | 96 |

### INDUSTRIAL APPLICABILITY

The naphthyridine derivative of the present invention or an acid addition salt thereof strongly inhibits ACAT activity in a specimen of rabbit liver or in rat peritoneal-derived macrophage. Therefore, the present compound or an acid addition salt thereof is useful not only as an agent for treatment of hyperlipidemia, but also in the prophylaxis or therapeutic treatment of atherosclerosis per se or various diseases accompanied by atherosclerosis, for example, cerebral infarction, cerebral thrombosis, transient cerebral ischemia, angina pectoris, myocardial infarction, peripheral thrombus or occlusion.

## Claims

1. A naphthyridine derivative of the formula (1): 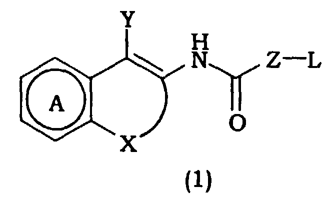 wherein Ring A is a substituted or unsubstituted pyridine ring,
X is a group of the formula: 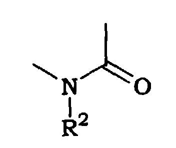 wherein R² is a hydrogen atom, an alkyl group, a substituted alkyl group, an alkenyl group, a substituted alkenyl group, an alkynyl group, a substituted alkynyl group, a cycloalkyl group, or a substituted cycloalkyl group, or a group of the formula: 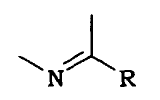 wherein R is a hydrogen atom or a group: ―OR¹ (R¹ is an alkyl group, a substituted alkyl group, an alkenyl group, a substituted alkenyl group, an alkynyl group, or a substituted alkynyl group),
Z is a direct bond, ―NH―, an alkylene group having 1 to 2 carbon atoms, or ―CH=CH―,
Y is an an aromatic group or a substituted aromatic group,
L is an alkyl group, a substituted alkyl group, an alkenyl group, a substituted alkenyl group, a cycloalkyl group, a substituted cycloalkyl group, an aromatic group, or a substituted aromatic group, or an acid addition salt thereof.

2. The naphthyridine derivative according to claim 1, wherein Z is ―NH―, or an acid addition salt thereof.

3. The naphthyridine derivative according to claim 1 or 2, wherein Ring A is a group selected from the following formulae (a), (b) and (c): 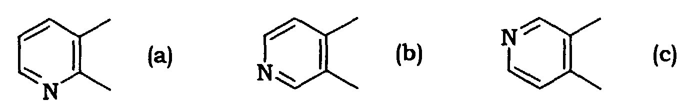 or an acid addition salt thereof.

4. The naphthyridine derivative according to claim 3, wherein X is a group of the formula: 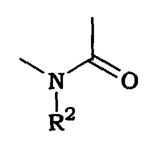 wherein R² is the same as defined in claim 1, or an acid addition salt thereof.

5. The naphthyridine derivative according to claim 4, wherein L is an aromatic group or a substituted aromatic group, or an acid addition salt thereof.

6. The naphthyridine derivative according to claim 5, wherein R² is an alkyl group, a substituted alkyl group, an alkenyl group, or a substituted alkenyl group, or an acid addition salt thereof.

7. The naphthyridine derivative according to claim 6, wherein Y is a substituted phenyl group and said substituent is a group of the formula: ―M¹―E―Q (M¹ is a direct bond, an oxygen atom, a sulfur atom or a group of the formula: ―NR³― (R³ is a hydrogen atom or a lower alkyl group), E is a divalent hydrocarbon group having 1 to 15 carbon atoms and optionally containing an unsaturated bond, or a phenylene group, Q is a hydrogen atom, a hydroxy group, a carboxyl group, a lower alkoxycarbonyl group, a benzyloxycarbonyl group, a halogen atom, a cyano group, a benzyloxy group, a lower alkoxy group, a lower alkanoyloxy group, a lower alkylthio group, a lower alkylsulfinyl group, a lower alkylsulfonyl group, a benzenesulfonyloxy group optionally being substituted by an alkyl group, a lower alkanoylamino group, a lower alkoxycarbonylamino group, a lower alkylsulfonamido group, a phthalimido group, a cycloakyl group, an aryl group, a substituted aryl group, a heteroaryl group, a substituted heteroaryl group, a group of the formula: ―NR⁴R⁵ (R⁴ and R⁵ are independently a hydrogen atom, a lower alkyl group, a di-lower alkylamino-substituted lower alkyl group, a lower alkoxy-substituted lower alkyl group, a cycloalkyl group, a lower alkoxycarbonyl group, a heteroarylmethyl group, or an aralkyl group, or R⁴ and R⁵ may combine each other, and with the adjacent nitrogen atom to which they bond, form a saturated cyclic amino group having 4 to 8 carbon atoms as ones forming the said ring, and optionally having one ―NR⁸― (R⁸ is a hydrogen atom, a lower alkyl group, a phenyl group, a lower alkoxycarbonyl group, or a benzyl group) or one oxygen atom in the cycle thereof), or a group of the formula: ―C(=O)NR⁴R⁵ (R⁴ and R⁵ are the same as defined above)), or an acid addition salt thereof.

8. The naphthyridine derivative according to claim 7, wherein M¹ is an oxygen atom, or an acid addition salt thereof.

9. The naphthyridine derivative according to claim 8, wherein E is an alkylene group having 1 to 4 carbon atoms, Q is a substituted or unsubstituted pyridyl group, or a 1,2,4-triasolyl group, or an acid addition salt thereof.

10. The naphthyridine derivative according to claim 7, wherein M¹ is a direct bond.

11. The naphthyridine derivative according to claim 10, wherein ―E― is a group fo the formula: 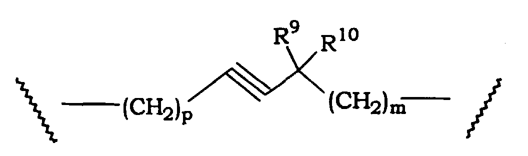 (in which R⁹ and R¹⁰ are independently a hydrogen atom, a methyl group, an ethyl group, or a propyl group, or may combine each other to form a 3- to 7-membered cycloalkane ring, m is an integer of 0 to 6, and p is an integer of 0 to 6), or an acid addition salt thereof.

12. The naphthyridine derivative according to claim 11, wherein R⁹ and R¹⁰ are hydrogen atoms, and m is an integer of 0 to 6, or an acid addition salt thereof.

13. The naphthyridine derivative according to claim 12, wherein m is 0 or 1, or an acid addition salt thereof.

14. The naphthyridine derivative according to claim 11, wherein R⁹ and R¹⁰ are independently a hydrogen atom, a methyl group, an ethyl group, or a propyl group, or may combine each other to form a 3- to 7-membered cycloalkane ring, and m is 0, or an acid addition salt thereof.

15. The naphthyridine derivative according to any one of claims 10, 11, 12, 13 or 14, wherein Q is a hydrogen atom, a hydroxy group, a carboxyl group, a lower alkoxycarbonyl group, a benzyoxycarbonyl group, a benzyloxy group, a lower alkoxy group, a lower alkanoyloxy group, a lower alkanoylamino group, a heteroaryl group, a substituted heteroaryl group, or a group of the formula: ―NR⁴R⁵ (in which R⁴ and R⁵ are the same as defined in claim 7), or an acid addition salt thereof.

16. The naphthyridine derivative according to any of claims 10, 11, 12, 13, 14 or 15, wherein R² is an alkyl group or a substituted alkyl group, or an acid addition salt thereof.

17. The naphthyridine derivative according to any of claims 7, 8, 9, 10, 11, 12, 13, 14, 15 or 16, wherein Ring A is an unsubstituted pyridine ring, or an acid addition salt thereof.

18. A pharmaceutical composition containing a naphthyridine derivative as set forth in any one of claims 1-17, or an acid addition salt thereof.

19. An acyl-CoA: cholesterol acyl transferase (ACAT) inhibitor, which contains as an active ingredient a naphthyridine derivative as set forth in any one of claims 1-17, or an acid addition salt thereof.

20. An agent for treatment of hyperlipidemia and atherosclerosis, which contains as an active ingredient a naphthyridine derivative as set forth in any one of claims 1-17, or an acid addition salt thereof.
